# EUROPÄISCHE PATENTSCHRIFT

(11) **EP 1 161 423 B1**
(45) Veröffentlichungstag und Bekanntmachung des Hinweises auf die Patenterteilung: **10.11.2004**
(21) Anmeldenummer: 00907614.2
(22) Anmeldetag: 24.02.2000
(51) Int. Cl.: C07D 277/54, C07D 417/12, C07D 487/04, A61K 31/426, A61K 31/427, A61P 31/12

(54) **THIAZOLYLHARNSTOFF-DERIVATE UND IHRE VERWENDUNG ALS ANTIVIRALE MITTEL**
THIAZOLYL UREA DERIVATIVES AND THEIR UTILIZATION AS ANTIVIRAL AGENTS
DERIVES DE LA THIAZOLYLUREE ET LEUR UTILISATION COMME ANTIVIRAUX

(30) Priorität: 08.03.1999 DE 19910245; 13.12.1999 DE 19959958
(43) Veröffentlichungstag der Anmeldung: 12.12.2001
(73) Patentinhaber: Bayer HealthCare AG, 51368 Leverkusen (DE)
(72) Erfinder: FISCHER, Rüdiger, D-50933 Köln (DE); KLEYMANN, Gerald, D-42113 Wuppertal (DE); BAUMEISTER, Judith, D-42277 Wuppertal (DE); BENDER, Wolfgang, D-42113 Wuppertal (DE); BETZ, Ulrich, D-42287 Wuppertal (DE); ECKENBERG, Peter, D-42115 Wuppertal (DE); HANDKE, Gabriele, D-42489 Wülfrath (DE); HENDRIX, Martin, D-51061 Köln (DE); SCHNEIDER, Udo, D-51373 Leverkusen (DE); WEBER, Olaf, D-42489 Wülfrath (DE); HENNINGER, Kerstin, D-42115 Wuppertal (DE); JENSEN, Axel, D-42553 Velbert (DE); KELDENICH, Jörg, D-42113 Wuppertal (DE)
(86) Internationale Anmeldenummer: PCT/EP2000/001498
(87) Internationale Veröffentlichungsnummer: WO 2000/053591

(56) Entgegenhaltungen:
- WO-A-97/24343
- WO-A-99/37291

## Beschreibung

Die vorliegende Erfindung betrifft Thiazolylharnstoff-Derivate, ein Verfahren zu ihrer Herstellung sowie ihre Verwendung. als Arzneimittel, insbesondere als antivirale Arzneimittel.

Aus der Publikation C. Ziegler et al., J. Org. Chem. 25, 1960, 1454-1455 sind 2-Aminothiazol-5-sulfonamide bekannt. Außerdem werden in der deutschen Offenlegungsschrift 2101640 N-Thiazol-2-yl-amide und -harnstoffe mit einer herbiziden Wirkung beschrieben.

Die WO97/24343 betrifft Phenylthiazolderivate mit Anti-Herpes Virus-Eigenschaften.

Die WO 99/42455 betrifft ebenfalls Phenylthiazolderivate mit Anti-Herpes Virus-Eigenschaften.

Die WO99/47507 betrifft 1,3,4-Thiadiazolderivate mit Anti-Herpes Virus-Eigenschaften.

Die vorliegende Erfindung betrifft Thiazolylharnstoff-Derivate der allgemeinen Formel (I), 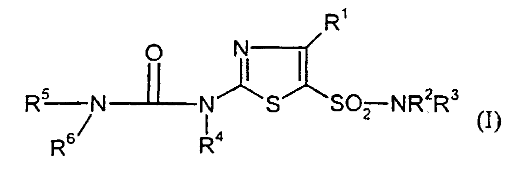 in welcher
- R¹: für Wasserstoff, Halogen, (C₁-C₆)-Alkyl, (C₁-C₆)-Alkoxy, Amino(C₁-C₆)alkyl oder Halogen(C₁-C₆)alkyl steht,
- R² und R³: gleich oder verschieden sind und für Wasserstoff, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Resten der Formel 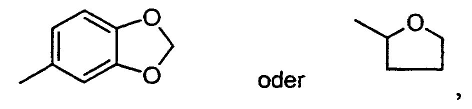 einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8- gliedrigen gesättigten oder ungesättigten, nicht aromatischen, Heterocyclus mit bis zu 3 Heteroatomen aus der Reihe S, N und/oder O, und (C₆-C₁₀)-Aryl, das seinerseits durch Hydroxy oder (C₁-C₆)-Alkoxy substituiert sein kann, besteht, oder
- R² und R³: gemeinsam mit dem Stickstoffatom einen 5- bis 6-gliedrigen gesättigten Heterocyclus bildet, der gegebenenfalls noch ein Sauerstoffatom besitzen kann,
- R⁴: für Wasserstoff, (C₁-C₆)-Acyl, (C₂-C₆)-Alkenyl steht oder
- R⁴: für (C₁-C₆)-Alkyl steht, das gegebenfalls substituiert sein kann durch 1 bis 3 Substituenten, die aus der Gruppe ausgewählt werden, die aus Halogen, Hydroxy, (C₁-C₆)-Acyl, (C₁-C₆)-Alkoxy, Phenoxy, (C₆-C₁₀)-Aryl und -NR⁷R⁸ besteht,
worin R⁷ und R⁸ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxycarbonyl bedeuten, oder
R⁷ und R⁸ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR⁹ enthalten kann, und durch Oxo substituiert sein kann,
worin
R⁹ Wasserstoff oder (C₁-C₄)-Alkyl bedeutet, oder
- R⁴: für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 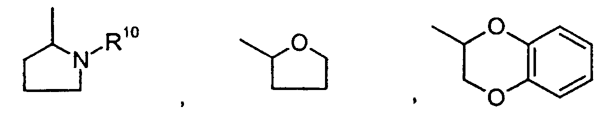 oder 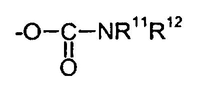 substituiert ist, worin
R¹⁰ Wasserstoff oder (C₁-C₆)-Alkyl bedeutet,
R¹¹ und R¹² gleich oder verschieden sind und Wasserstoff, (C₁-C₆) Alkyl oder (C₆-C₁₀)-Ary) bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
- R⁵: für Wasserstoff oder (C₁-C₆)-Alkyl steht,
- R⁶: für einen Rest der Formel 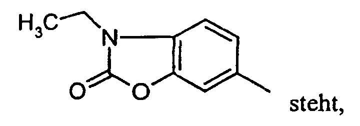
oder
- R⁶: für Phenyl steht, das gegebenenfalls mit ein bis drei Substituenten substituiert sein kann, die aus der Gruppe ausgewählt werden, die aus
Halogen, (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Resten der Formeln 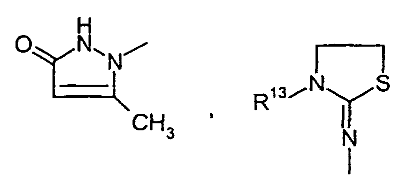 oder, 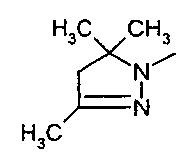 worin
R¹³ Wasserstoff oder (C₁-C₆)-Alkyl bedeutet, (C₁-C₆)-Alkyl, das gegebenenfalls durch einen Rest der Formel 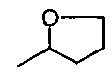 substituiert ist,
einem gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann,
einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)Alkoxycarbonylamino, (C₁-C₆)-Alkyl, Halogen(C₁-C₆)-alkyl und Hydroxy(C₁-C₆)-alkyl substituiert sein kann,
und Gruppen der Formeln -OR¹⁴, -NR¹⁵R¹⁶ oder -CO-NR¹⁷R¹⁸ besteht,
worin
- R¹⁴: einen Rest der Formel 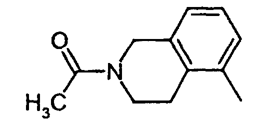 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten, oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten,
und deren Salze.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Die Erfindung schließt in ihrem Umfang auch solche Verbindungen ein, die erst im Körper zu den eigentlichen Wirkstoffen der Formel (I) umgewandelt werden (sogenannte Prodrugs).

Die erfindungsgemäßen Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

(C₁-C₆)-Alkyl steht für einen geradkettigen oder verzweigten Alkylrest mit 1 bis 6 Kohlenstoffatomen. Bevorzugt ist ein geradkettiger oder verzweigter Alkylrest mit 1 bis 4 Kohlenstoffatomen (C₁-C₄). Beispielsweise seien genannt: Methyl, Ethyl, n-Propyl, Isopropyl, n-Butyl, sek-Butyl, tert.-Butyl, n-Pentyl und n-Hexyl. Besonders bevorzugt ist ein geradkettiger oder verzweigter Alkylrest mit 1 bis 3 Kohlenstoffatomen ((C₁-C₃)Alkyl).

Halogen(C₁-C₆)-alkyl steht für eine (C₁-C₆)Alkylgruppe, die wie oben definiert sein kann, und die 1 bis 3 Halogenatome, nämlich F, Cl, Br und/oder I, bevorzugt Chlor oder Fluor, als Substituenten aufweist, beispielsweise seien erwähnt Trifluormethyl, Fluormethyl etc.

Hydroxy(C₁-C₆)-alkyl steht für eine (C₁-C₆)Alkylgruppe, die wie oben definiert sein kann, und die 1 bis 3 Hydroxygruppen als Substituenten aufweist, beispielsweise seien erwähnt Hydroxymethyl etc.

(C₂-C₆)-Alkenyl steht im Rahmen der Erfindung für einen geradkettigen oder verzweigten Alkenylrest mit 2 bis 6 Kohlenstoffatomen. Beispielsweise seien genannt: Ethenyl, n-Prop-2-en-1-yl und n-But-2-en-1-yl. Bevorzugt ist ein geradkettiger oder verzweigter Alkenylrest mit 2 bis 4 Kohlenstoffatomen.

(C₁-C₆)-Alkoxy steht für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen (C₁-C₄). Beispielsweise seien genannt: Methoxy, Ethoxy, n-Propoxy, Isopropoxy, tert.Butoxy, n-Pentoxy und n-Hexoxy. Besonders bevorzugt ist ein geradkettiger oder verzweigter Alkoxyrest mit 1 bis 3 Kohlenstoffatomen (C₁-C₃).

Partiell fluoriertes (C₁-C₆)-Alkoxy mit bis zu 6 Fluoratomen steht für einen geradkettigen oder verzweigten Alkoxyrest mit 1 bis 6 Kohlenstoffatomen, der mit 1 bis 6, bevorzugt 1 bis 4, noch bevorzugter 1 bis 3 Fluoratomen substituiert sein kann. Bevorzugt ist ein geradkettiger oder verzweigter Alkoxyrest mit 1 bis 4 Kohlenstoffatomen und 1 bis 4 Fluoratomen. Beispielsweise seien genannt: Methoxy, Ethoxy, n-Propoxy, Isopropoxy, tert.Butoxy, n-Pentoxy und n-Hexoxy, die jeweils ein bis 4 Fluoratome aufweisen. Besonders bevorzugt sind (1,3-Difluorprop-2-yl)oxy und 1, 1,2,2-Tetrafluorethoxy.

(C₁-C₆)-Alkylthio steht für einen geradkettigen oder verzweigten Alkythiorest mit 1 bis 6 Kohlenstoffatomen. Bevorzugt ist ein geradkettiger oder verzweigter Alkylthiorest mit 1 bis 4 Kohlenstoffatomen (C₁-C₄). Beispielsweise seien genannt: Methylthio, Ethylthio, n-Propylthio, Isopropylthio, tert.Butylthio, n-Pentylthio und n-Hexylthio. Besonders bevorzugt ist ein geradkettiger oder verzweigter Alkylthiorest mit 1 bis 3 Kohlenstoffatomen (C₁-C₃)Alkylthio.

(C₁-C₆)-Alkoxycarbonyl steht für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen (C₁-C₄). Beispielsweise seien genannt: Methoxycarbonyl, Ethoxycarbonyl, n-Propoxycarbonyl, Isopropoxycarbonyl und tert.Butoxycarbonyl. Besonders bevorzugt ist ein geradkettiger oder verzweigter Alkoxycarbonylrest mit 1 bis 3 Kohlenstoffatomen (C₁-C₃).

Mono- oder Di(C₁-C₆)alkylaminocarbonyl steht im Rahmen der Erfindung zweckmäßig für eine Carbamoylgruppe (H₂N-CO-), in der ein oder beide Wasserstoffatome durch eine (C₁-C₆)Alkylgruppe, ersetzt sind. Bezüglich der Definition der (C₁-C₆)Alkylgruppe sei auf die obigen Erläuterung von (C₁-C₆)Alkyl verwiesen. Beispielsweise seien erwähnt Methylaminocarbonyl, Dimethylaminocarbonyl etc.

Mono- oder Di- (C₁-C₆)acylamino steht im Rahmen der Erfindung zweckmäßig für eine Aminogruppe (H₂N-), in der ein oder beide Wasserstoffatome durch eine (C₁-C₆)Acylgruppe ersetzt sind. Bezüglich der Definition der (C₁-C₆)Acylgruppe sei auf die obigen Erläuterung von (C₁-C₆)Acyl verwiesen. Beispielsweise seien erwähnt (C₁-C₆)Alkanoyl, wie in der Definition von (C₁-C₆)Acyl erwähnt.

(C₁-C₆)Alkylsulfoxy stellt zweckmäßig eine (C₁-C₆)Alkyl-S(=O)-Gruppe dar, wobei bezüglich der (C₁-C₆)Alkylgruppe auf die diesbezügliche obige Definition verwiesen werden kann.

(C₁-C₆)Alkylsulfonyl stellt zweckmäßig eine (C₁-C₆)Alkyl-SO₂-Gruppe dar, wobei bezüglich der (C₁-C₆)Alkylgruppe auf die diesbezügliche obige Definition verwiesen werden kann.

(C₆-C₁₀)-Aryl steht im allgemeinen für einen aromatischen Rest mit 6 bis 10 Kohlenstoffatomen. Bevorzugte Arylreste sind Phenyl und Naphthyl.

(C₁-C₆)-Acyl steht im Rahmen der Erfindung zweckmäßig für einen geradkettigen oder verzweigten Acylrest mit 1 bis 6 Kohlenstoffatomen. Beispielsweise seien genannt: Formyl, Acetyl, Ethylcarbonyl, Propylcarbonyl, Isopropylcarbonyl, Butylcarbonyl, Isobutylcarbonyl, Pentylcarbonyl und Hexylcarbonyl. Bevorzugt ist ein geradkettiger oder verzweigter Acylrest mit 1 bis 4 Kohlenstoffatomen. Besonders bevorzugt sind Acetyl und Ethylcarbonyl.

(C₃-C₈)-Cycloalkyl steht im Rahmen der Erfindung zweckmäßig für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl. Die Bedeutung von (C₃ C₆)Cycloalkyl steht entsprechend zweckmäßig für Cyclopropyl, Cyclopentyl, Cyclobutyl, Cyclohexyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Ein 5- bis 6-gliedriger aromatischer Heterocyclus mit bis zu 3 Heteroatomen aus der Reihe S, O und/oder N steht beispielsweise für Pyridyl, Pyrimidyl, Thienyl, Furyl, Pyrrolyl, Thiazolyl, N-Triazolyl, Oxazolyl oder Imidazolyl. Bevorzugt sind Pyridyl, Furyl, Thiazolyl und N-Triazolyl.

Ein 5- bis 6-gliedriger aromatischer benzokondensierter Heterocyclus mit bis zu 3 Heteroatomen aus der Reihe S, O und/oder N steht beispielsweise für Benzimidazolyl.

Ein 5- bis 6-gliedriger über ein Stickstoffatom gebundener gesättigter Heterocyclus, der aus zwei Substituentengruppen zusammen mit dem Stickstoffatom, an das sie gebunden sind, gebildet werden kann, und der gegebenenfalls ein weiteres Heteroatom aus der Reihe S oder O oder einem Rest der Formel -NR⁹, worin R⁹ wie oben definiert ist, enthalten kann, steht im Rahmen der Erfindung im allgemeinen für Morpholinyl, Piperidinyl, Piperazinyl, Methylpiperazinyl, Thiomorpholinyl oder Pyrrolidinyl Besonders bevorzugt sind Morpholinyl, Piperidinyl, Pyrrolidinyl und Thiomorpholinyl.

Ein gegebenenfalls über ein Stickstoffatom gebundener 3- bis 8- gliedriger gesättigter oder ungesättigter, nicht aromatischer Heterocyclus mit bis zu 3 Heteroatomen aus der Reihe S, N und/oder O schließt z.B. die oben genannten 5- bis 6-gliedrigen über ein Stickstoffatom gebundenen gesättigten Heterocyclen ein sowie 3-, 7- und 8-gliedrige Heterocyclen, wie z.B. Aziridine (z.B. 1-Azacyclopropan-1-yl), Azetidine (z.B. 1-Azacyclobutan-3-yl) und Azepine (z.B. 1-Azepan-1-yl) ein. Die ungesättigten Vertreter können 1 bis 2 Doppelbindungen im Ring enthalten.

In einer bevorzugten Ausführungsform betrifft die Erfindung Verbindungen der allgemeinen Formel (I), worin
- R⁶: für Phenyl steht, das gegebenenfalls mit ein bis drei Substituenten substituiert sein kann, die aus der Gruppe ausgewählt werden, die aus Halogen, (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen, (C₁-C₆)-Alkyl, einem gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)Alkoxycarbonylamino, (C₁-C₆)-Alkyl, Halogen(C₁-C₆)-alkyl und Hydroxy(C₁-C₆)-alkyl substituiert sein kann,
und Gruppen der Formeln -OR¹⁴, -NR¹⁵R¹⁶ oder -CO-NR¹⁷R¹⁸ besteht,
worin R¹⁴ Phenyl bedeutet,. das. seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten, oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten, und deren Salze.

In einer weiteren bevorzugten Ausführungsform betrifft die Erfindung Verbindungen der allgemeinen Formel (I), worin
- R⁶: für Phenyl steht, das gegebenenfalls mit ein bis drei Substituenten substituiert sein kann, die aus der Gruppe ausgewählt werden, die aus Halogen, (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen und (C₁-C₆)-Alkyl besteht,
und deren Salze.

In einer weiteren Ausführungsform betrifft die Erfindung Verbindungen der Formel (I), worin
- R¹: für Wasserstoff, Halogen oder für (C₁-C₆)-Alkyl steht,
- R² und R³: gleich oder verschieden sind und für Wasserstoff oder (C₃-C₈)-Cycloalkyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Resten der Formel 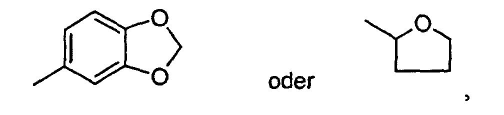 einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann, und
(C₆-C₁₀)-Aryl, das seinerseits durch Hydroxy oder (C₁-C₆)-Alkoxy substituiert sein kann, besteht, oder
- R² und R³: gemeinsam mit dem Stickstoffatom einen 5- bis 6-gliedrigen gesättigten Heterocyclus bildet, der gegebenenfalls noch ein Sauerstoffatom besitzen kann,
- R⁴: für Wasserstoff, (C₁-C₆)-Acyl, (C₂-C₆)-Alkenyl steht oder
- R⁴: für (C₁-C₆)-Alkyl steht, das gegebenfalls substituiert sein kann durch 1 bis 3 Substituenten, die aus der Gruppe ausgewählt werden, die aus Halogen, Hydroxy, (C₁-C₆)-Acyl, (C₁-C₆)-Alkoxy, Phenoxy, (C₆-C₁₀)-Aryl und -NR⁷R⁸ besteht,
worin R⁷ und R⁸ gleich oder verschieden sind und Wasserstoff, (C₁-C₆)-Acyl, (C₁-C₆)-Alkyl, Carbamoyl, Mono- oder Di(C₁-C₆)-alkylaminocarbonyl oder (C₁-C₆)-Alkoxycarbonyl bedeuten, oder R⁷ und R⁸ gemeinsam mit dem Stickstoffatom einen 5- bis 6- gliedrigen gesättigten Heterocyclus bilden, der gegebenenfalls ein weiteres Heteroatom aus der Reihe S oder O oder einen Rest der Formel -NR⁹ enthalten kann,
worin
R⁹ Wasserstoff oder (C₁-C₄)-Alkyl bedeutet, oder
- R⁴: für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 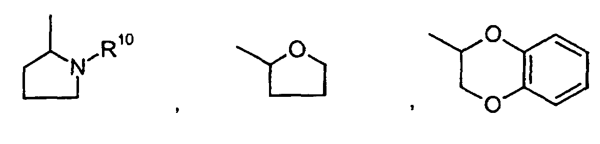 oder 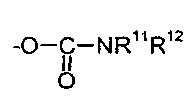 substituiert ist, worin
R¹⁰ Wasserstoff oder (C₁-C₆)-Alkyl bedeutet,
R¹¹ und R¹² gleich oder verschieden sind und Wasserstoff, (C₁-C₆)-Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
- R⁵: für Wasserstoff oder (C₁-C₆)-Alkyl steht,
- R⁶: für einen Rest der Formel 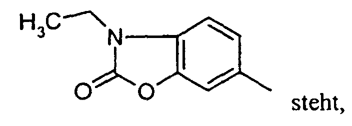
oder
- R⁶: für Phenyl steht, das gegebenenfalls mit ein bis zwei Substituenten substituiert sein kann, die aus der Gruppe ausgewählt werden, die aus
Halogen, (C₆-C₁₀)-Aryl, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Resten der Formeln 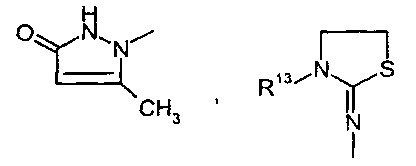 oder, 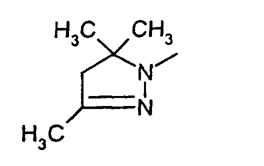 worin
R¹³ Wasserstoff oder (C₁-C₆)-Alkyl bedeutet, (C₁-C₆)-Alkyl, das gegebenenfalls durch einen Rest der Formel 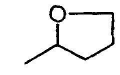 substituiert ist,
einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch ein bis drei Halogenatome substituiert sein kann,
einem gegebenenfalls über ein Stickstoffatom gebundenen 3- bis 8- gliedrigen gesättigten oder ungesättigten, nicht aromatischen Heterocyclus mit bis zu 3 Heteroatomen aus der Reihe S, N und/oder O der gegebenenfalls durch 1 bis 3 Substituenten ausgewählt aus Oxo, Halogen, Hydroxy, (C₁-C₆)-Alkoxycarbonyl, (C₁-C₆)-Alkyl, Halogen(C₁-C₆)-alkyl und Hydroxy(C₁-C₆)-alkyl substituiert sein kann,
und Gruppen der Formeln -OR¹⁴, -NR¹⁵R¹⁶ oder -CO-NR¹⁷R¹⁸ besteht,
worin
R¹⁴ einen Rest der Formel 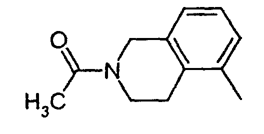 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten,
oder
- R¹⁴: (C₁-C₆)-Alkyl bedeutet, das gegebenenfalls ein- bis dreifach durch Hydroxy substituiert ist,
- R¹⁵ und R¹⁶: gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
- R¹⁷ und R¹⁸: gleich oder verschieden sind und Wasserstoff oder (C₁-C₆)-Alkyl bedeuten,
und deren Salze.

Bevorzugt sind Verbindungen der allgemeinen Formel (I),
in welcher
- R¹: für Wasserstoff, Chlor oder für (C₁-C₃)-Alkyl steht,
- R² und R³: gleich oder verschieden sind und für Wasserstoff oder Cyclopropyl oder Cyclopentyl stehen, oder
für (C₁-C₃)-Alkyl stehen, das gegebenenfalls durch 1 bis 3 Substituenten substituiert ist, die aus der Gruppe ausgewählt werden, die aus Cyclopropyl, Cyclopentyl, (C₁-C₃)-Alkoxy, Chlor, Fluor, Hydroxy, Resten der Formel 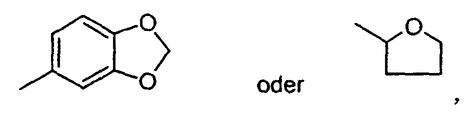 und Pyridyl, Furyl, Thienyl, Imidazolyl, N-Triazolyl oder Pyrrolyl, Phenyl, das seinerseits durch Hydroxy oder (C₁-C₃)-Alkoxy substituiert sein kann, besteht, oder
- R² und R³: gemeinsam mit dem Stickstoffatom einen Morpholin-, Piperidin- oder Pyrrolidinring bilden,
- R⁴: für Wasserstoff, (C₁-C₃)-Acyl, (C₂-C₃)-Alkenyl steht oder
- R⁴: für (C₁-C₆)-Alkyl steht, das gegebenfalls substituiert sein kann durch 1 bis 3 Substituenten, die aus der Gruppe ausgewählt werden, die aus Chlor, Fluor, Hydroxy, (C₁-C₃)-Acyl, (C₁-C₃)-Alkoxy, Phenoxy, Phenyl und -NR⁷R⁸ besteht,
worin R⁷ und R⁸ gleich oder verschieden sind und Wasserstoff, (C₁-C₄)-Acyl, (C₁-C₄)-Alkyl, Carbamoyl, Mono- oder Di(C₁-C₃)-alkylaminocarbonyl oder (C₁-C₄)-Alkoxycarbonyl bedeuten, oder
R⁷ und R⁸ gemeinsam mit dem Stickstoffatom einen Morpholino-, Piperidinyl- oder Pyrrolidinylring bilden, oder
- R⁴: für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 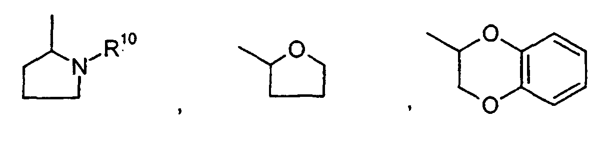 oder 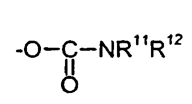 substituiert ist,
worin
R¹⁰ Wasserstoff oder (C₁-C₄)-Alkyl bedeutet,
R¹¹ und R¹² gleich oder verschieden sind und Wasserstoff, (C₁-C₃) Alkyl oder Phenyl bedeuten, wobei zuvor genanntes (C₁-C₃)-Alkyl und Phenyl gegebenenfalls durch 1 bis 3 Substituenten substituiert sein können, die aus der Gruppe ausgewählt werden, die aus Hydroxy, (C₁-C₃)-Alkoxy, Chlor und Fluor besteht,
- R⁵: für Wasserstoff oder (C₁-C₃)-Alkyl steht,
- R⁶: für Phenyl steht, das gegebenenfalls mit ein bis zwei Substituenten substituiert sein kann, die aus der Gruppe ausgewählt werden, die aus
Chlor, Fluor, Phenyl, (C₁-C₄)-Alkoxy, (C₁-C₄)-Alkoxycarbonyl, (C₁-C₃)-Alkylthio, Hydroxy, Carboxyl, partiell fluoriertem (C₁-C₄)-Alkoxy mit bis zu 5 Fluoratomen,
Resten der Formeln 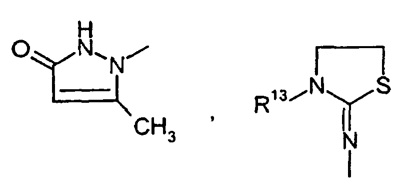 oder, 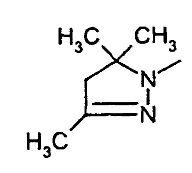 worin
R¹³ Wasserstoff oder (C₁-C₃)-Alkyl bedeutet, (C₁-C₆)-Alkyl, das gegebenenfalls durch einen Rest der Formel 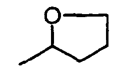 substituiert ist,
Triazolyl,
Morpholino, Thiomorpholino, Piperidinyl, Pyrrolidinyl, Azacycloheptanyl; Azacyclobutanyl, die gegebenenfalls durch 1 bis 2 Substituenten ausgewählt aus Oxo, Chlor, Fluor, Hydroxy, (C₁-C₄)-Alkoxycarbonyl, (C₁-C₃)-Alkyl, Chlor- oder Fluor(C₁-C₃)-alkyl und Hydroxy(C₁-C₄)-alkyl substituiert sein können, 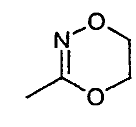 und Gruppen der Formeln -OR¹⁴, -NR¹⁵R¹⁶ oder -CO-NR¹⁷R¹⁸ besteht,
worin
R¹⁴ einen Rest der Formel 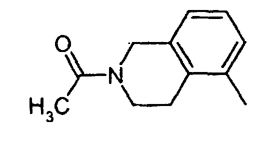 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₃)-Alkyl oder (C₁-C₃)-Acyl bedeuten, oder
- R¹⁴: (C₁-C₄)-Alkyl bedeutet, das gegebenenfalls ein- bis dreifach durch Hydroxy substituiert ist,
- R¹⁵ und R¹⁶: gleich oder verschieden sind und Wasserstoff, Carbamoyl, Mono- oder Di(C₁-C₃)alkylaminocarbonyl, Phenyl, (C₁-C₃)-Acyl oder (C₁-C₃)-Alkyl bedeuten, wobei (C₁-C₃)-Alkyl gegebenenfalls durch (C₁-C₃)-Alkoxy, (C₁-C₃)-Acyl, durch Phenyl oder Pyridyl substituiert ist,
wobei zuvor genanntes Phenyl und zuvor genanntes Pyridyl gegebenenfalls ein- bis zweifach gleich oder verschieden durch Chlor, Fluor und/oder Hydroxy substituiert sind, und
- R¹⁷ und R¹⁸: gleich oder verschieden sind und Wasserstoff oder (C₁-C₄)-Alkyl bedeuten,
und deren Salze.

In einer bevorzugten Ausfuhrungsfbrm der Erfindung ist R⁵ in den Verbindungen der allgemeinen Formel (I) Wasserstoff.

In einer weiteren bevorzugten Ausführungsform der Erfindung sind R² und R³ in den Verbindungen der allgemeinen Formel (1) Wasserstoff.

In einer weiteren bevorzugten Ausführungsform der Erfindung ist R⁶ in den Verbindungen der allgemeinen Formel (I) eine para-substituierte Phenylengruppe, die gegebenenfalls einen weiteren Substituenten aufweisen kann. D.h. die Verbindungen weisen die Formel 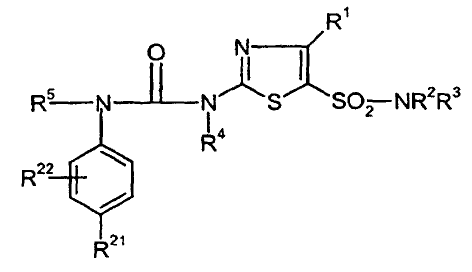 auf, worin
- R¹ bis R⁵: jeweils wie oben definiert sind und R²¹ steht für:
Halogen, (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylarninocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Reste der Formeln 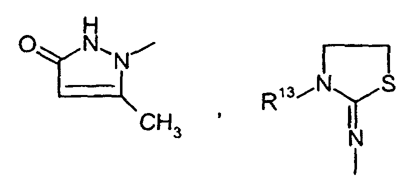 oder, 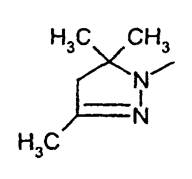 worin
R¹³ Wasserstoff oder (C₁-C₆)-Alkyl bedeutet, (C₁-C₆)-Alkyl, das gegebenenfalls durch einen Rest der Formel 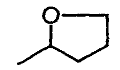 substituiert ist,
einen gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₂-C₆)Alkylsulfoxy, (C₁-C₆)Alkylsulfonyl, und/oder Cyano substituiert sein kann,
einen gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen(C₁-C₆)-alkyl und Hydroxy(C₁-C₆)-alkyl substituiert sein kann,
und Gruppen der Formeln -OR¹⁴, -NR¹⁵R¹⁶ oder -CO-NR¹⁷R¹⁸,
worin
R¹⁴ einen Rest der Formel 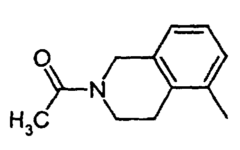 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten, oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten, und Salze davon
R²² kann dabei die obige Bedeutung von R²¹ aufweisen und mit dieser gleich oder verschieden sein oder kann für Wasserstoff stehen, d.h. der Phenylrest ist nur in para-Stellung substituiert. R²² ist bevorzugt Wasserstoff.

In einer weiteren bevorzugten Ausführungsform betrifft die Erfindung Verbindungen, die die folgende Formel aufweisen: 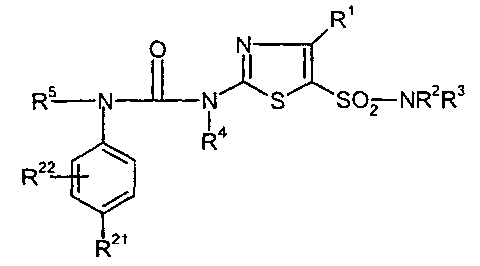 worin
- R¹ bis R⁵: wie oben definiert sind und R²¹ für
Halogen, (C₆-C₁₀)-Aryl, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Reste der Formeln 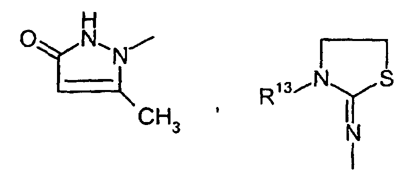 oder, 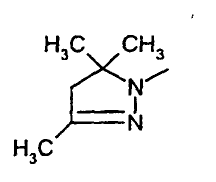 worin
R¹³ Wasserstoff oder (C₁-C₆)-Alkyl bedeutet, (C₁-C₆)-Alkyl, das gegebenenfalls durch einen Rest der Formel 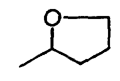 substituiert ist,
einen gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch ein bis drei Halogenatome substituiert sein kann,
einen gegebenenfalls über ein Stickstoffatom gebundenen 3- bis 8- gliedrigen gesättigten oder ungesättigten, nicht aromatischen Heterocyclus mit bis zu 3 Heteroatomen aus der Reihe S, N und/oder O der gegebenenfalls durch 1 bis 3 Substituenten ausgewählt aus Oxo, Halogen, Hydroxy, (C₁-C₆)-Alkoxycarbonyl, (C₁-C₆)-Alkyl, Halogen(C₁-C₆)-alkyl und Hydroxy(C₁-C₆)-alkyl substituiert sein kann,
oder für Gruppen der Formeln -OR¹⁴, NR¹⁵R¹⁶ oder -CO-NR¹⁷R¹⁸ steht,
worin
R¹⁴ einen Rest der Formel 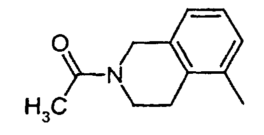 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten, oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten, und
- R²²: die obige Bedeutung von R²¹ aufweisen kann und mit dieser gleich oder verschieden sein kann oder R²² Wasserstoff ist.

R²² kann dabei die obige Bedeutung von R²¹ aufweisen und mit dieser gleich oder verschieden sein oder kann für Wasserstoff stehen, d.h. der Phenylrest ist nur in paraStellung substituiert. R²² ist bevorzugt Wasserstoff.

R²¹ steht bevorzugt für Phenyl, (C₁-C₄)-Alkoxy oder einen gegebenenfalls über ein Stickstoffatom gebundenen 3- bis 8- gliedrigen gesättigten oder ungesättigten, nicht aromatischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkyl, Halogen(C₁-C₆)-alkyl und Hydroxy(C₁-C₆)-alkyl substituiert sein kann.

Die Erfindung betrifft ferner Zwischenprodukte zur Herstellung der Verbindungen der allgemeinen Formel (I), die die Formel (II) aufweisen: 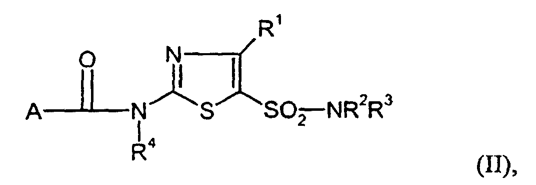 worin R¹, R², R³ und R⁴ die oben angegebene Bedeutung haben, und A für ein Halogenatom steht.

Außerdem wurde ein Verfahren zur Herstellung der erfindungsgemäßen Verbindungen der allgemeinen Formel (I) gefunden, dadurch gekennzeichnet, dass man
[A] Verbindungen der allgemeinen Formel (II) 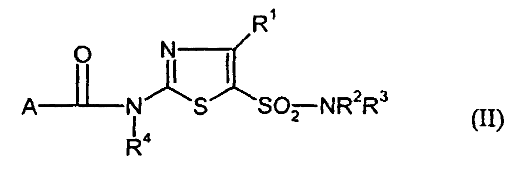 in welcher
   R¹, R², R³ und R⁴ die oben angegebene Bedeutung haben,
   und
   - A: für Halogen, vorzugsweise für Chlor steht,
   mit Aminen der allgemeinen Formel (III)

   HNR⁵R⁶ (III)

   in welcher
   R⁵ und R⁶ die oben angegebene Bedeutung haben,
   in inerten Lösemitteln, gegebenenfalls in Anwesenheit einer Base und/oder Hilfsmittels umsetzt,
   oder
[B] Isocyanate der allgemeinen Formel (IV)

   R⁶-NCO (IV)
in welcher
- R⁶: die oben angegebene Bedeutung hat,
mit Thiazolylaminen der allgemeinen Formel (V) 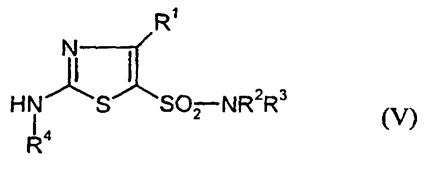 in welcher
R¹, R², R³ und R⁴ die oben angegebene Bedeutung haben,
in inerten Lösemitteln umsetzt, und im Falle, dass R⁵ von Wasserstoff verschieden ist, eine Alkylierung nach üblichen Verfahren durchführt.

Die erfindungsgemäßen Verfahren können durch folgende Formelschemata beispielhaft erläutert werden:

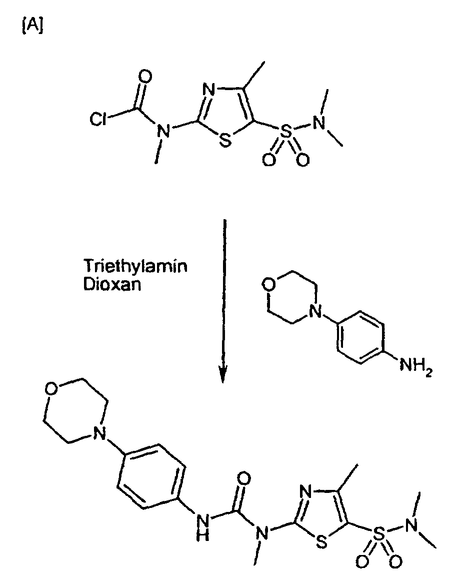

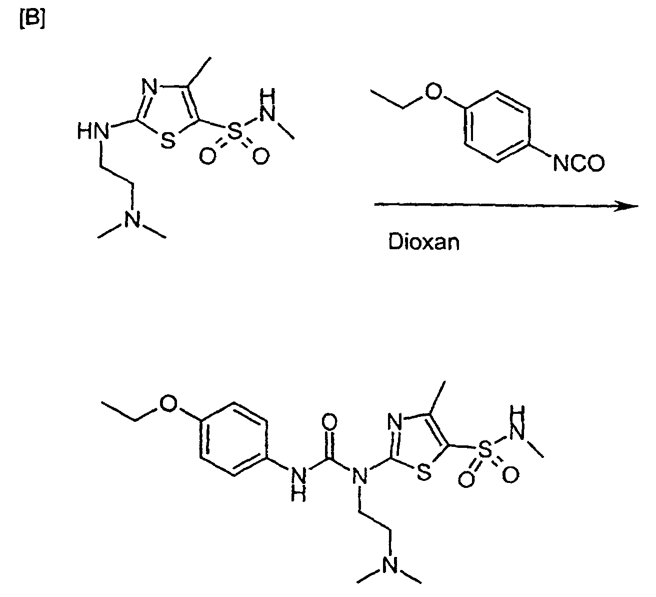

Als Lösemittel für die Verfahren [A] und [B] eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid oder Acetonitril. Ebenso ist es möglich, Gemische der genannten Lösemittel zu verwenden. Bevorzugt ist Dioxan.

Als Basen für das erfindungsgemäße Verfahren [A] können im allgemeinen anorganische oder organische Basen eingesetzt werden. Hierzu gehören vorzugsweise organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Bevorzugt ist Triethylamin.

Im allgemeinen setzt man die Base in einer Menge von 0,05 Mol bis 10 Mol, bevorzugt von 1 Mol bis 2 Mol bezogen auf 1 Mol der Verbindung der Formel (II) ein.

Die erfindungsgemäßen Verfahren werden im allgemeinen in einem Temperaturbereich von -50°C bis +100°C, bevorzugt von -30°C bis +60°C, durchgeführt.

Die erfindungsgemäßen Verfahren werden im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (II) sind neu und können hergestellt werden, indem man

Verbindungen der allgemeinen Formel (VI) 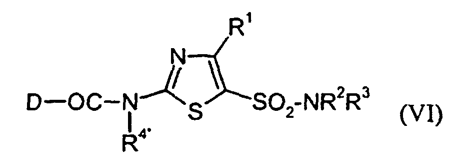 in welcher
R', R² und R³ die oben angegebene Bedeutung haben
und
- D: für (C₁-C₄)-Alkyl, vorzugsweise für Methyl steht,
zunächst durch eine Alkylierung mit Verbindungen der allgemeinen Formel (VII)

R^{4'}-J (VII)

in welcher
- R^{4'}: die oben angegebene Bedeutung von R⁴ hat, aber nicht für Wasserstoff steht,
in inerten Lösemitteln und in Anwesenheit einer Base in die Verbindungen der allgemeinen Formel (VIII) 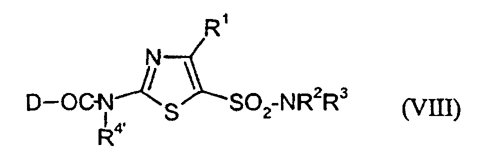 in welcher
R¹, R², R³, R⁴' und D die oben angegebene Bedeutung haben,
überführt,
in einem weiteren Schritt durch Umsetzung mit Salzsäure die Verbindungen der allgemeinen Formel (IX) 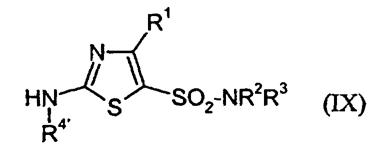 in welcher
R¹, R², R³ und R^{4'} die oben angegebene Bedeutung haben,
herstellt und abschließend mit Chlorameisensäuretrichlormethylester in Ethern umsetzt.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether oder Kohlenwasserstoffe wie Benzol, Toluol, Xylol, Hexan, Cyclohexan oder Erdölfraktionen oder Halogenkohlenwasserstoffe wie Dichlormethan, Trichlormethan, Tetrachlormethan, Dichlorethylen, Trichlorethylen oder Chlorbenzol oder Essigester oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dichlormethan, Dimethylsulfoxid und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natriumoder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt ist Natriumhydrid.

Die Alkylierung erfolgt im allgemeinen in einem der oben aufgeführten Lösemitteln, vorzugweise in Dimethylformamid in einem Temperaturbereich von 0°C bis +70°C, vorzugsweise von 0°C bis +30°C und Normaldruck.

Im allgemeinen setzt man die Base in einer Menge von 0,05 Mol bis 10 Mol, bevorzugt von 1 Mol bis 2 Mol bezogen auf 1 Mol der Verbindung der Formel (VI) ein.

Die Herstellung der Verbindungen der allgemeinen Formel (IX) erfolgt unter Rückflusstemperatur und Normaldruck.

Als Lösemittel für die Umsetzung mit Chlorameisensäuretrichlormethylester eignen sich im allgemeinen Ether wie Diethylether, Dioxan, Tetrahydrofuran oder Glykoldimethylether. Bevorzugt ist Dioxan.

Die Umsetzung von Verbindungen der allgemeinen Formel (IX) mit Chlorameisensäuretrichlormethylester erfolgt zunächst bei Raumtemperatur und anschließend unter der Rückflusstemperatur des jeweiligen Ethers.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (VI) sind an sich bekannt [vgl. DE 748376] oder können nach den dort publizierten Methoden hergestellt werden.

Die Verbindungen der allgemeinen Formel (VII) sind bekannt.

Die Verbindungen der allgemeinen Formeln (VIII) und (IX) sind neu und können wie oben beschrieben hergestellt werden.

Die Verbindungen der allgemeinen Formel (V) sind neu und können beispielsweise hergestellt werden, indem man

Verbindungen der allgemeinen Formel (X) 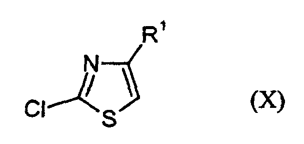 in welcher
- R¹: die oben angegebene Bedeutung hat,
durch Umsetzung mit dem System Chlorsulfonsäure/SOCl₂ in die Verbindungen der allgemeinen Formel (XI) 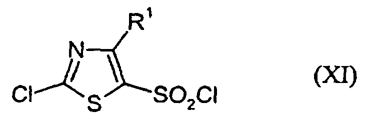 in welcher
- R¹: die oben angegebene Bedeutung hat,
überführt, anschließend mit Aminen der allgemeinen Formel (XII)

HNR²R³ (XII)

in welcher
R² und R³ die oben angegebene Bedeutung haben,
in inerten Lösemitteln die Verbindungen der allgemeinen Formel (XIII) 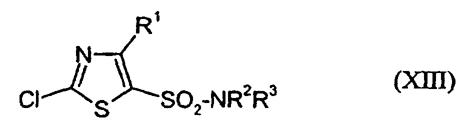 in welcher
R¹, R² und R³ die oben angegebene Bedeutung haben,
herstellt, und in einem letzten Schritt eine Umsetzung mit Aminen der allgemeinen Formel (XIV)

H₂N-R^{4''} (XIV)

in welcher
- R^{4''}: die oben angegebene Bedeutung von R^{4'} hat und mit dieser gleich oder verschieden ist,
in inerten Lösemitteln und in Anwesenheit einer Base durchführt.

Die Reaktion mit Chlorsulfonsäure/SO₂Cl erfolgt zunächst bei Raumtemperatur und anschließend unter der Rückflusstemperatur des jeweiligen Ethers.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Lösemittel für die Umsetzung mit den Aminen der allgemeinen Formel (XII) eignen sich Alkohole wie beispielsweise Methanol, Ethanol, Propanol und Isopropanol. Bevorzugt ist Methanol.

Die Umsetzung mit den Aminen der allgemeinen Formel (XIII) erfolgt zunächst bei Raumtemperatur und anschließend unter der Rückflusstemperatur des jeweiligen Ethers.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Umsetzung mit den Verbindungen der allgemeinen Formel (XIV) erfolgt in Ethem wie beispielsweise Diethylether, Dioxan, Tetrahydrofuran oder Glykoldimethylether. Bevorzugt ist Methanol.

Als Basen können im allgemeinen anorganische oder organische Basen eingesetzt werden. Hierzu gehören vorzugsweise organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Bevorzugt ist Triethylamin.

Im allgemeinen setzt man die Base in einer Menge von 0,05 Mol bis 10 Mol, bevorzugt von 1 Mol bis 2 Mol bezogen auf 1 Mol der Verbindung der Formel (XIII) ein.

Die Verbindungen der allgemeinen Formel (X) sind teilweise bekannt oder. nach üblichen Methoden herstellbar [vgl. Hantzsch, Chem. Ber. 1927,60, 2544].

Die Verbindungen der allgemeinen Formel (XI) und (XIII) sind neu und können wie oben beschrieben hergestellt werden.

Die Amine der allgemeinen Formeln (XII) und (XIV) sind bekannt.

Die Verbindungen der allgemeinen Formeln (III) und (IV) sind literaturbekannt.

Biphenylaminderivate der Formel (III) aus denen sich auch die entsprechenden Isocyanate (IV) herstellen lassen, lassen sich nach bekannten, übergangsmetallkatalysierten Kupplungsreaktionen wie z.B. der Suzuki- oder Stille-Kupplung herstellen. Das folgende Reaktionsschema illustriert beispielhaft die Synthese eines Biphenylamin-Derivates durch die an sich bekannte palladiumkatalysierte Suzuki-Kupplungsreaktion eines Halogenaromaten mit der jeweiligen Boronsäure.

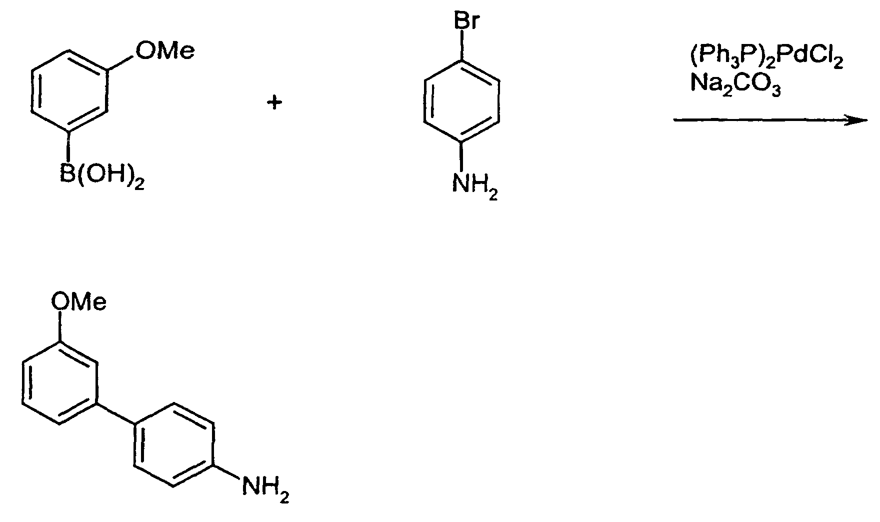

Die Pyridylphenylaminderivate der Formel (III) sind literaturbekannt.

Die Erfindung betrifft ferner die Verwendung der 2-[(Aminocarbonyl)amino]-1,3-thiazol-5-sulfonamid-Derivate zur Herstellung von Arzneimitteln. 2-[(Aminocarbonyl)amino]-1,3-thiazol-5-sulfonamid-Derivate sind solche Verbindungen, die sich aus der Substitution eines oder mehrerer Wasserstoffatome aus 2-[(Aminocarbonyl)amino]-1,3-thiazol-5-sulfonamid ableiten. Bevorzugt werden die 2-[(Aminocarbonyl)amino]-1,3-thiazol-5-sulfonamid-Derivate zur Herstellung von Mitteln zur Behandlung und/oder Prävention von viralen Infektionen bei Menschen oder Tieren, besonders bevorzugt zur Herstellung von Mitteln zur Behandlung und/oder Prävention von Infektionen bei Menschen oder Tieren durch Herpes-Viren verwendet.

Die erfindungsgemäßen Verbindungen der allgemeinen Formeln (I etc.) zeigen ein nicht vorhersehbares überraschendes Wirkspektrum. Sie zeigen eine antivirale Wirkung gegenüber Vertretern der Gruppe Herpes viridae, besonders gegenüber den Herpes Simplex Viren (HSV). Sie sind somit zur Behandlung und Prophylaxe von Erkrankungen, die durch Herpes-Viren, insbesondere Erkrankungen, die durch Herpes Simplex Viren bei Menschen oder Tieren hervorgerufen werden.

### In vitro-Aktivität

### Viren und Zellen:

HSV (HSV-1 Walki, HSV-1F oder HSV-2G) wurde auf Vero-Zellen (ATCC CCL-81) unter folgenden Bedingungen vermehrt: Die Zellen wurden in M199 Medium (5 % fötales Kälberserum, 2mM Glutamin, 100IU/ml Penicillin, 100µg/ml streptomycin) in Zellkulturflaschen bei 37°C und 5 % CO₂ gezüchtet. Die Zellen wurden zweimal pro Woche jeweils 1:4 gesplittet. Für die Infektion wurde das Medium abgenommen, die Zellen mit "Hank's solution" gewaschen, mit 0.05 %Trypsin, 0.02 %EDTA (Seromed L2143) abgelöst und mit einer Dichte von 4x10⁵ Zellen pro ml unter den oben genannten Bedingungen für 24 Stunden inkubiert. Dann wurde das Medium abgenommen und die Viruslösung mit einer m.o.i von < 0.05 in einem Volumen von 2 ml pro 175 cm² Oberfläche dazugegeben. Nach einstündiger Inkubation unter den genannten Bedingungen wurde das Medium auf ein Volumen von 50 ml pro 175 cm² -Flasche aufgefüllt. 3 Tage nach Infektion zeigten die Kulturen deutliche Zeichen eines zytopathischen Effektes. Das Virus wurde durch zweimaliges Frieren (-80°C) und Tauen (37°C) freigesetzt. Der Zelldebris wurde durch Zentrifugation (300g, 10min, 4°C) abgetrennt und der Überstand in Aliquots bei -80°C weggefroren.

Der Virustiter wurde über einen Plaque-Assay bestimmt. Dafür wurden Verozellen in einer Dichte von 4x10⁵ Zellen pro Vertiefung in 24 well Platten ausgesät und nach 24 Stunden Inkubation (37°C, 5 % CO₂) mit Verdünnungen des Virusstocks von 10⁻² bis 10⁻¹² (100µl Inokulum) infiziert. 1 Stunde nach Infektion wurde das Medium abgenommen und die Zellen mit 1 ml Overlay-Medium (0.5 % Methylzellulose, 0.225 Natriumbikarbonat, 2mM Glutamin, 100 IU/ml Penicillin, 100µg/ml Streptomycin, 5 % fötales Kälberserum in MEM-Eagle Medium mit Earl's Salz) überschichtet und für 3 Tage inkubiert. Im Anschluss wurden die Zellen mit 4 % Formalin für 1 Stunde fixiert, mit Wasser gewaschen, mit Giemsa (Merck) für 30 min gefärbt und im Anschluss gewaschen und getrocknet. Mit einem Plaque-viewer wurde der Virustiter bestimmt. Die für die Experimente verwendeten Virusstocks hatten einen Titer von 1x10⁶/ml - 1x10⁸/ml.

Die Anti-HSV-Wirkung wurde in einem Screening-Testsystem in 96-Well-Mikrotiterplatten unter Zuhilfenahme von diversen Zellinien neuronalen, lymphoiden und epithelialen Ursprungs wie zum Beispiel Vero (Nierenzellinie der grünen Meerkatze), MEF (murine embryonale Fibroblasten), HELF (humane embryonale Fibroblasten), NT2 (humane neuronale Zellinie) oder Jurkat (humane lymphoide T-Zellinie) bestimmt. Der Einfluss der Substanzen auf die Ausbreitung des cytopathogenen Effektes wurde im Vergleich zu der Referenzsubstanz Acyclovir-Natrium (Zovirax^{R}), einem klinisch zugelassenen anti-Herpes-Chemotherapeutikum, bestimmt.

Die in DMSO (Dimethylsulfoxid) gelösten Substanzen (50 mM) werden auf Mikrotiterplätten (z.B. 96-Well MTP) in Endkonzentrationen von 250 -0,5 µM (mikromolar) in Doppelbestimmungen (4 Substanzen/Platte) untersucht. Bei potenten Substanzen werden die Verdünnungen über mehrere Platten bis 0,5 pM (picomolar) weitergeführt. Toxische und cytostatische Substanzwirkungen werden dabei miterfasst. Nach den entsprechenden Substanzverdünnungen (1:2) auf der Mikrotiterplatte wird eine Suspension von Zellen (1x10⁴ Zellen pro Vertiefung) wie zum Beispiel von Vero-Zellen in M199 (Medium 199) mit 5 % fötalem Kälberserum, 2 mM Glutamin und optional 100 IU/ml Penicillin und 100 µg/ml Streptomycin oder MEF-Zellen in EMEM (Eagle's Minimum Essential Medium) mit 10 % fötalem Kälberserum, 2 mM Glutamin und optional 100 IU/ml Penicillin und 100 µg/ml Streptomycin, oder HELF-Zellen in EMEM mit 10 % fötalem Kälberserum, 2 mM Glutamin und optional 100 IU/ml Penicillin und 100 µg/ml Streptomycin, oder NT2- und Jurkat-Zellen in DMEM (4,5 mg/l Glukose plus Pyridoxin) mit 10 % fötalem Kälberserum 2 mM Glutamin, 1 mM Natrium Pyruvat, nicht essentiellen Aminosäuren und optional 100 IU/ml Penicillin und 100 µg/ml Streptomycin in jedes Näpfchen gegeben und die Zellen in den relevanten Vertiefungen mit einer entsprechenden Virusmenge infiziert (HSV-1 F oder HSV-2 G mit einer m.o.i (multiplicity of infection) von 0,0025 für HELF, Vero und MEF Zellen sowie einer m.o.i von 0,1 für NT2- und Jurkat-Zellen). Die Platten werden anschließend bei 37 °C in einem CO₂-Brutschran (5 % CO₂) über mehrere Tage inkubiert. Nach dieser Zeit ist der Zellrasen von z.B. Vero-Zellen in den substanzfreien Viruskontrollen, ausgehend von 25 infektiösen Zentren, durch den cytophatogenen Effekt der HSV-Viren völlig lysiert bzw. zerstört (100 % CPE). Die Platten werden zunächst optisch mit Hilfe eines Mikroskopes ausgewertet und dann mit einem Fluoreszenzfarbstoff analysiert. Hierzu wird der Zellkulturüberstand aller Näpfchen der MTP abgesaugt und mit 200 µl PBS-Waschlösung befüllt. Das PBS wird abermals abgesaugt und alle Wells mit 200 µl Fluoreszenzfarbstofflösung (Fluorescein-diacetate, 10 µg/ml in PBS) befüllt. Nach einer Inkubationszeit von 30-90 min werden die Testplatten in einem Fluoreszenzmessgerät bei einer Anregungswellenlänge von 485 nm und einer Emissionswellenlänge von 538 nm vermessen.

Die Ergebnisse sind für einige Verbindungen in der folgenden Tabelle zusammengefasst:

**Tabelle**

| **Beispiel** | **IC**_{**50**} **HSV-1 F/Vero** | **IC**_{**50**} **HSV-2 G/Vero** |
|---|---|---|
| 43 | 0,5 µM | 1,5 µM |
| 123 | 1 nM | 5 nM |
| 94 | 20 nM | 50 nM |
| 2 | 0,2 µM | 1 µM |
| Zovirax | 1 µM | 3 µM |
| (Aciclovir-Natrium) | | |

IC₅₀ bedeutet hier die halbmaximale Fluoreszenzintensität mit Bezug zur nicht infizierten Zellkontrolle.

Die 2-[(Aminocarbonyl)amino)-1,3-thiazol-5-sulfonamid-Derivate der Erfindung weisen bevorzugt einen IC₅₀ von weniger als 50 µM, bevorzugter von weniger als 25 µM und ganz besonders bevorzugt von weniger als 10 µM auf.
Die erfindungsgemäßen Verbindungen stellen somit wertvolle Wirkstoffe zur Behandlung und Prophylaxe von Erkrankungen dar, die durch Herpes Virus, insbesondere Herpes simplex Virus ausgelöst werden. Als Indikationsgebiete können beispielsweise genannt werden :
1) Behandlung und Prophylaxe von Herpes-Infektionen, insbesondere Herpes simplex-Infektionen bei Patienten mit Krankheitsbildern wie Herpes labialis, Herpes genitalis, und HSV bedingter Keratitis, Enzephalitis, Pneumonie, Hepatitis etc.
2) Behandlung und Prophylaxe von Herpes-Infektionen, insbesondere Herpes simplex-Infektionen bei immunsupprimierten Patienten (z.B.AIDS-Patienten, Krebspatienten, Patienten mit genetisch bedingter Immundefiziens, Transplantationspatienten)
3) Behandlung und Prophylaxe von Herpes-Infektionen, insbesondere Herpes simplex-Infektionen bei Neugeborenen und Kleinkindern
4) Behandlung und Prophylaxe von Herpes-Infektionen, insbesondere Herpes simplex-Infektionen und Herpes-, insbesondere Herpes simplex-positiven Patienten zur Unterdrückung der Rekurrenz (Suppressionstherapie)

### In vivo-Wirkung

### Tiere:

6 Wochen alte weibliche Mäuse, Stamm BALB/cABom, wurden von einem kommerziellen Züchter (Bomholtgard Breeding and Research Centre Ltd.) bezogen.

### Infektion:

Die Tiere wurden in einem dichten Glasgefäß mit Diethylether (Merck) anästhesiert. 50µl einer Verdünnung des Virusstocks (Infektionsdosis 5x10⁴ Pfu) wurden mit einer Eppendorfpipette in die Nase der anästhesierten Tiere eingebracht. Diese Infektionsdosis führt bei 90-100 % der Tiere durch eine generalisierte Infektion mit prominenten respiratorischen und zentralnervösen Symptomen im Mittel zwischen 5 und 8 Tagen zum Tode.

### Behandlung und Auswertung:

6 Stunden nach Infektion wurden die Tiere mit Dosen von 0,1-100 mg/kg Körpermasse 3 mal täglich 7.00 Uhr, 14.00 Uhr und 19 Uhr über einen Zeitraum von 5 Tagen behandelt. Die Substanzen wurden in DMSO vorgelöst und in Tylose/PBS(Hoechst) resuspendiert (Endkonzentration 1,5 % DMSO, 0,5 % Tylose in PBS).

Nach der letzten Applikation wurden die Tiere weiter beobachtet und die Todeszeitpunkte festgestellt.

Ein Vergleich der Überlebenskurven erbrachte für die Verbindung des Beispiels 43 z.B eine ED₅₀ von 30-40 mg/kg, wobei ED₅₀ bedeutet, das bei dieser Dosis 50 % der Tiere überleben.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe und Lösemittel. Hierbei soll die therapeutisch wirksame Verbindung jeweils in einer Konzentration von etwa 0,5 bis 90 Gew. % der Gesamtmischung vorhanden sein, d.h. in Mengen, die ausreichend, sind um den angegebenen Dosierungsspielraum zu erreichen.

Die Formulierungen werden beispielsweise hergestellt durch Verstrecken der Wirkstoffe mit Lösemitteln und/oder Trägerstoffe, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral, parenteral oder topisch, insbesondere perlingual oder intravenös.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 20 mg/kg, vorzugsweise etwa 0,01 bis 10 mg/kg Körpergewicht zur Erzielung wirksamer Ergebnisse zu verabreichen, und bei oraler Applikation beträgt die Dosierung etwa 0,01 bis 30 mg/kg, vorzugsweise 0,1 bis 20 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Gegebenenfalls kann es sinnvoll sein, die erfindungsgemäßen Verbindungen mit anderen Wirkstoffen zu kombinieren.

### Ausgangsverbindungen

### Beispiel I

### N-{5-[(Dimethylamino)sulfonyl]-4-methyl-1,3-thiazol-2-yl}-N-methylacetamid

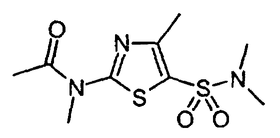

6,0 g Natriumhydrid (60 % Dispersion in Mineralöl, 0,151 mol) wurden in kleinen Portionen bei 0°C zu einer Lösung aus 36,2 g (0,137 mo1) N-{5-[(Dimethylamino)-sulfonyl]-4-methyl-1,3-thiazol-2-yl}acetamid in Dimethylform-amid zugetropft. Anschließend werden 21,4 g (0,151 mol) Methyliodid über eine Spritze zugetropft. Nach vollständigem Umsatz laut DC wurde der Ansatz durch Zugabe einer gesättigten Ammoniumchlorid Lösung gequencht. Die Mischung wurde bis zur Trockne eingeengt, der Rückstand in Wasser aufgenommen, 16 h gerührt und abfiltriert. Nach Trocknen des Rückstands im Vakuum wurde er weiter gereinigt durch Chromatographie an Kieselgel mit Toluol/Essigester (15-66 % Essigester) als Laufmittel. Es wurden 31,0 g eines weißen Pulvers erhalten (Rf = 0,43 (Toluol/Essigester=1/2), Ausbeute 74 %).

### Beispiel II

### N,N,4-Trimethyl-2-(methylamino)-1,3-thiazol-5-sulfonamid

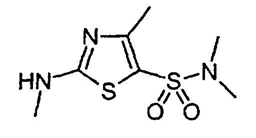

31g (0,111mol) N-{5-[(Dimethylamino)sulfonyl]-4-methyl-1,3-thiazol-2-yl}-N-methylacetamid wurden in 500 ml 4N Salzsäure suspendiert und zum Rückfluss erhitzt bis die Reaktion laut DC (Toluol/Essigester 1/2) vollständig war. Die Mischung wurde mit Dichlormethan gewaschen, durch Zugabe von 20 %-iger Natronlauge basisch gestellt und mit Dichlormethan extrahiert. Danach wurde die organische Phase über Natriumsulfat getrocknet und eingeengt. Das Rohprodukt wurde weiter gereinigt durch Chromatographie an Kieselgel mit Toluol/Essigester (50-85 % Essigester) als Laufmittel. Nach Abdestillieren des Lösungsmittels wurden 22,9 g eines Feststoffs erhalten. (Rf = 0,37 (Toluol/Essigester = 1/2), Ausbeute 87,7 %)

### Beispiel III

### 5-[(Dimethylamino)sulfonyl]-4-methyl-1,3-thiazol-2-yl(methyl)carbamidsäurechlorid

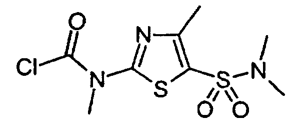

15,4 g (78 mmol) Chlorameisensäuretrichlormethylester("Diphosgen") wurden bei Raumtemperatur zu einer Lösung aus 22,9 g (97 mmol) N,N,4-Trimethyl-2-(methylamino)-1,3-thiazol-5-sulfonamid in 360 ml Dioxan gegeben und die Mischung wurde zum Rückfluss erhitzt bis eine homogene Lösung erhalten wurde und laut DC kein Startmaterial mehr vorhanden war. Nach Entfernen des Lösungsmittels im Vakuum wurden 29,0 g des Produktes in Form eines Öl erhalten.(Ausbeute 100 %, NMR (CDCl₃): 3.89 (3H), 2.81 (6H), 2.62 (3H) ppm).

### Beispiel IV

### 2-Chlor-4-methyl-1,3-thiazol-5-sulfonylchlorid

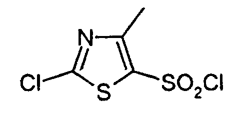

150 g (1,12mol) 2-Chlor-4-methyl-1,3-thiazol werden bei Raumtemperatur zu einer Lösung von 331 g (2,81 mmol) Thionylchlorid in 653 g (5,61 mmol) Chlorsulfonsäure zugetropft. Die Lösung wird 48 h zum Rückfluss erhitzt. Anschließend wird die Mischung auf 3 1 Eiswasser gegeben und mit 4x400 ml Dichlormethan extrahiert. Die vereinigten organischen Phasen werden mit 2,5 1 Wasser gewaschen, über Natriumsulfat getrocknet und eingeengt. Nach Destillation des Rohproduktes werden 233,7 g Produkt in Form eines Öls erhalten. (Sdp 87-96°C, 0,7 mbar, Ausbeute 89,6 %).

### Beispiel V

### 2-Chlor-N,4-dimethyl- 1,3-thiazol-5-sulfonamid

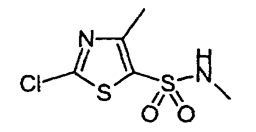

Zu einer Lösung aus 41 g (0,177mol) 2-Chlor-4-methyl-1,3-thiazol-5-sulfonylchlorid in 360 ml Dichlormethan werden 177 ml einer Lösung von Methylamin in Methanol (2 M, 0,354 mol) bei 0°C zugegeben. Man läßt 30 min bei 0°C rühren, gibt 1,8 l Wasser zu und extrahiert die Mischung 5 mal mit jeweils 400 ml Dichlormethan. Die vereinigten organischen Phasen werden über Natriumsulfat getrocknet und das Lösungsmittel abdestilliert. Es werden 39,93 g eines Öls erhalten, das beim Stehenlassen fest wird (Rf = 0,43 (Toluol / Essigester = 2/1), Ausbeute 99,7 %)

### Beispiel VI

### 2- {[2-(Dimethylamino)ethyl]amino}-N,4-dimethyl-1,3-thiazol-5-sulfonamid

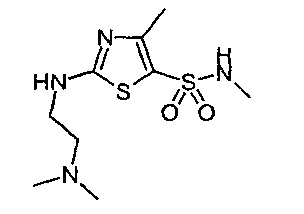

Eine Lösung von 5,0 g (22,1 mmol) 2-Chlor-N,4-dimethyl-1,3-thiazol-5-sulfonamid, 2,92 g (33,1 mmol) 2-N,N-Dimethylaminoethylamin und 6,10 ml Triethylamin in 6,65 ml Dioxan wird 16 h bei 100°C gerührt. Anschließend werden 20 ml Wasser zugegeben und die Mischung mit 3x100 ml Dichlormethan extrahiert. Die vereinigten organischen Phasen werden über Natriumsulfat getrocknet und eingeengt.

Nach Chromatographie des Rohprodukts an Kieselgel mit einer Mischung aus Chloroform und Methanol als Laufmittel (0-30 % Methanol) werden 3,05 g eines Öls erhalten. (Rf= 0,04 (Chloroform / 5 % Methanol), Ausbeute 49,7 %).

### Herstellungsbeispiele

### Beispiel 1

### N,N,4-Trimethyl-2-{methyl[4-morpholinoanilino)carbonyl]amino}-1,3-thiazol-5-sulfonamid

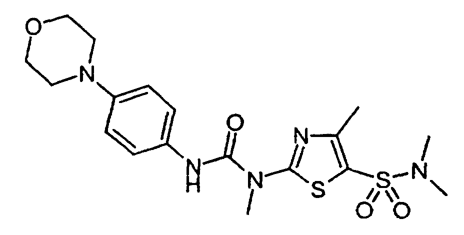

12,0 g (40,3 mmol) 5-[(Dimethylarnino)sulfonyl)-4-methyl-1,3-thiazol-2-yl(methyl)-carbamidsäurechlorid und 4,08 g (40,3 mmol) Triethylamin wurden in 150 ml Dioxan gelöst und 7,18 g (40,3 mmol) 4-Morpholinoanilin, gelöst in 60 Ml-Dioxan, zugetropft. Die Mischung wurde für 12 h bei Raumtemperatur gerührt, der entstandene Niederschlag abfiltriert und in einem Lösungsmittelgemisch aus Dichlormethan/Wasser (1:1) gelöst. Die organische Phase wurde abgetrennt, über Natriumsulfat getrocknet und im Vakuum eingeengt.
Ausbeute: 12,4 g (70 %)
Fp.: 191°C

### Beispiel 2

### 2-{[2-(Dimethylamino)ethyl][(4-ethoxyanilino)carbonyl]amino}-N,4-dimethyl-1,3-thiazol-5-sulfonamid

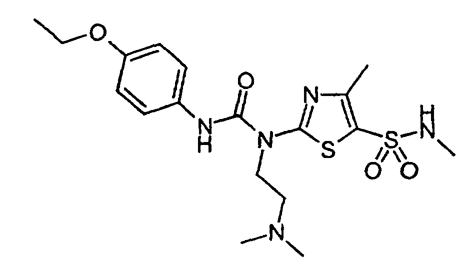

1,0 g (3,59 mmol) 2-{[2-Dimethylamino)ethyl)amino}-N,4-dimethyl-1,3-thiazol-5-sulfonamid und 586 mg (3,59 mmol) 4-Ethoxyphenylisocyanat werden in 30 ml Dioxan gelöst und 12 h bei Raumtemperatur gerührt. Anschließend wird die Mischung im Vakuum eingeengt und der Rückstand aus 2-Propanol umkristallisiert.
Ausbeute: 1,19 g (75 %)
Fp.: 153°C

Analog der oben aufgeführten Vorschriften werden die in der folgenden Tabelle aufgeführten Verbindungen hergestellt:

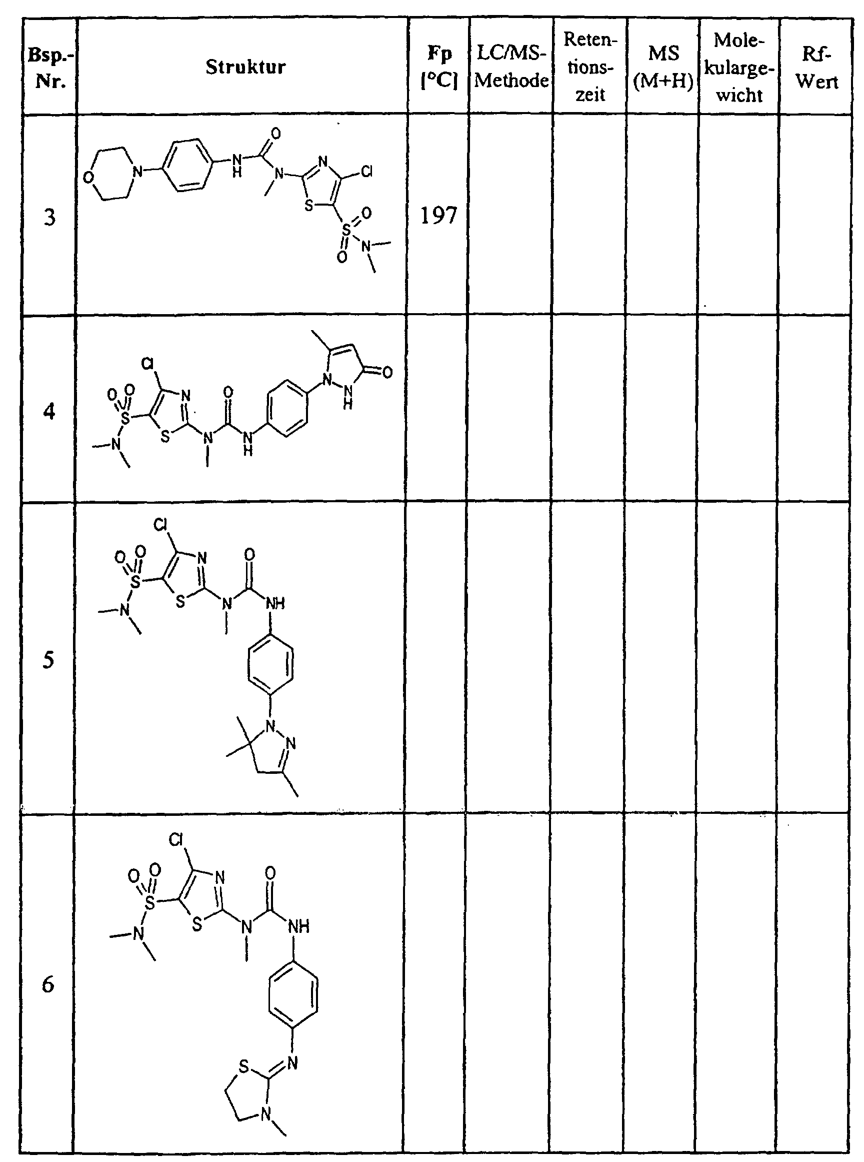

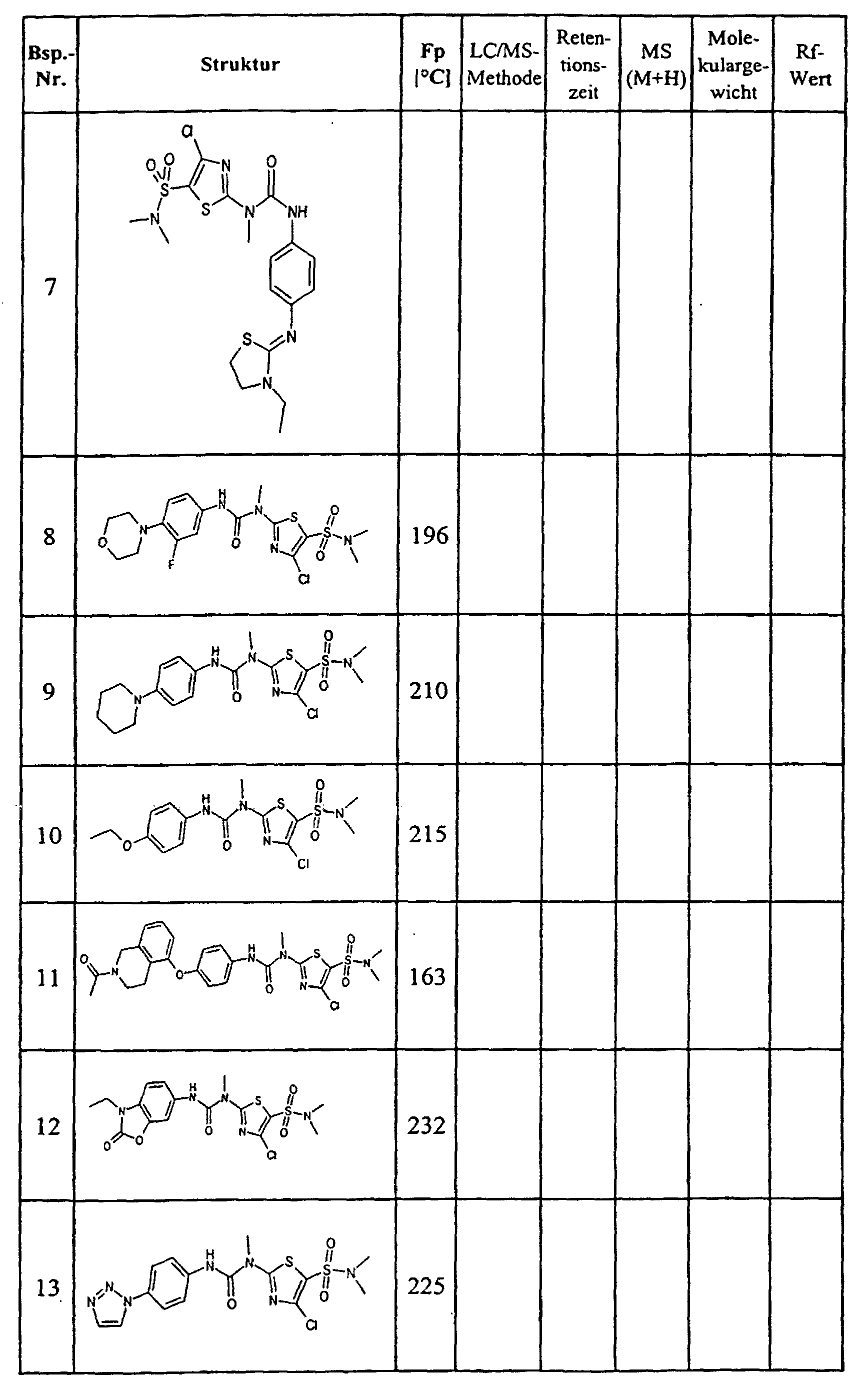

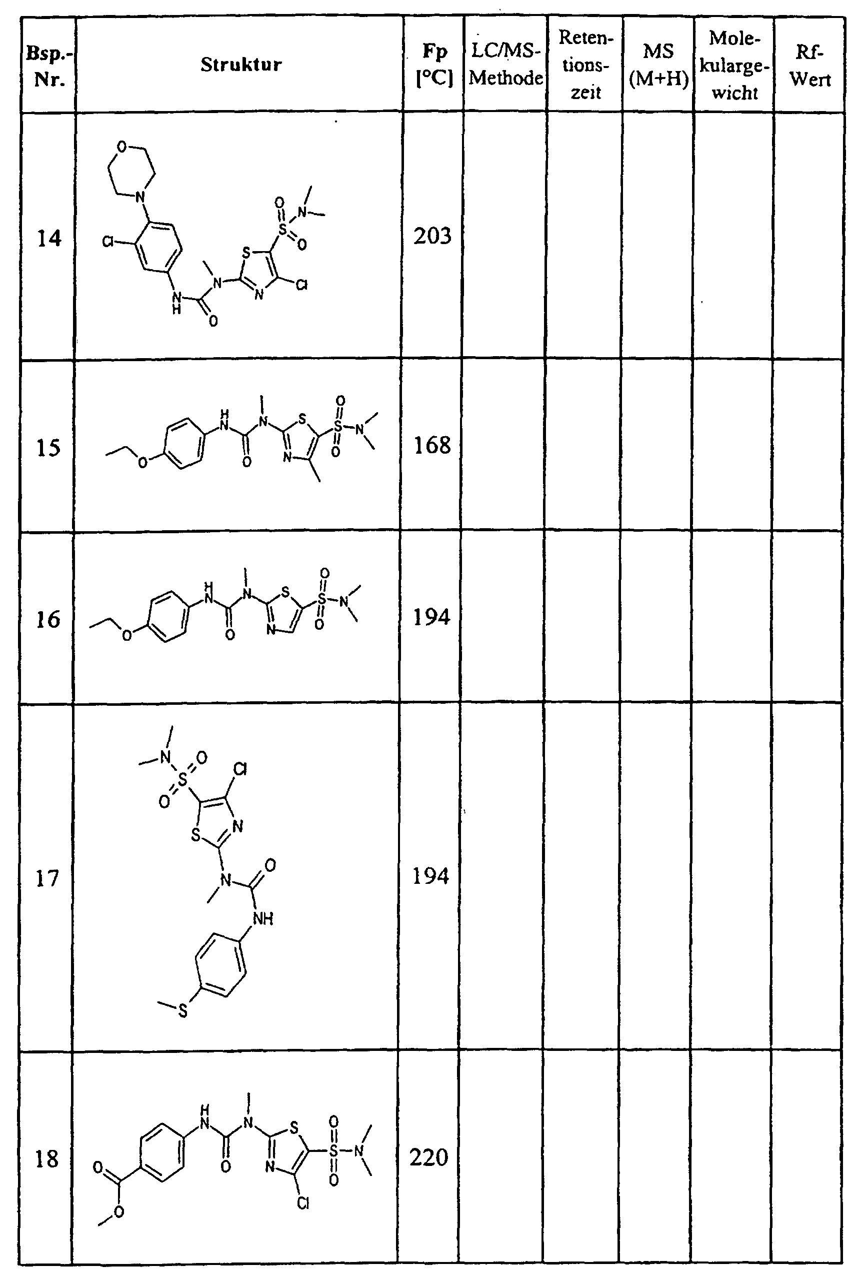

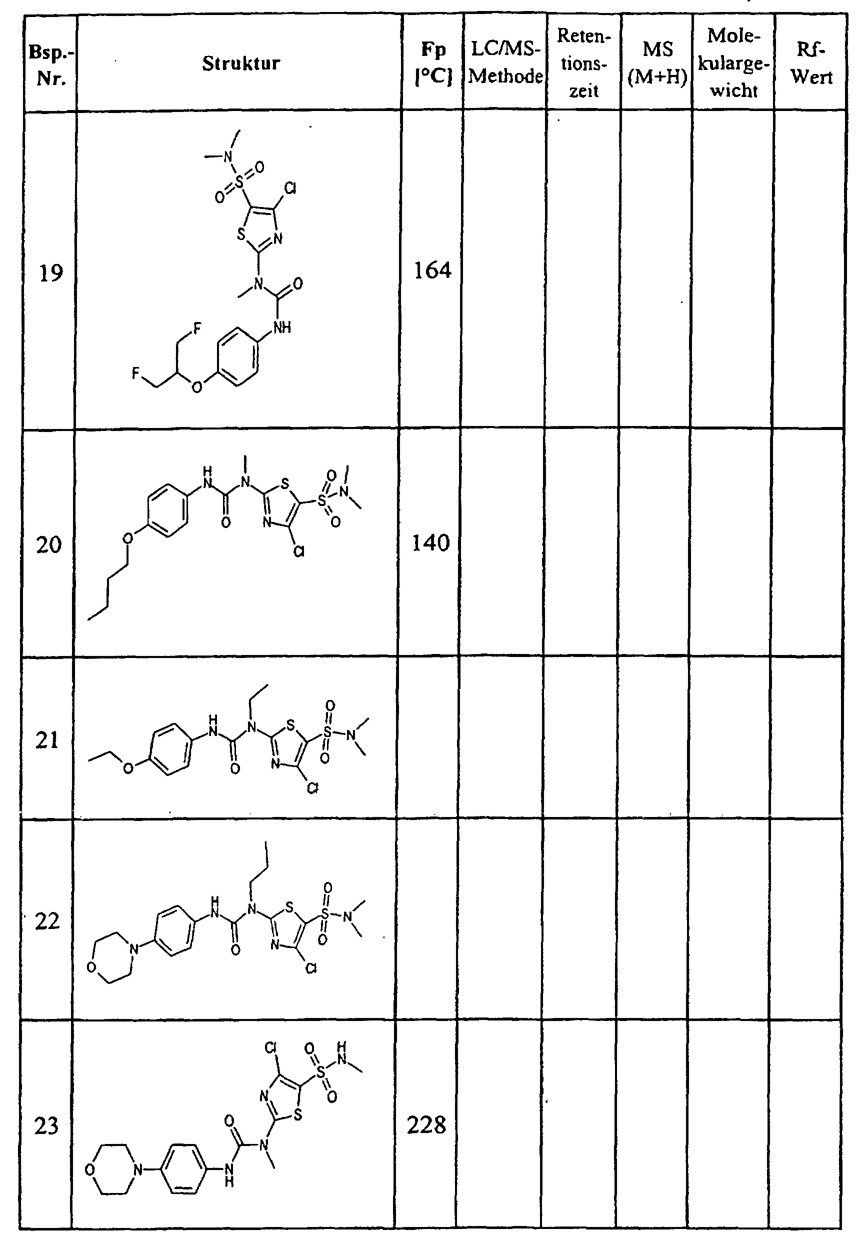

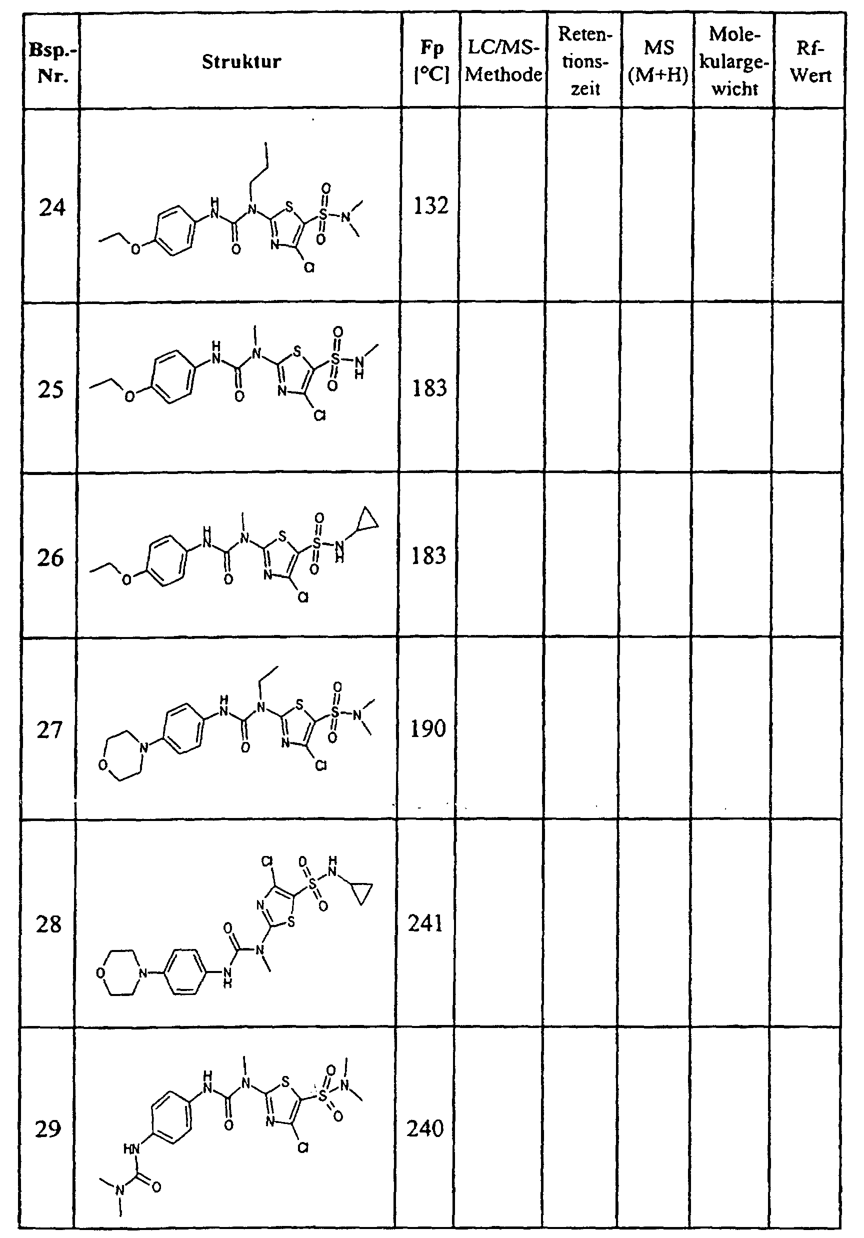

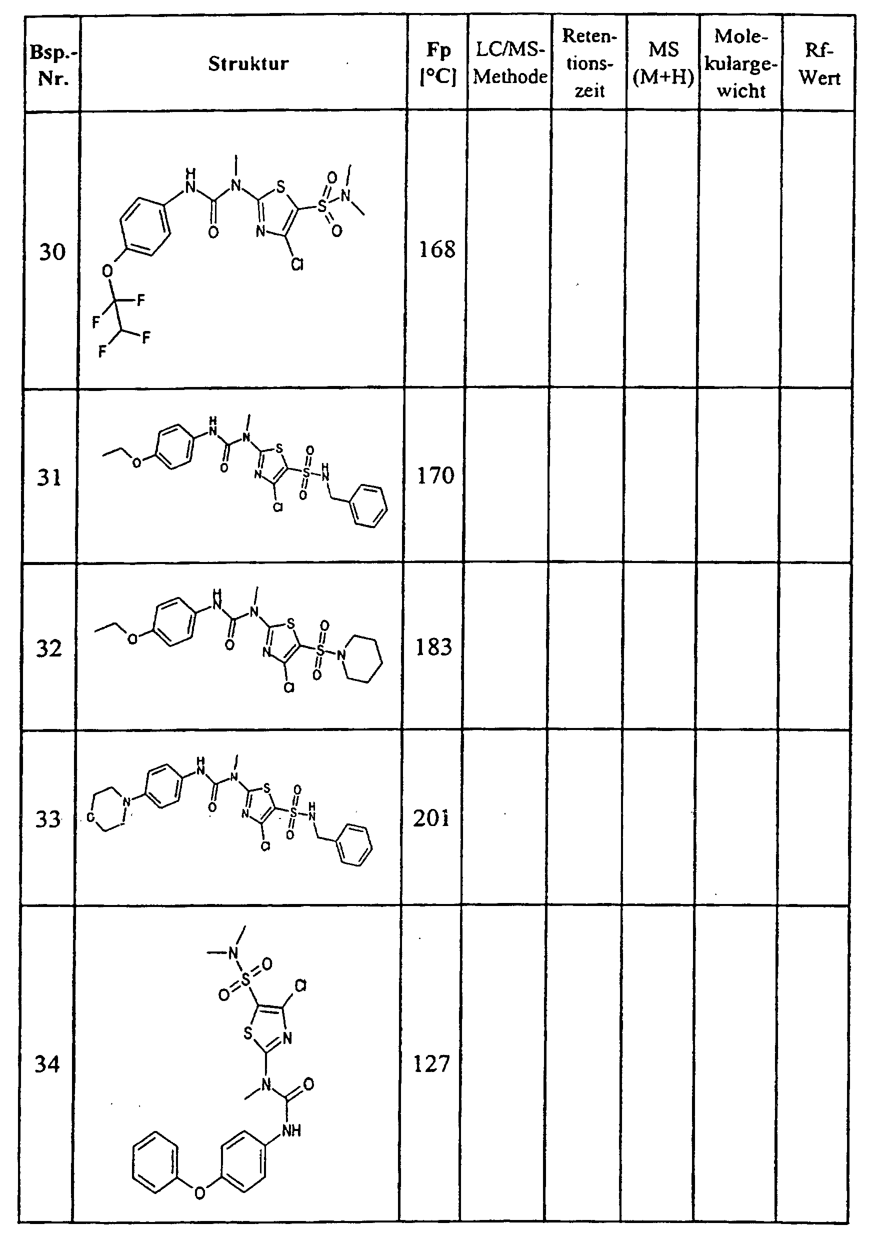

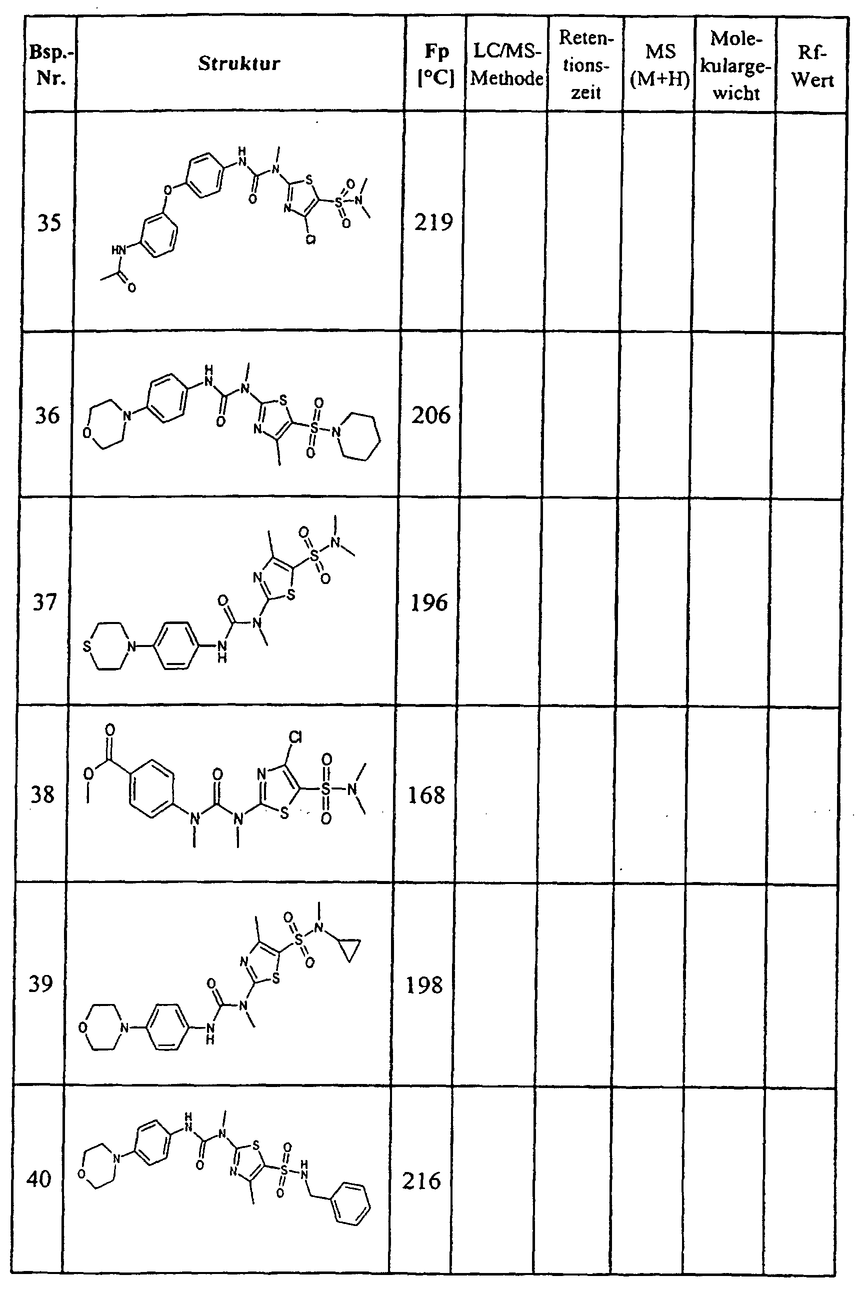

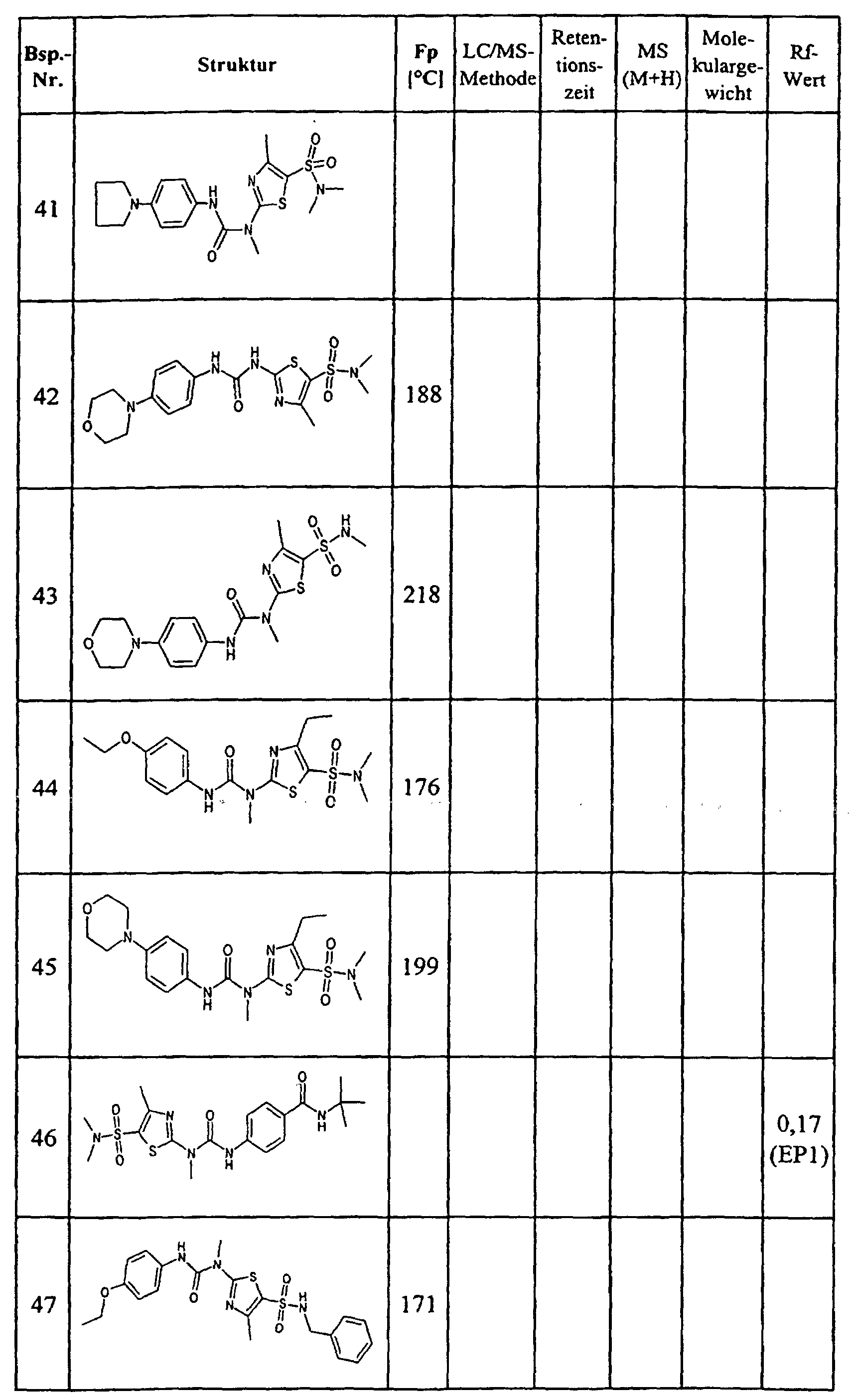

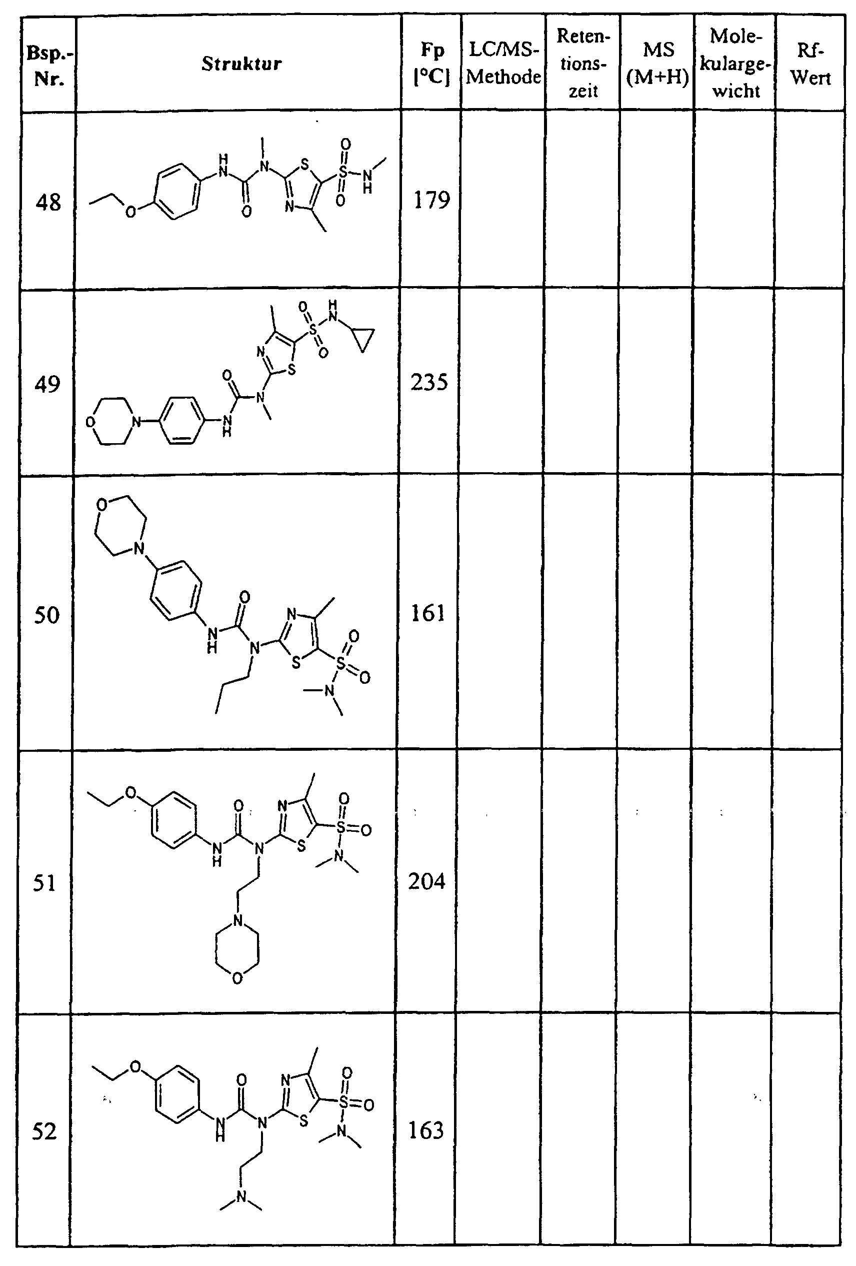

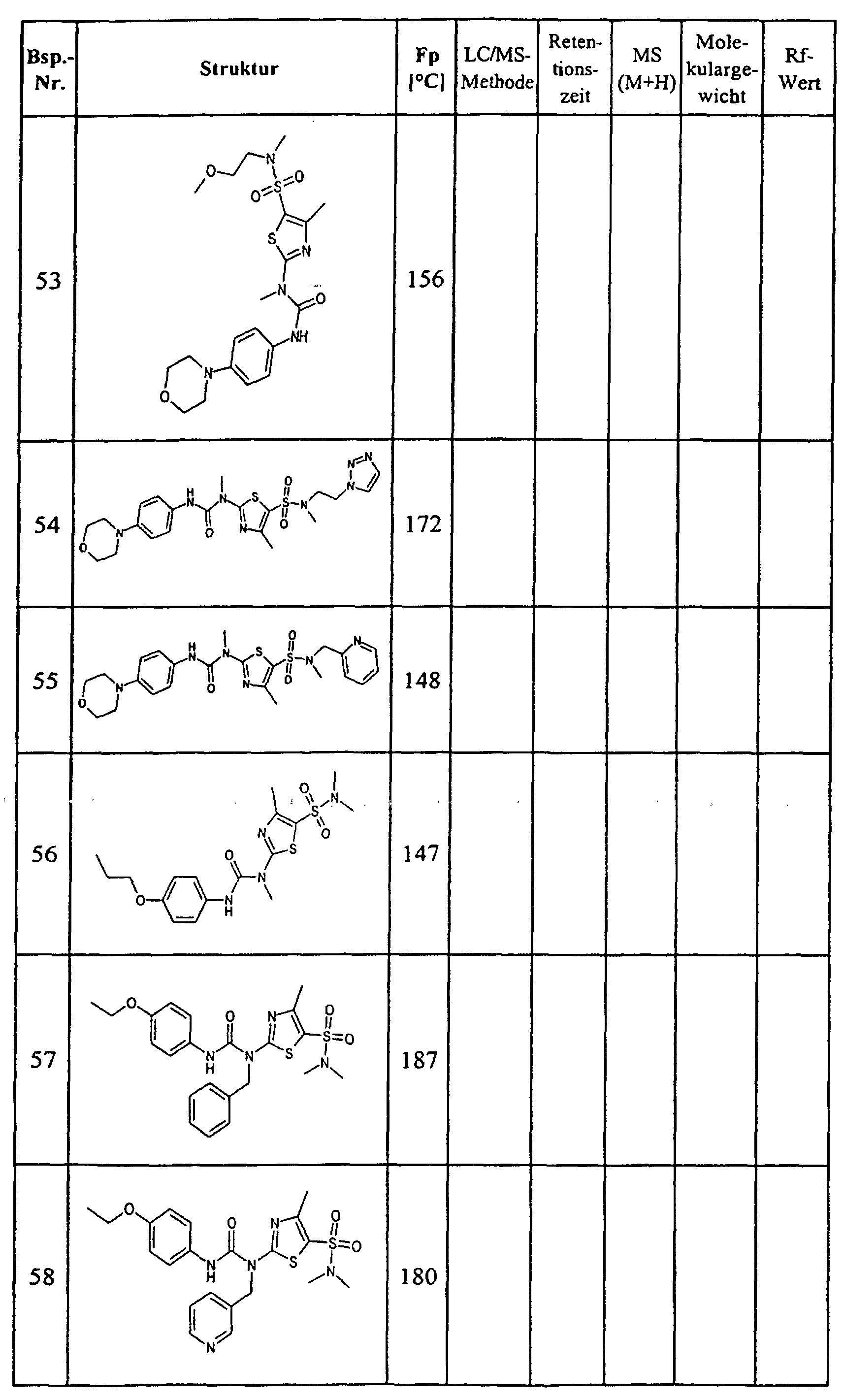

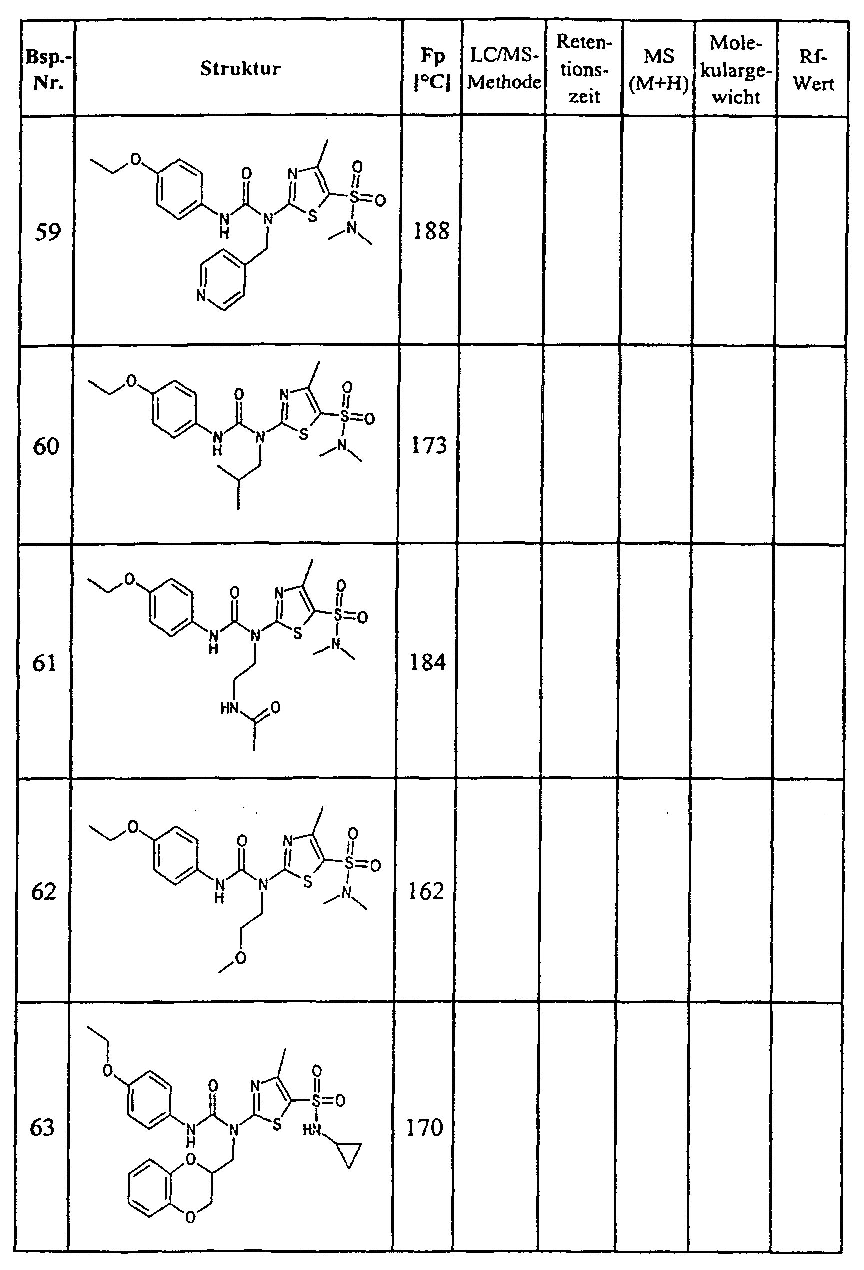

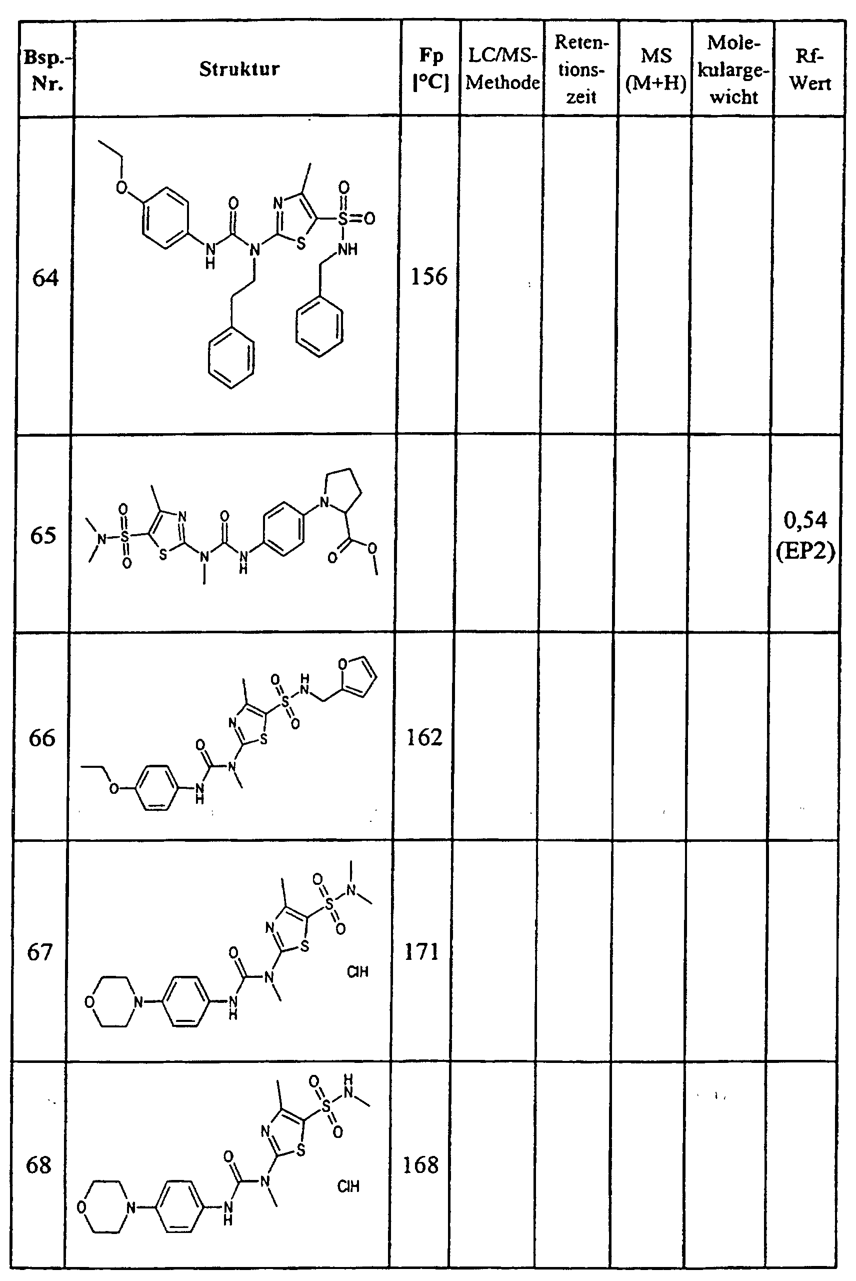

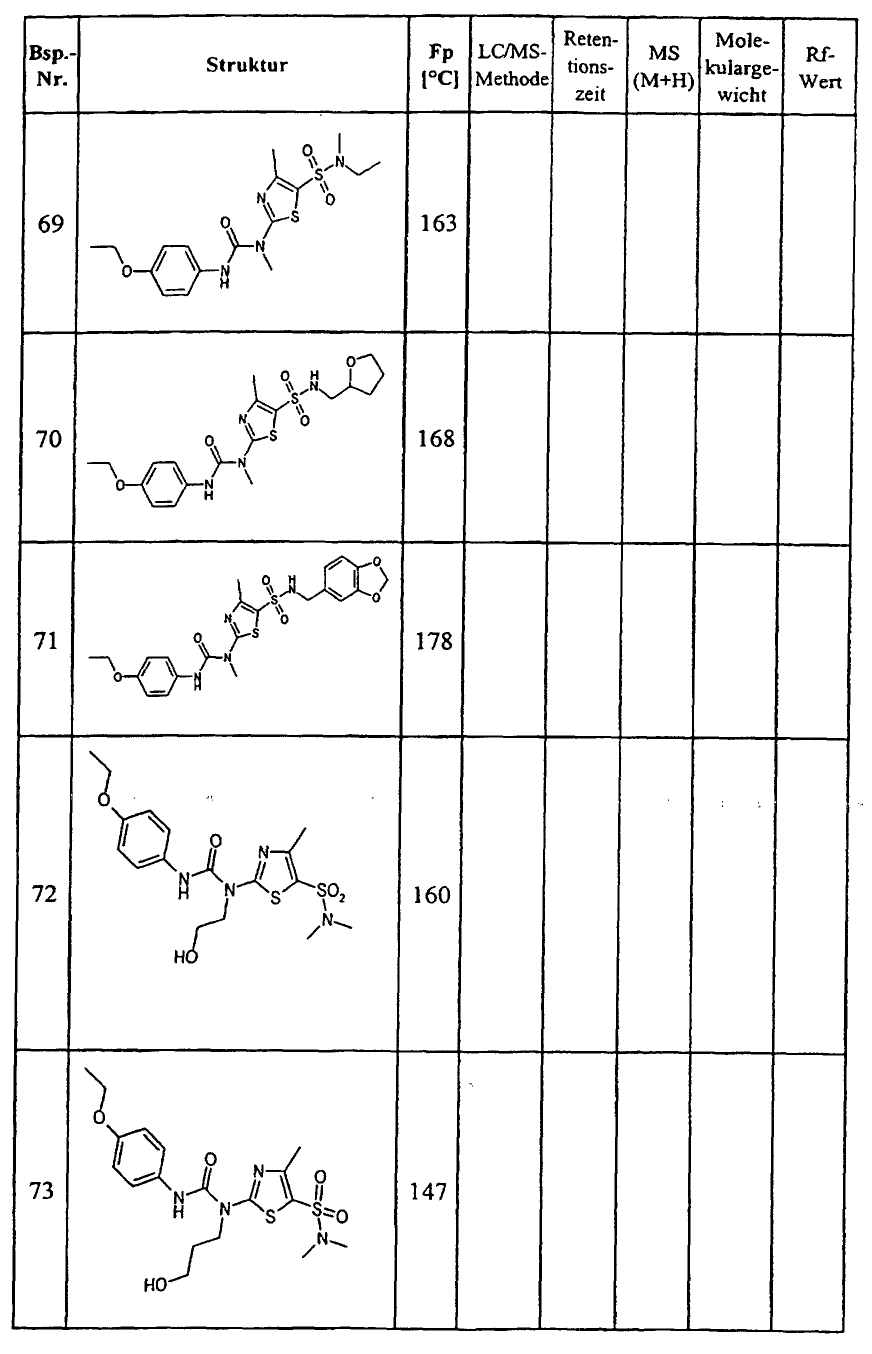

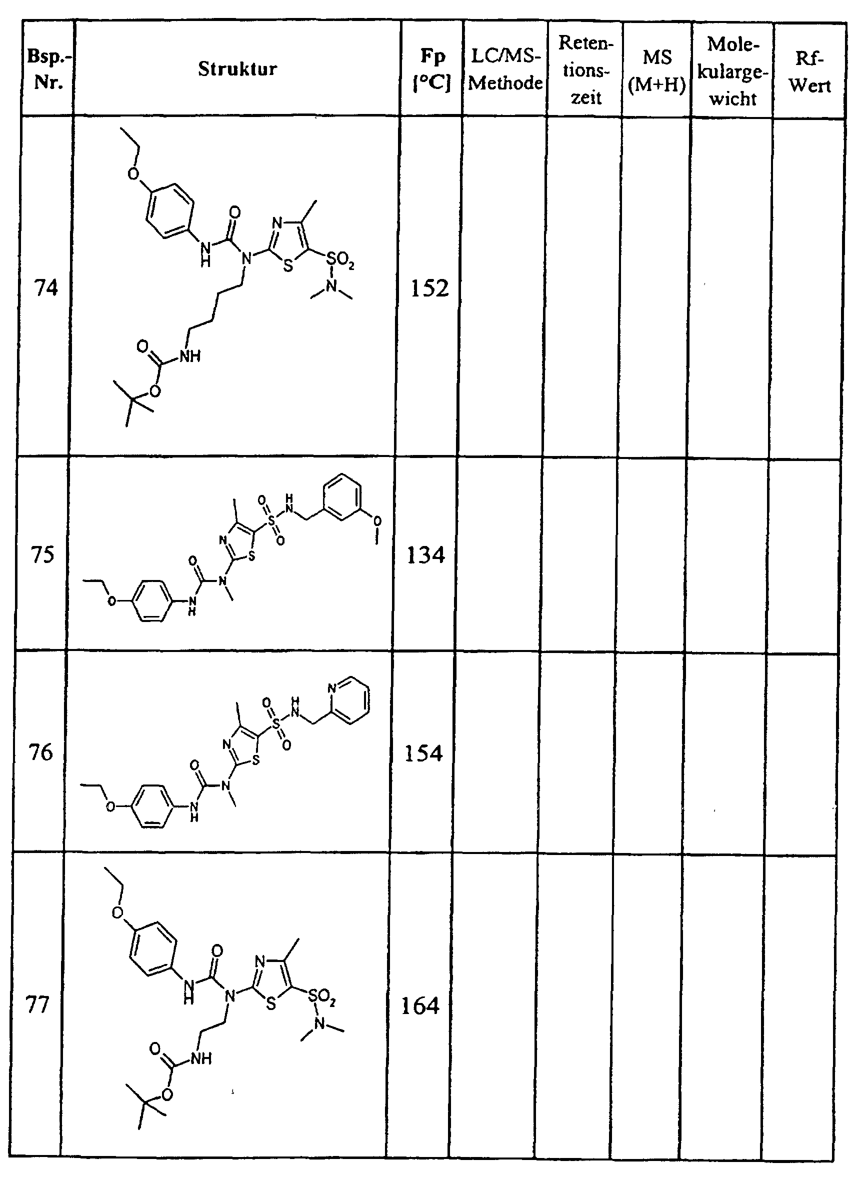

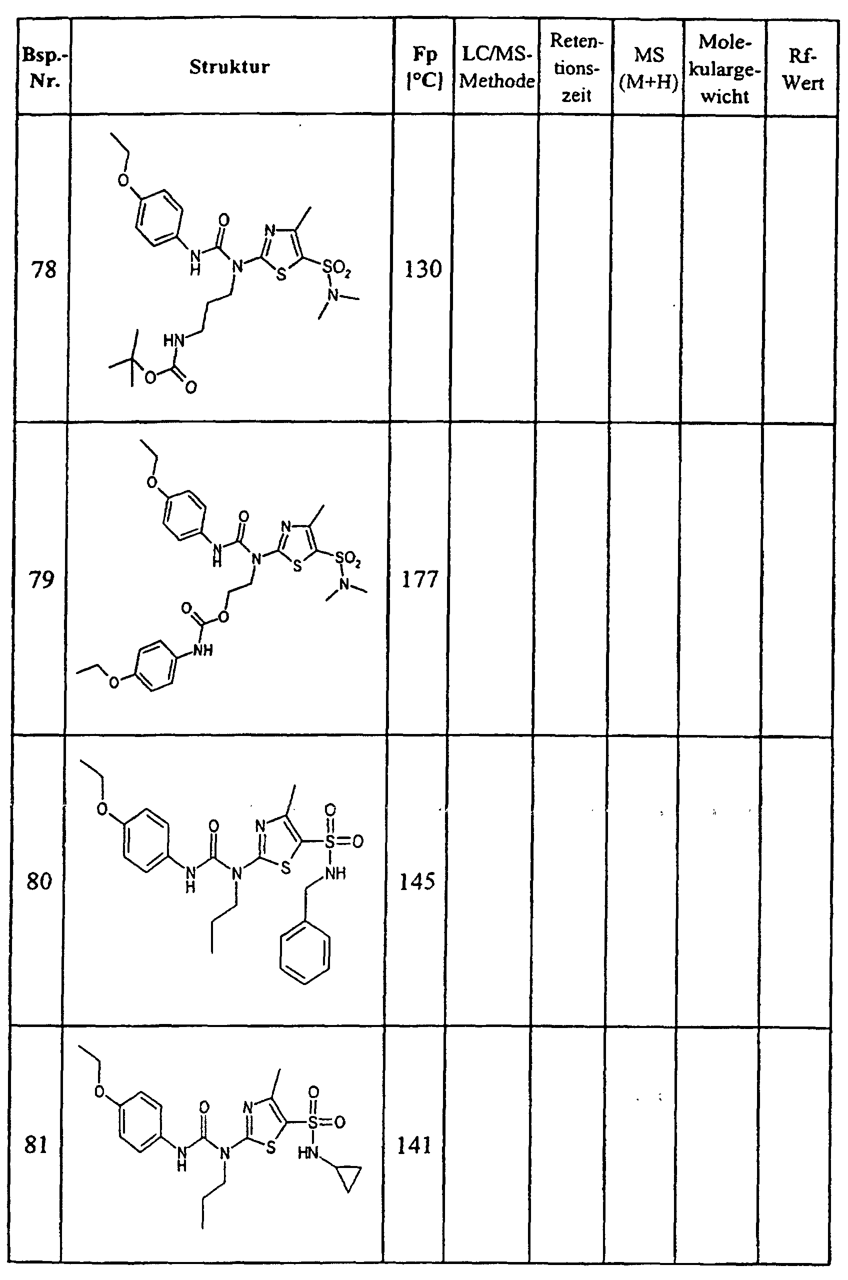

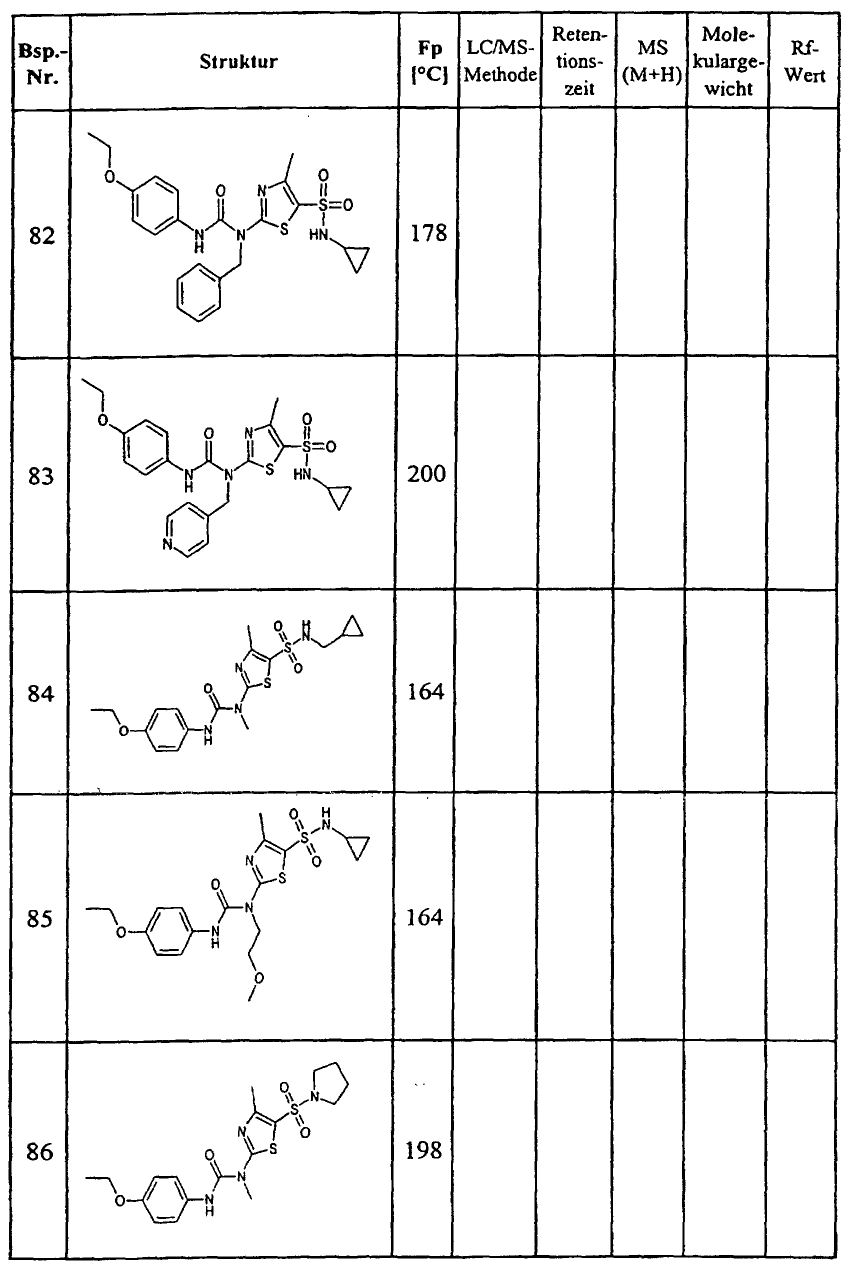

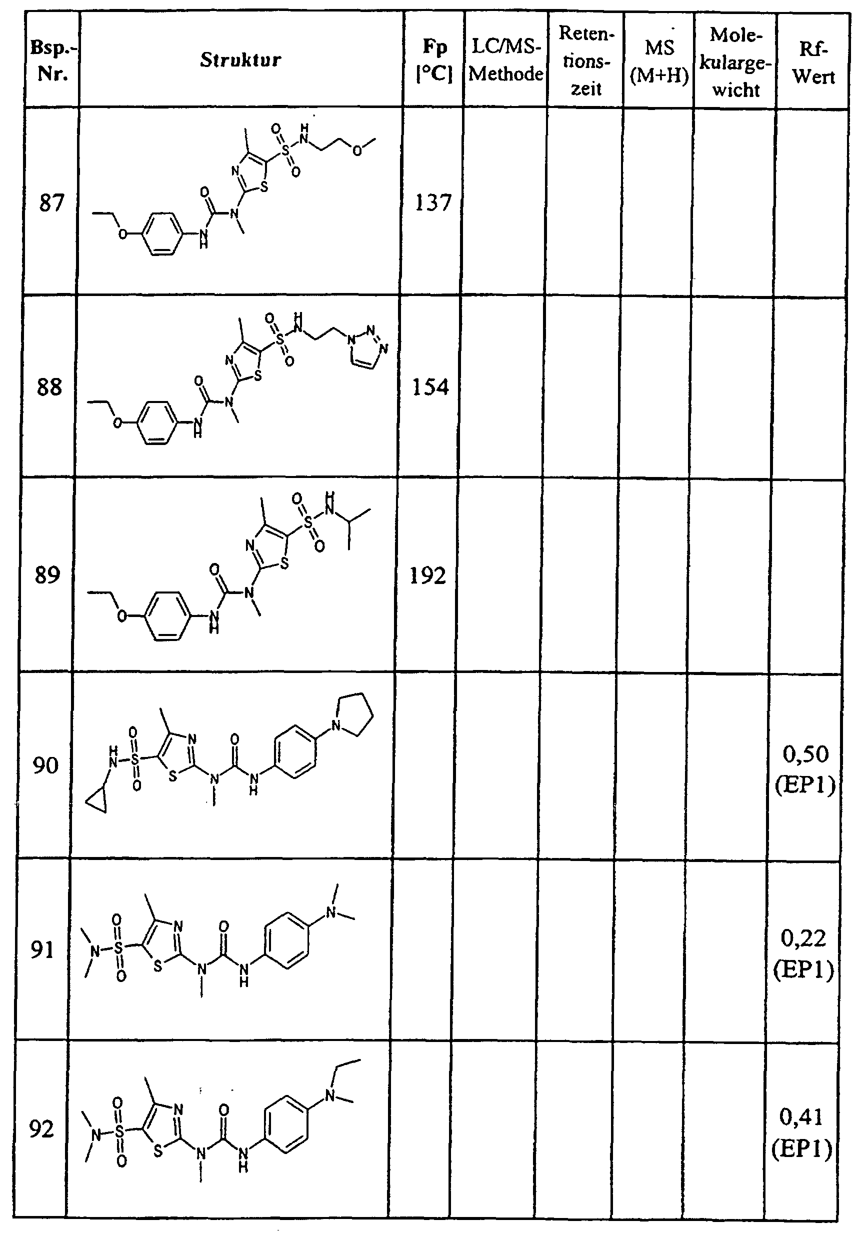

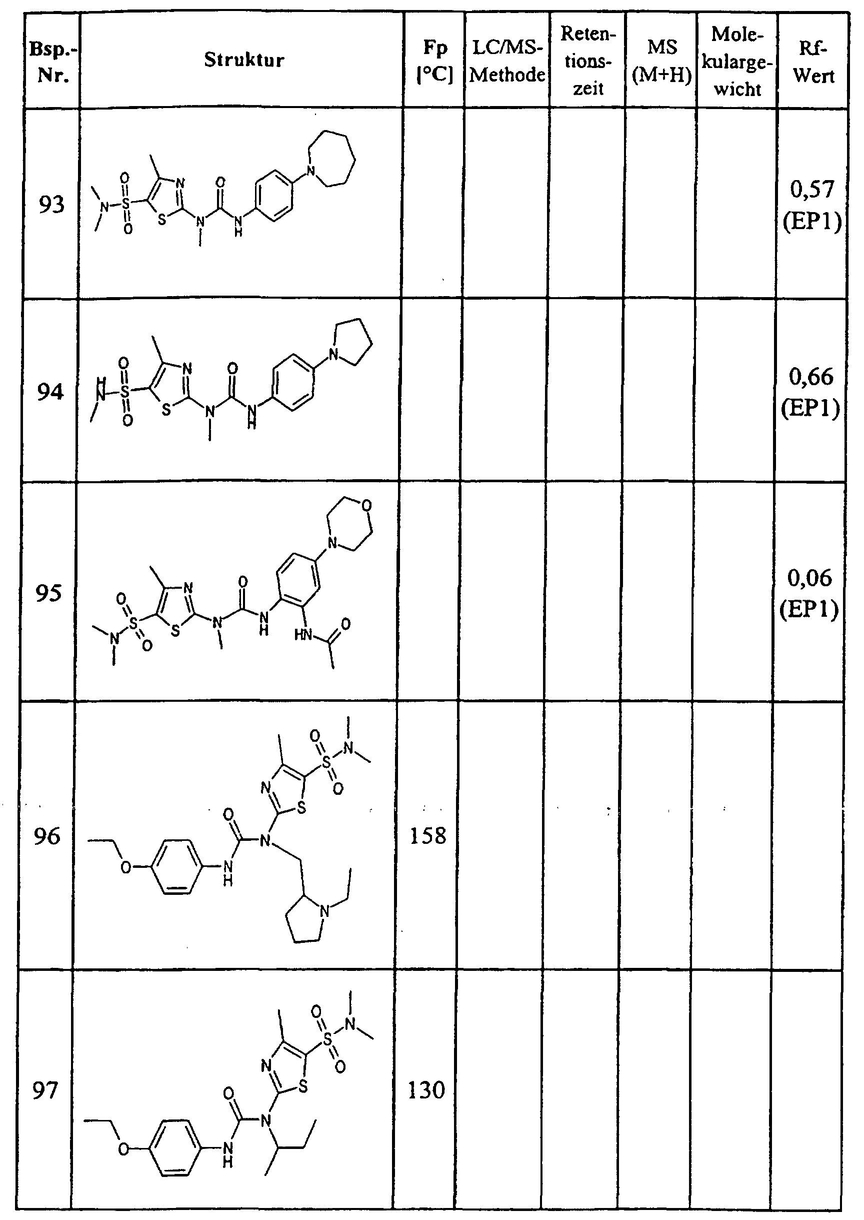

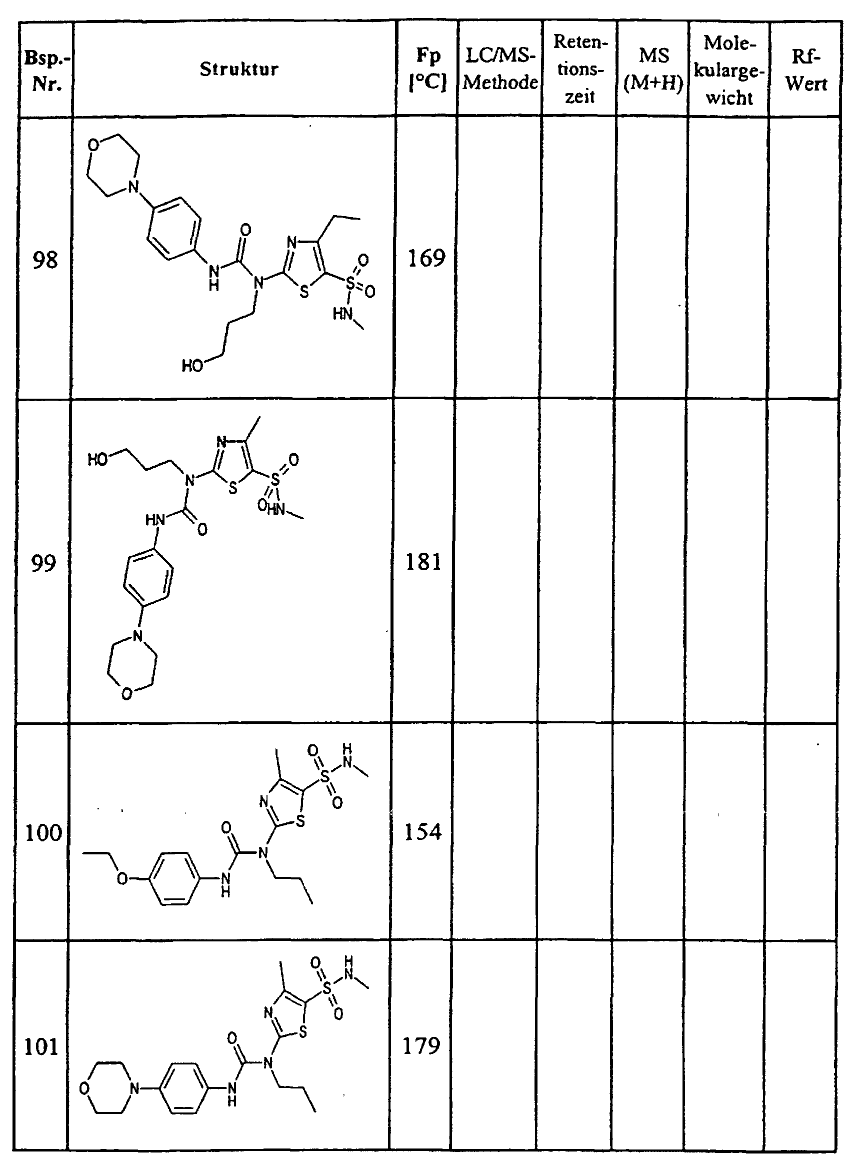

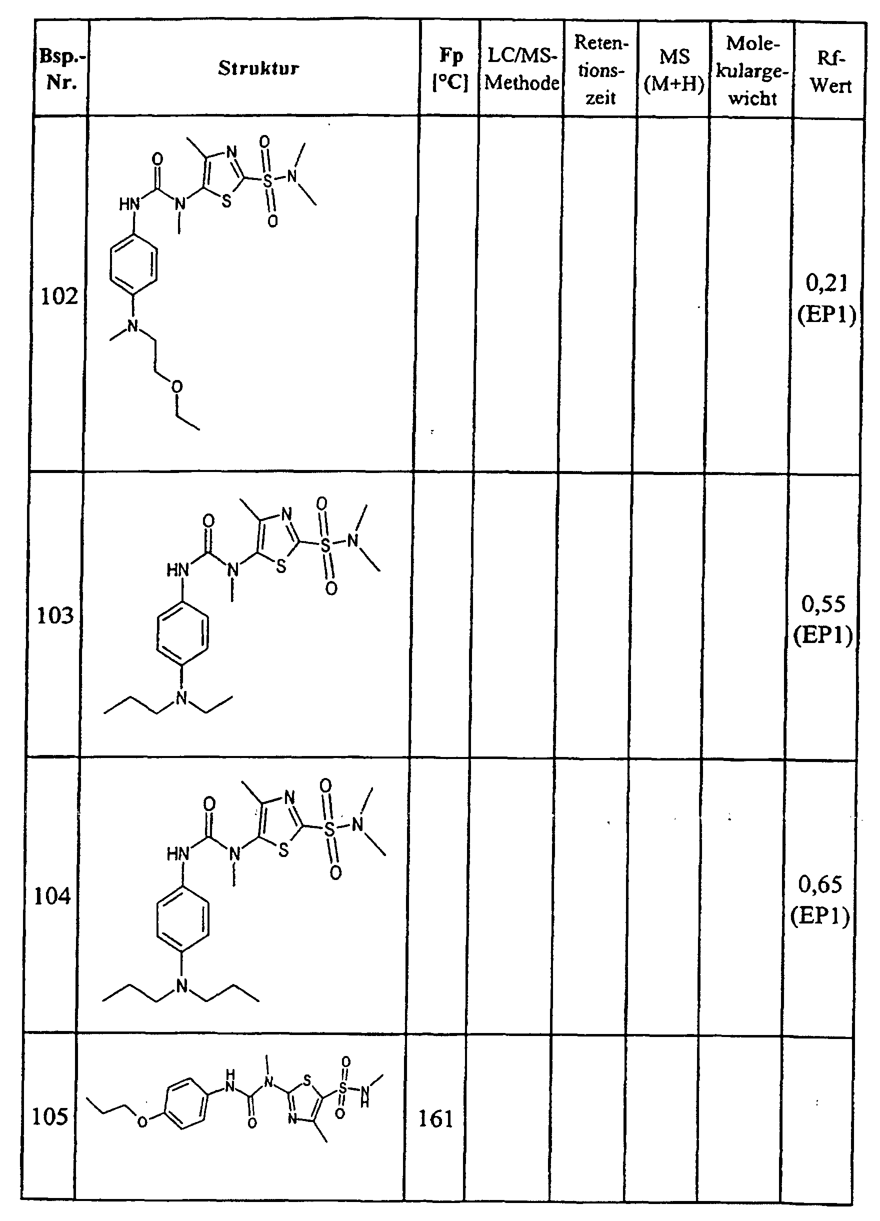

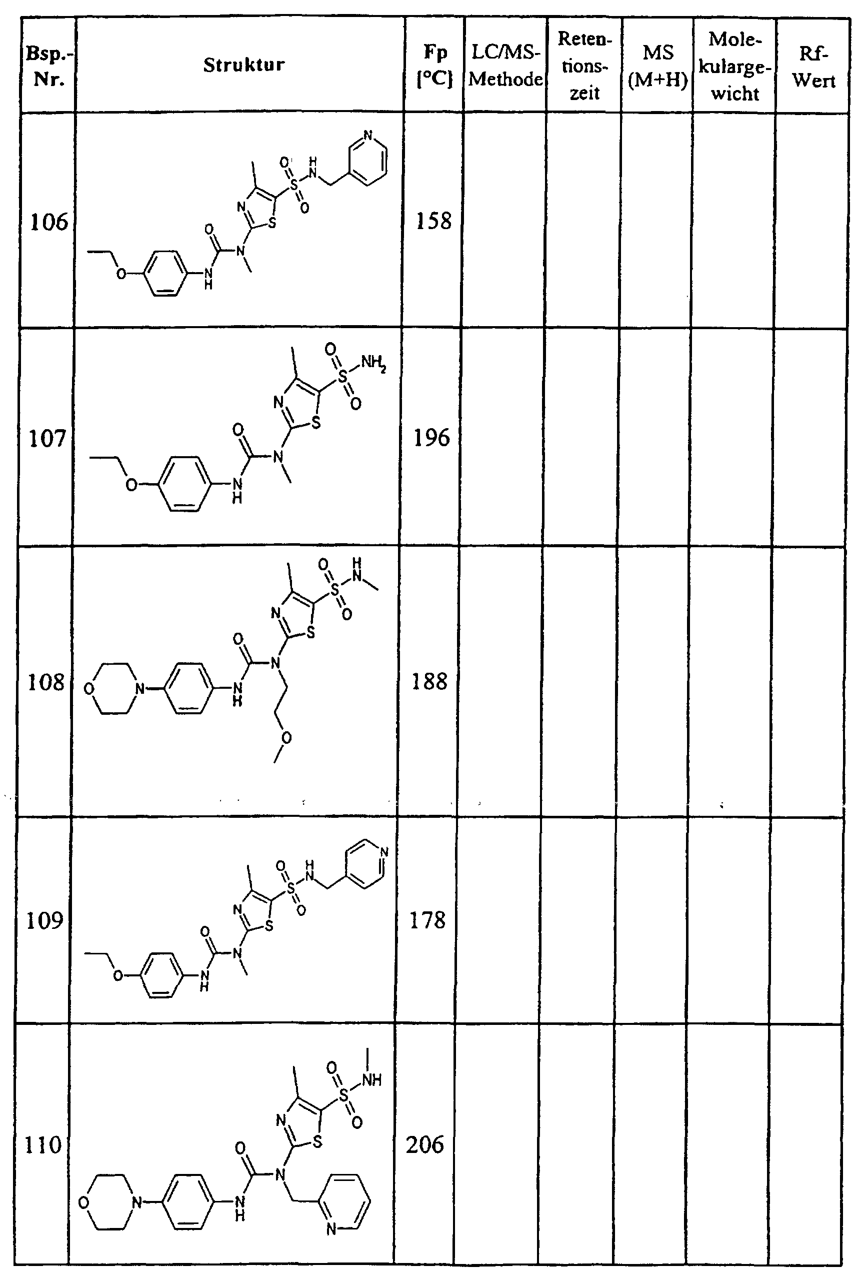

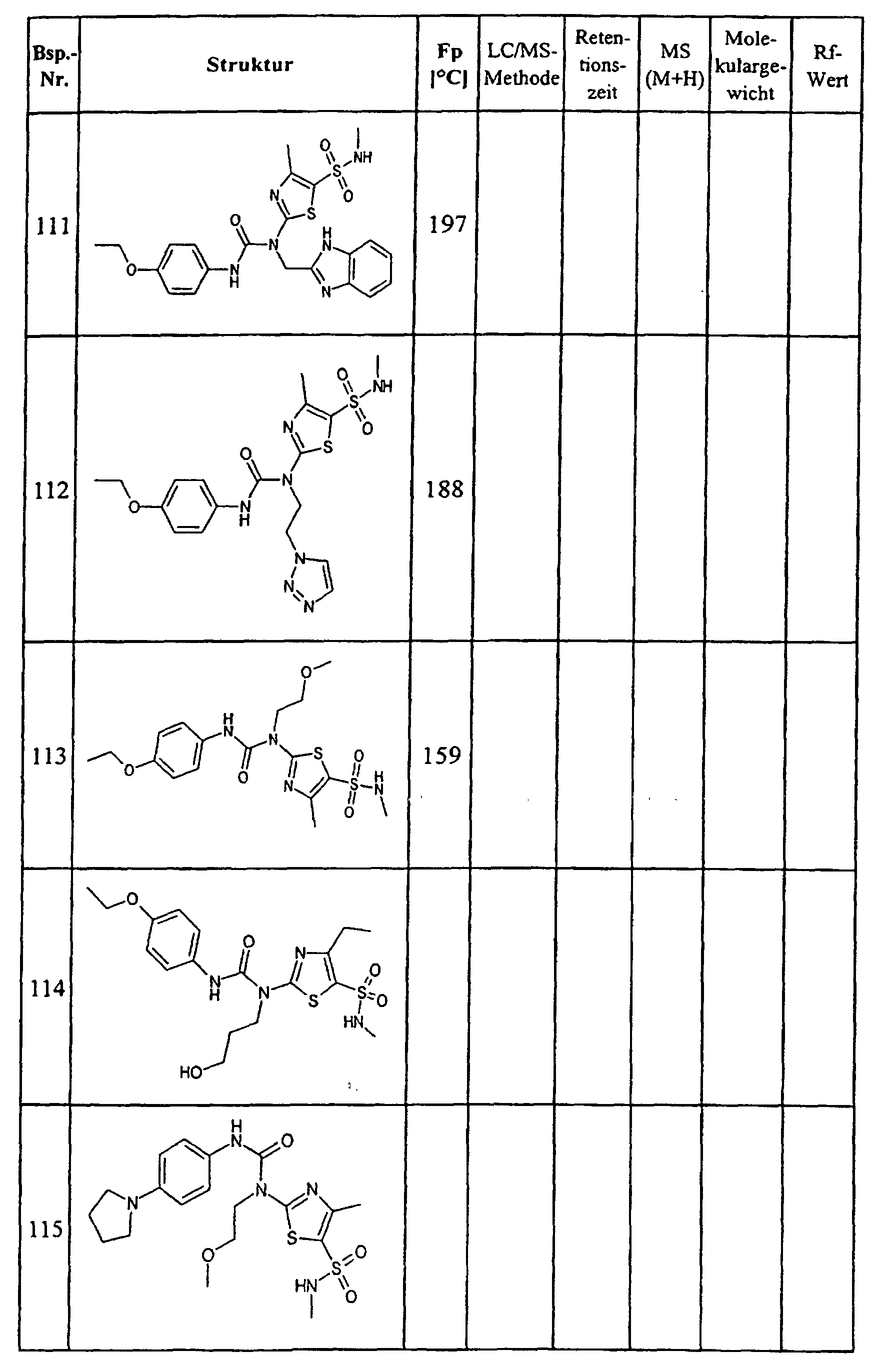

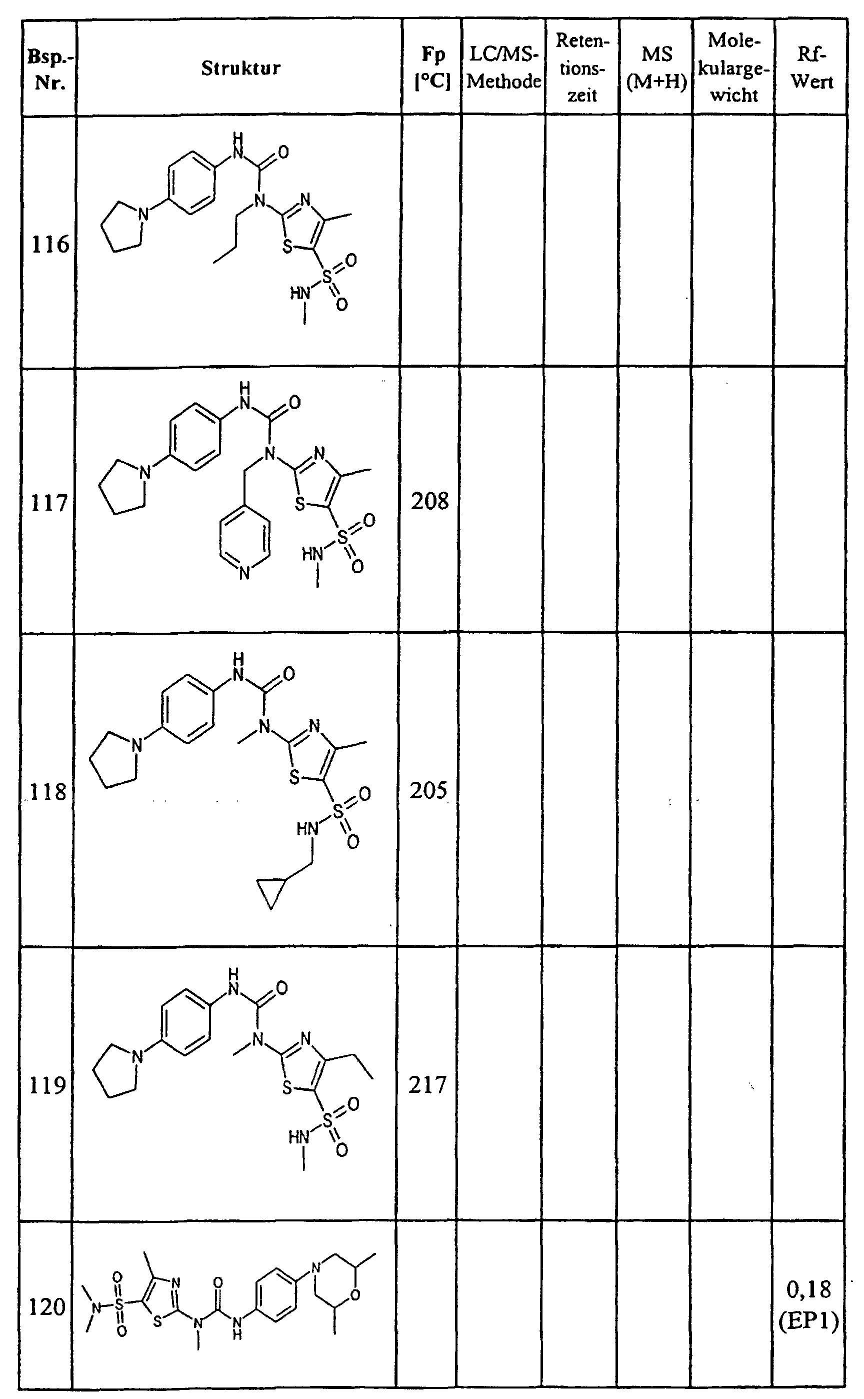

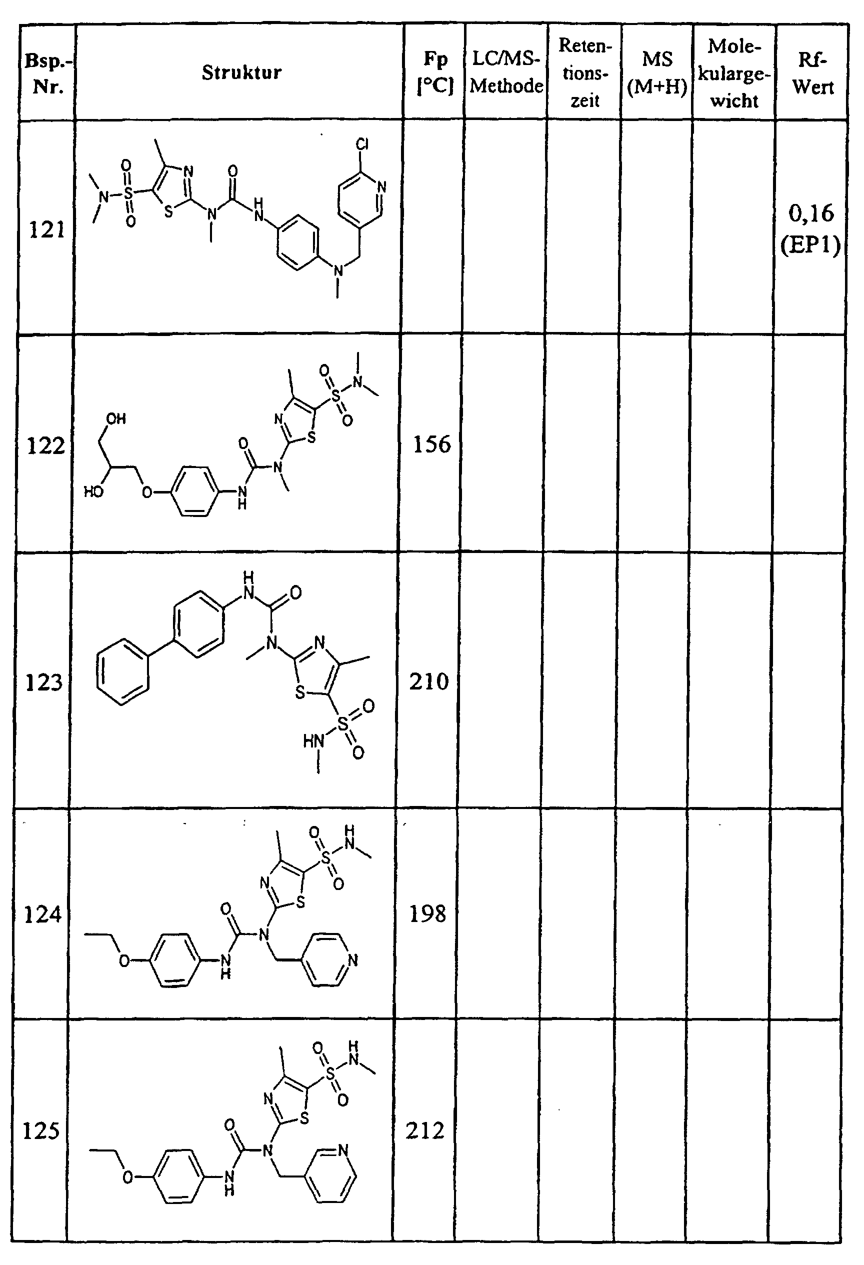

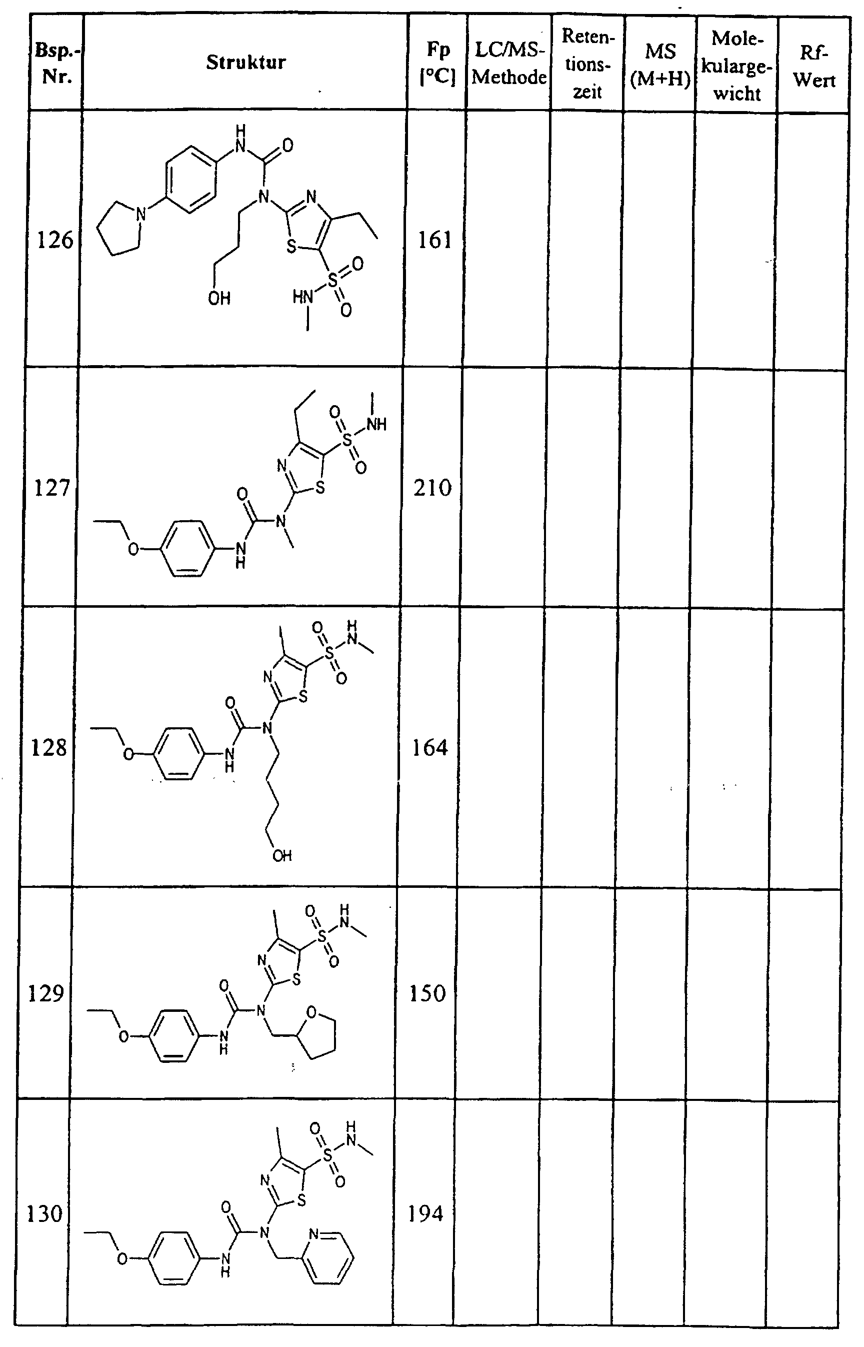

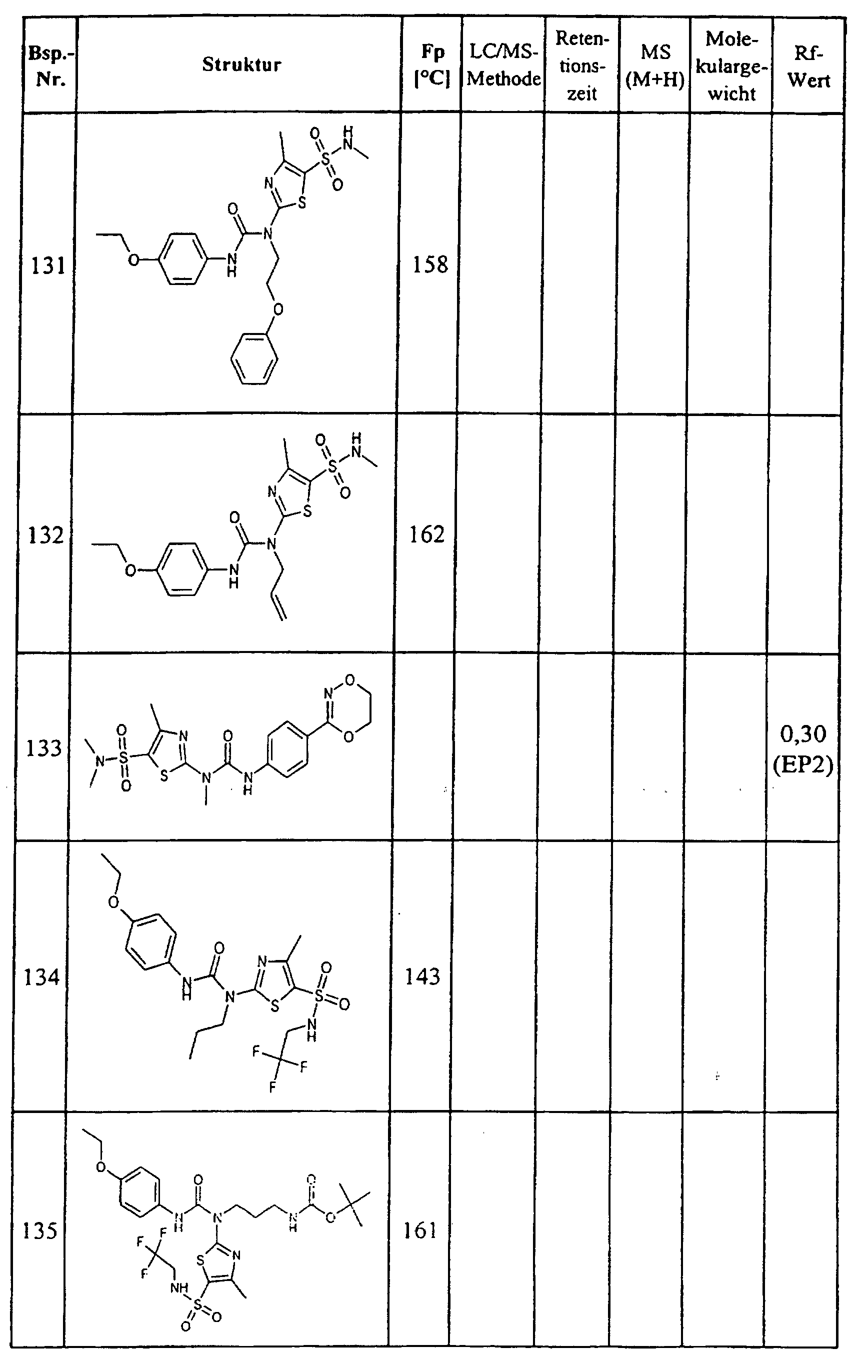

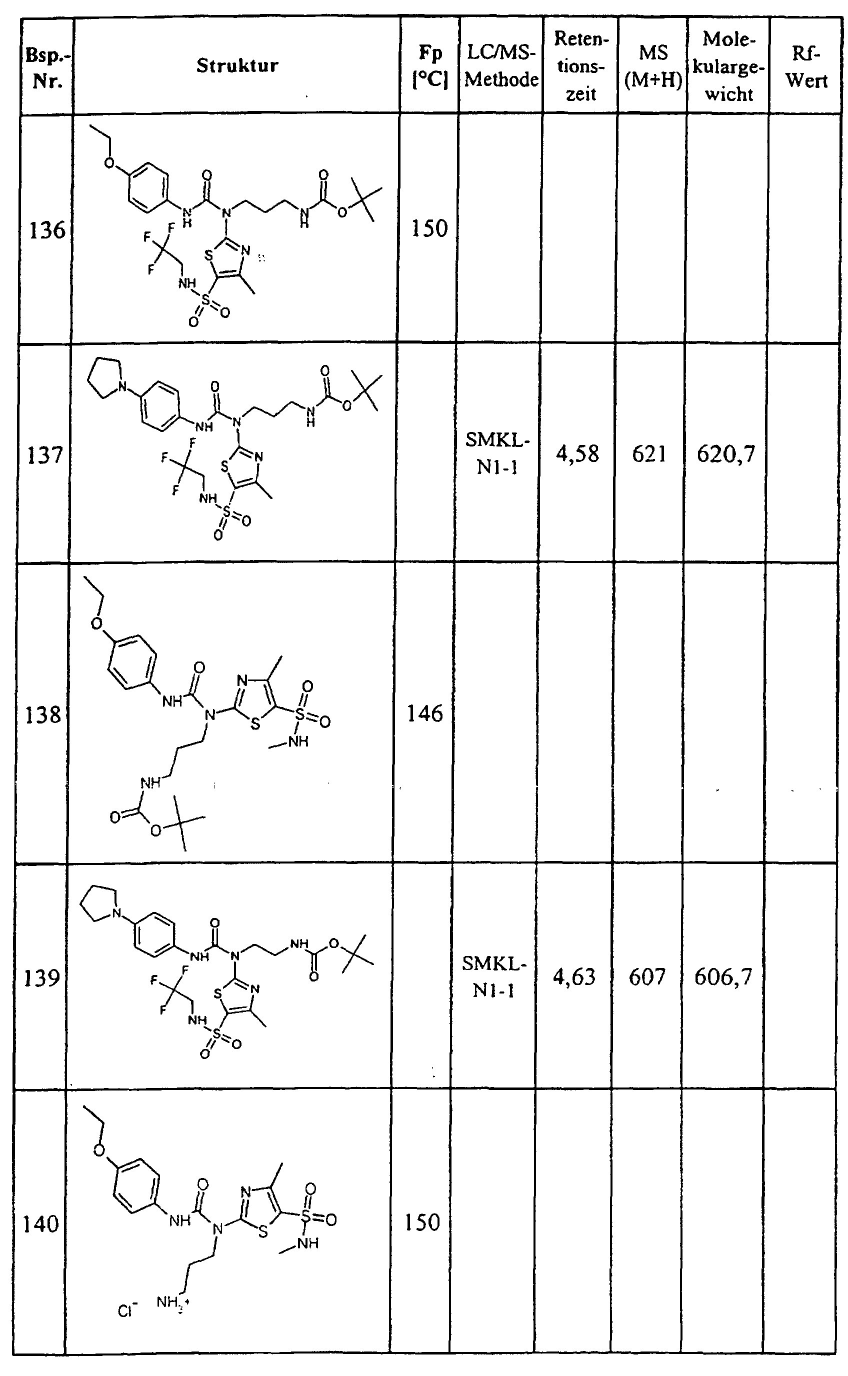

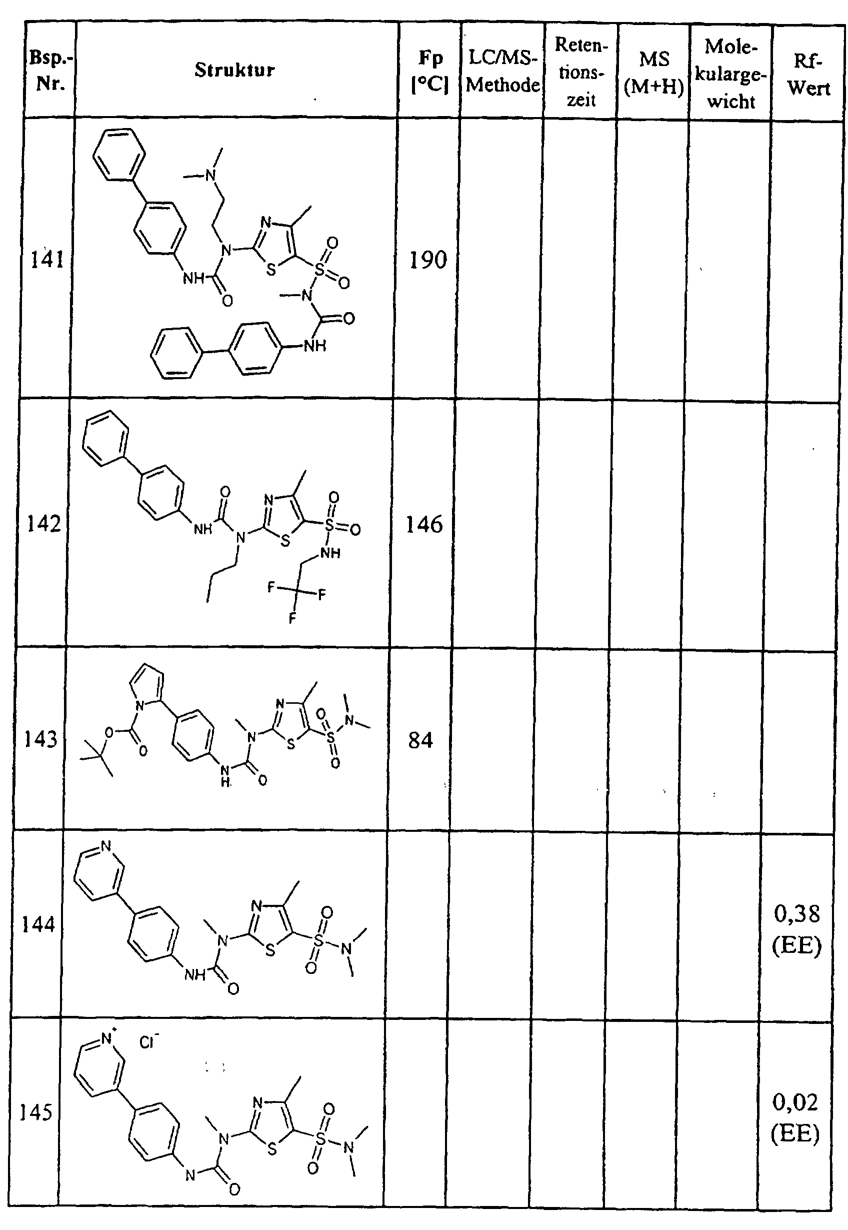

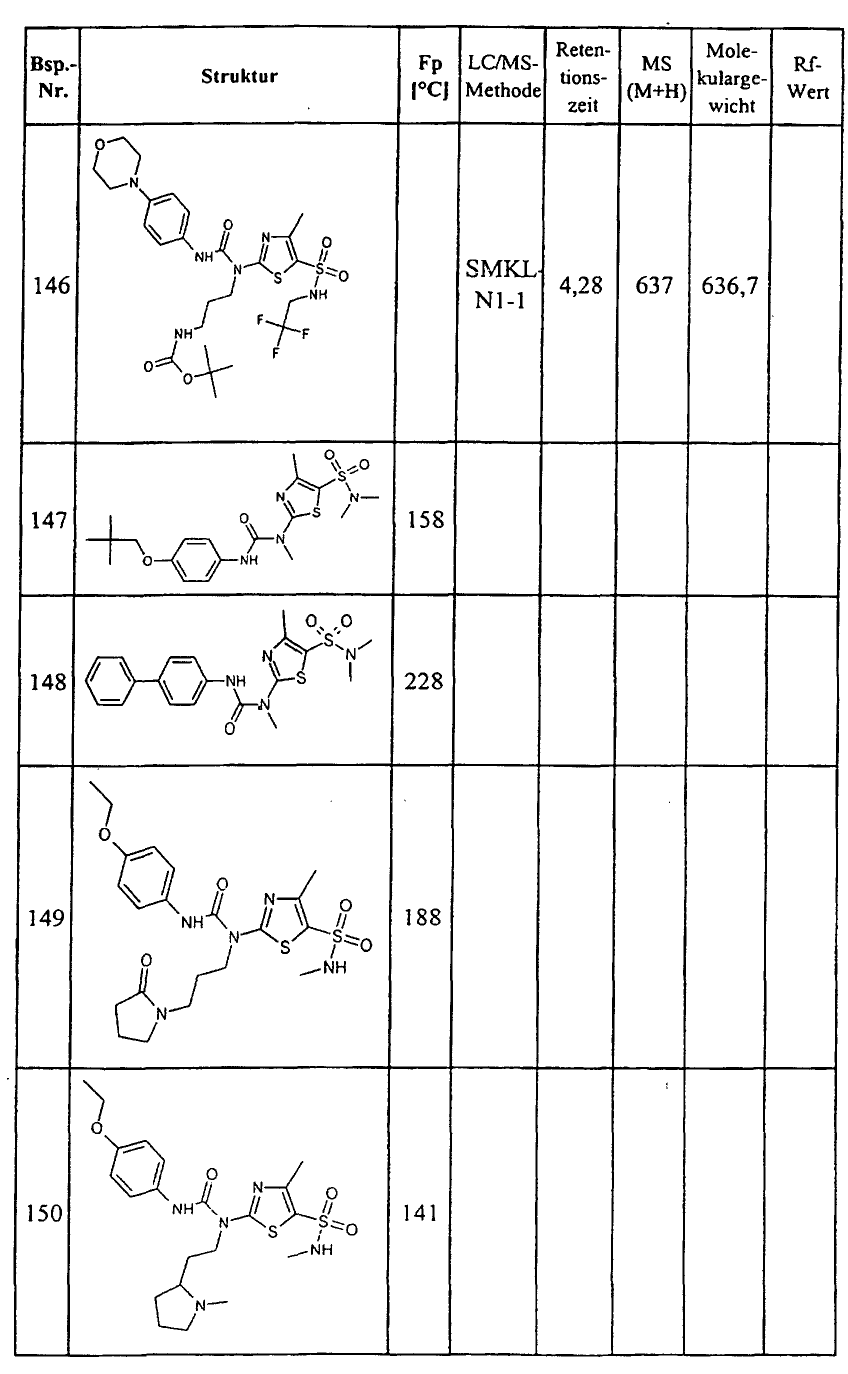

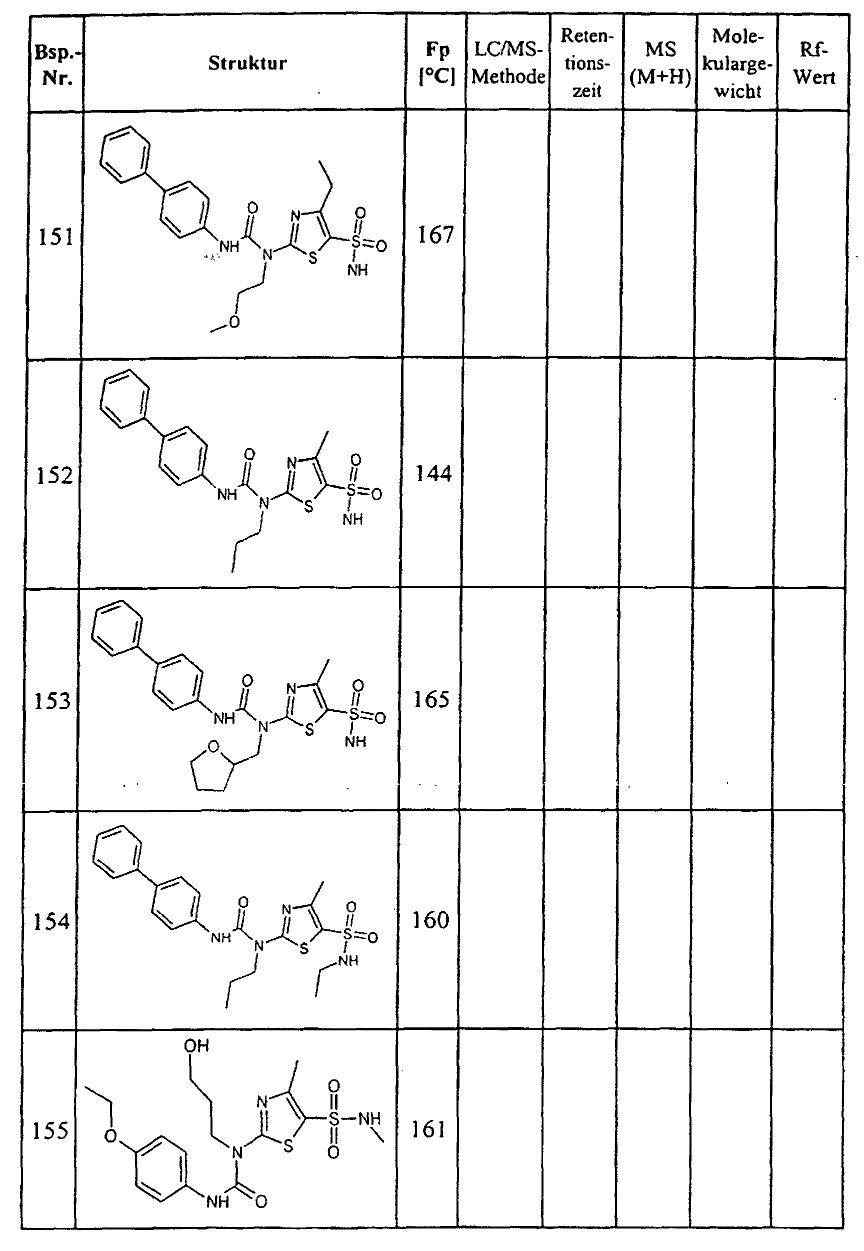

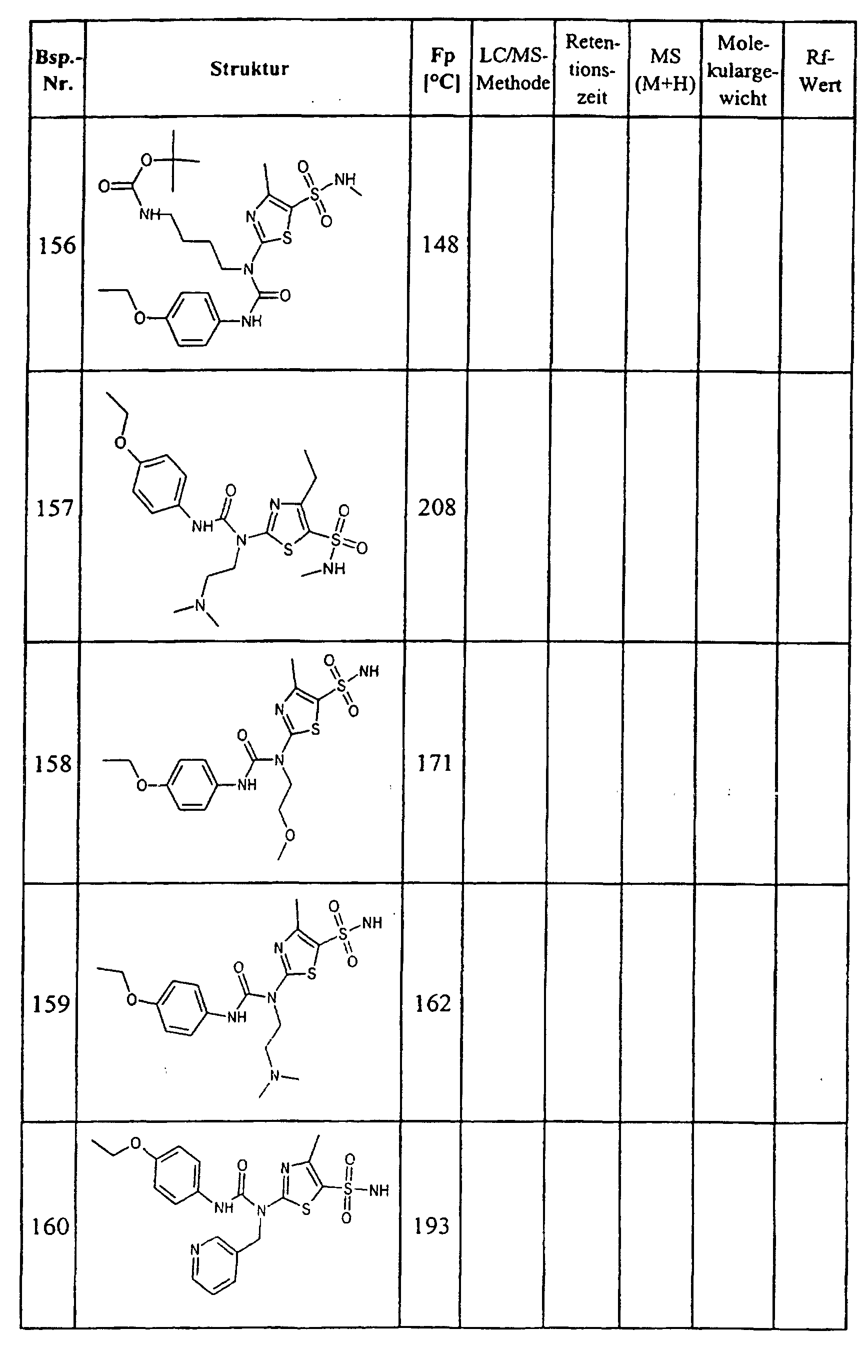

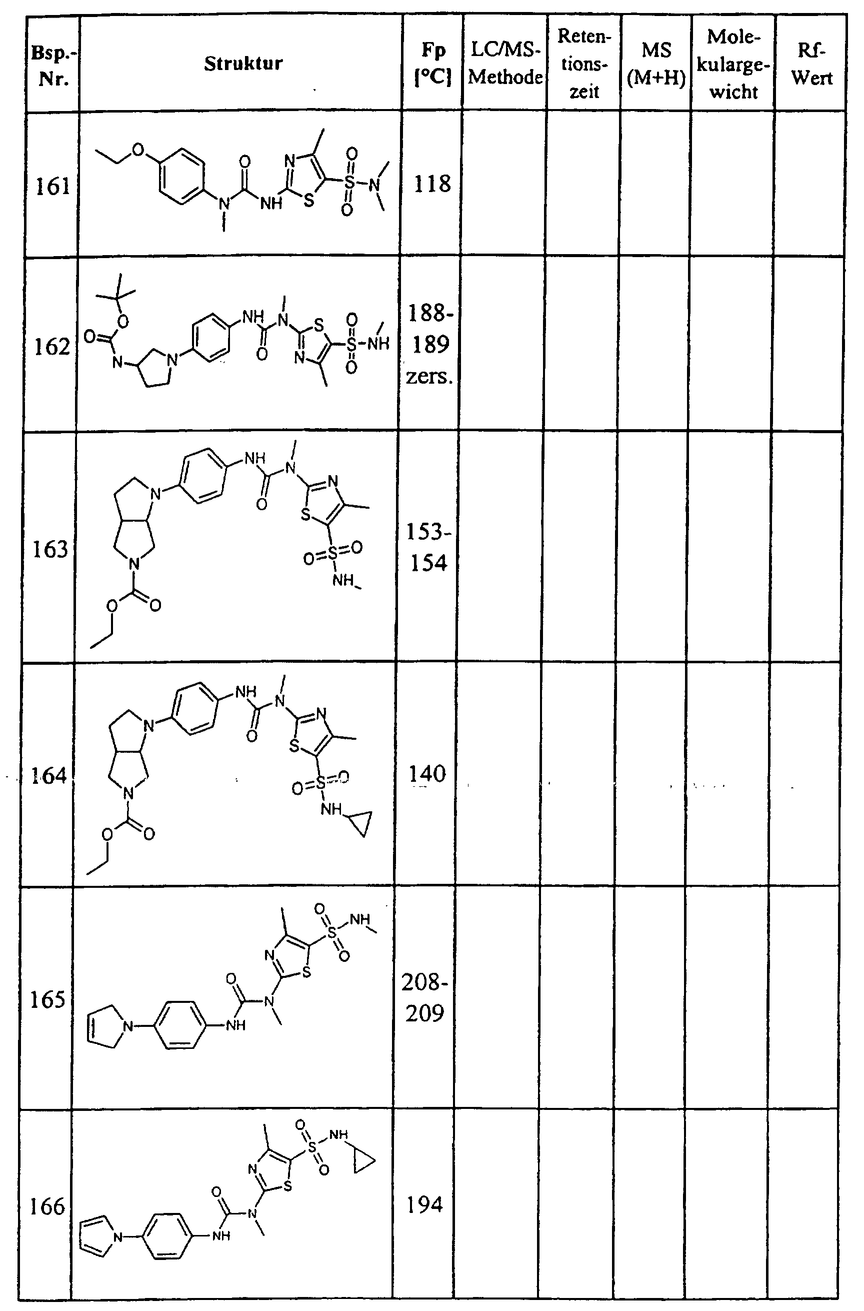

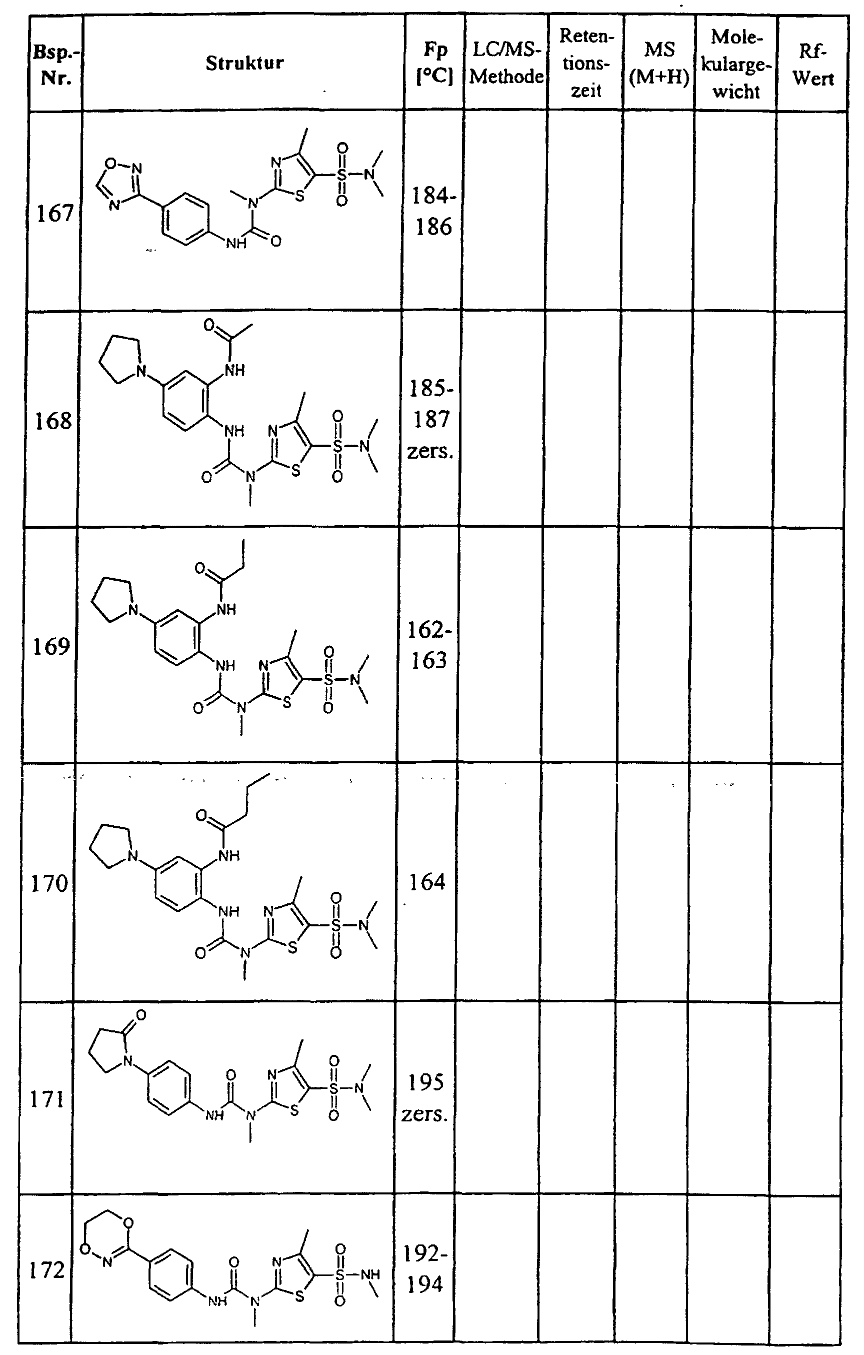

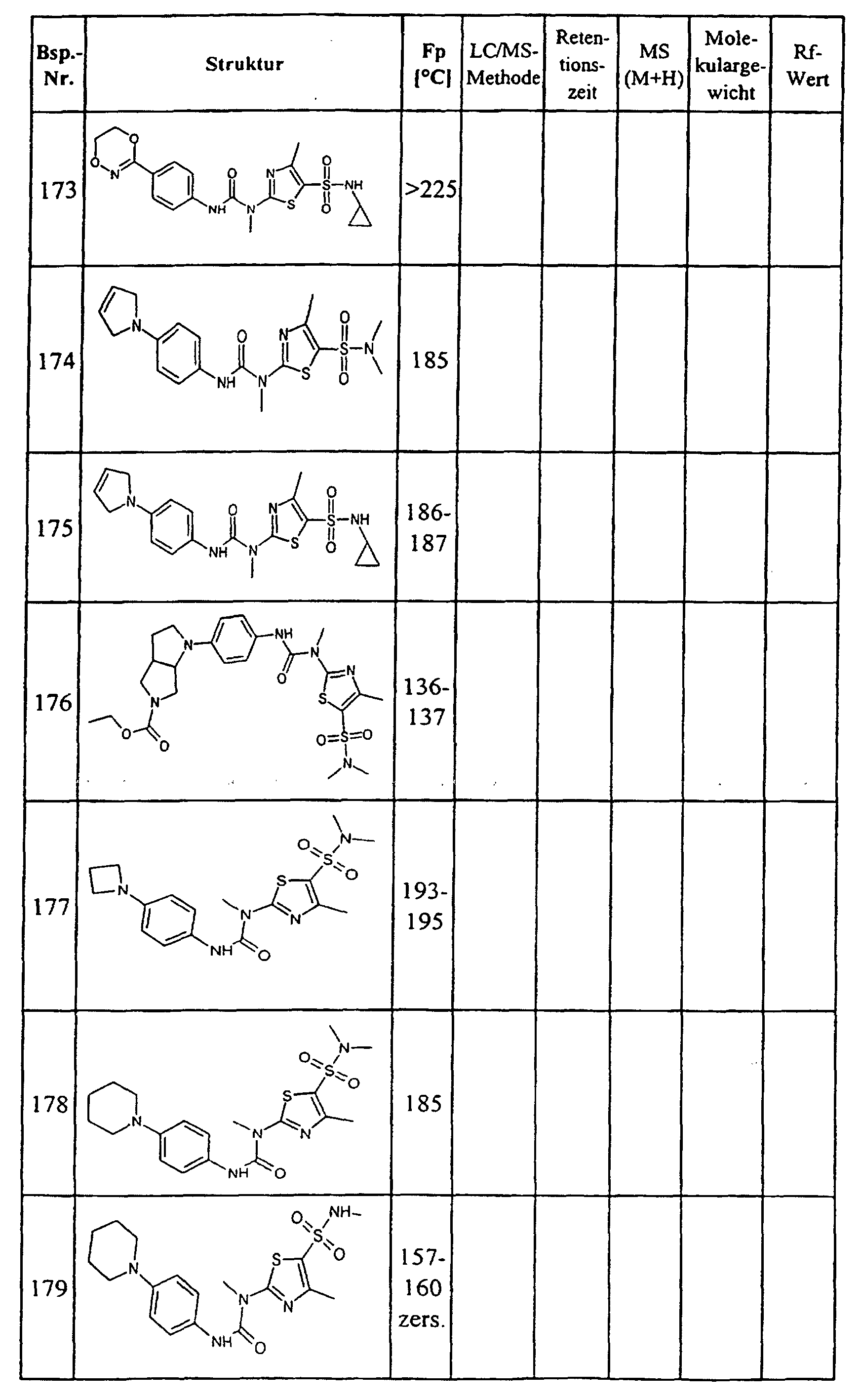

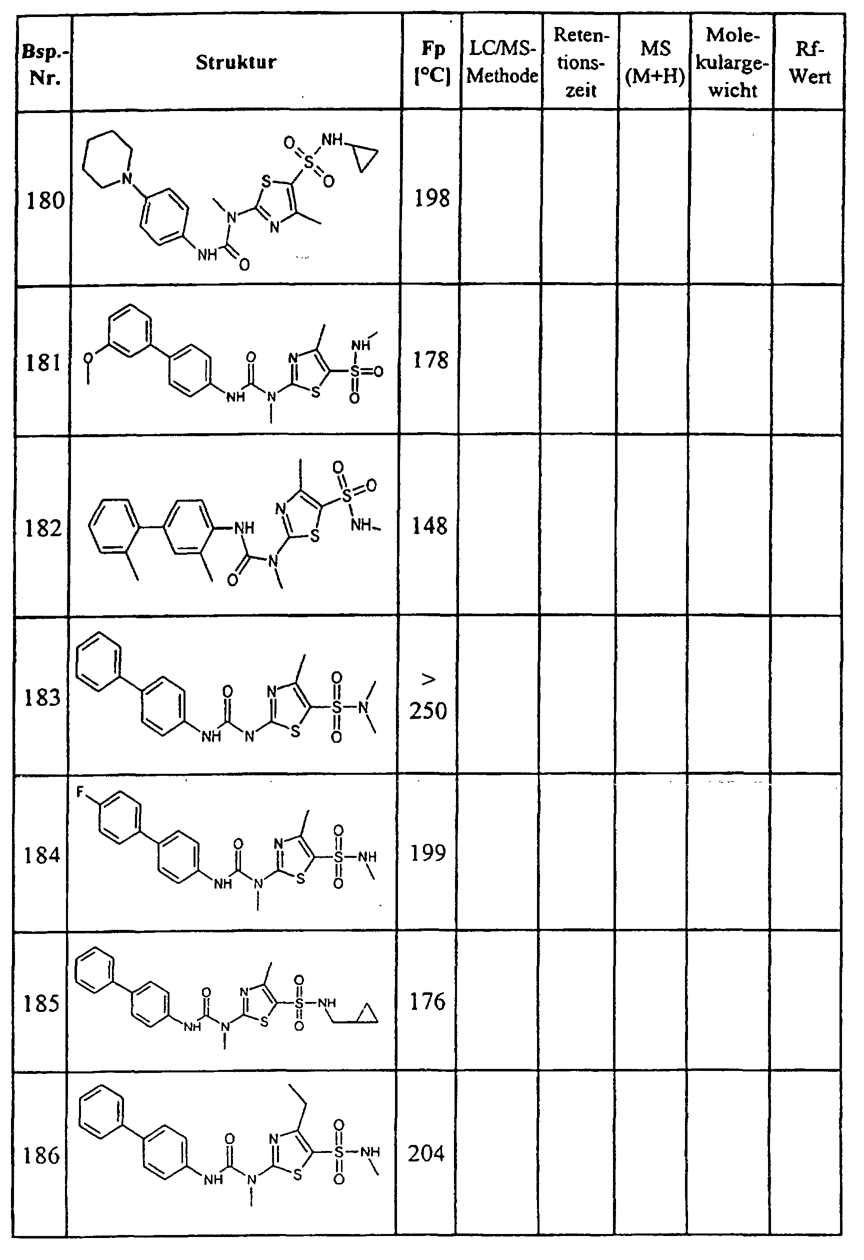

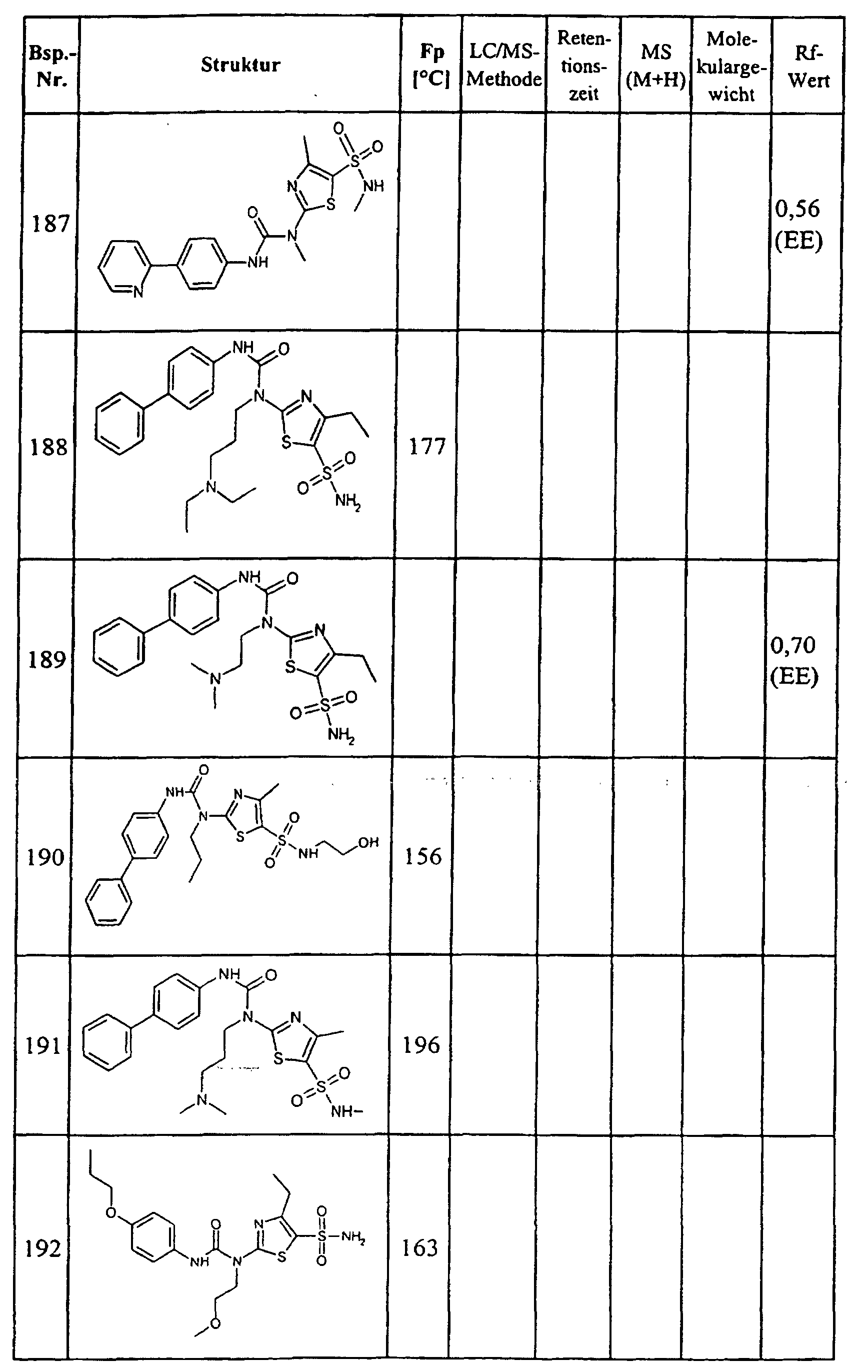

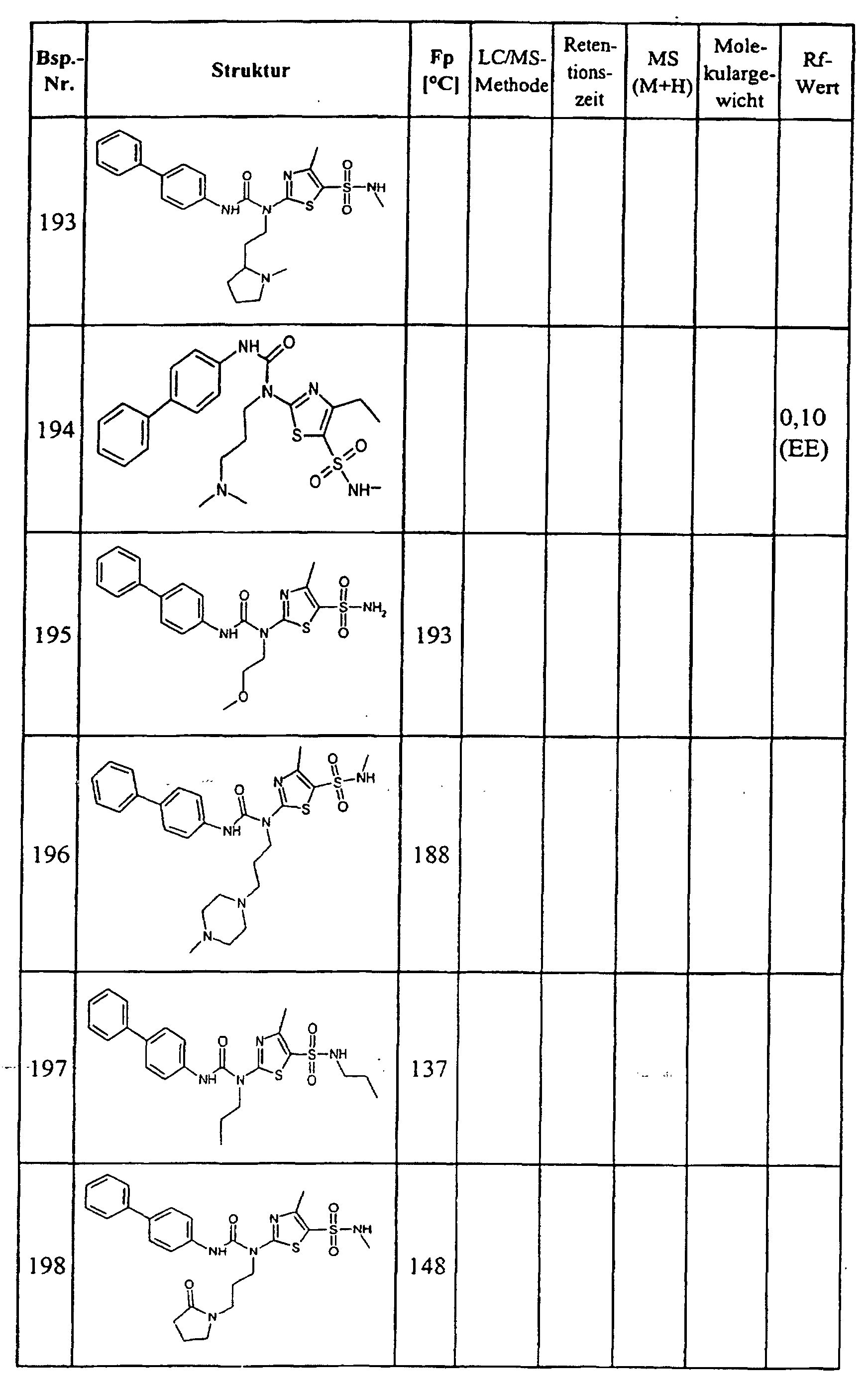

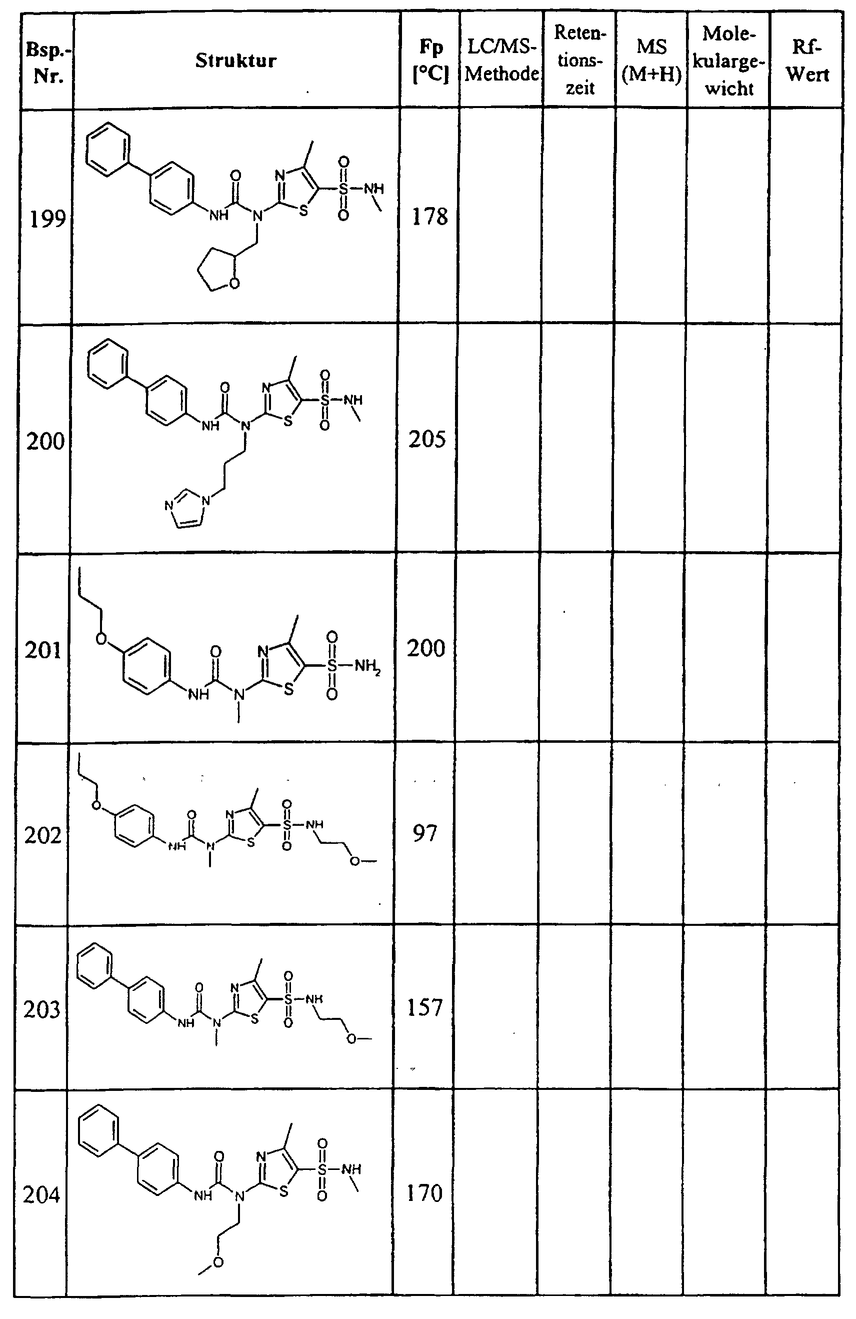

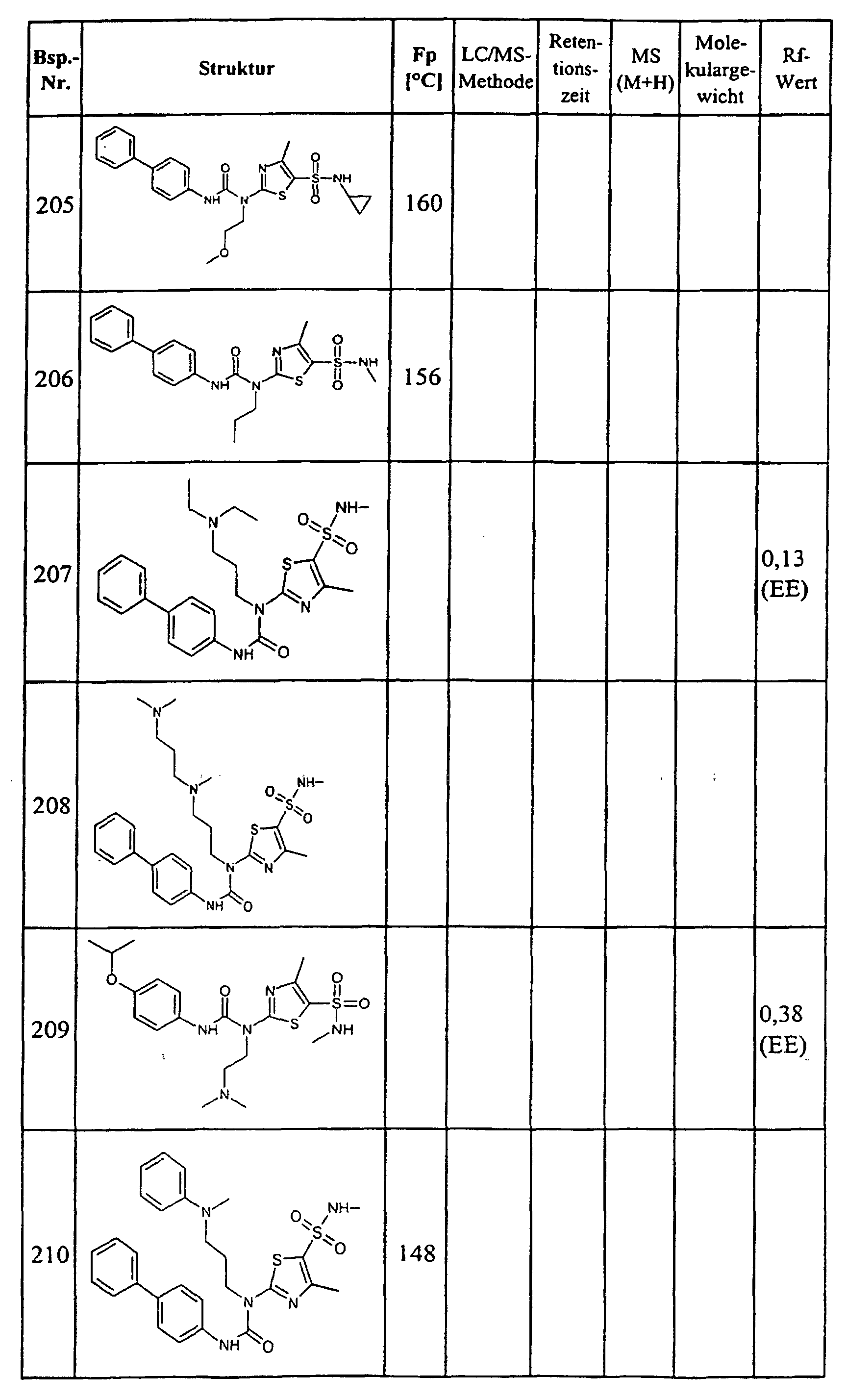

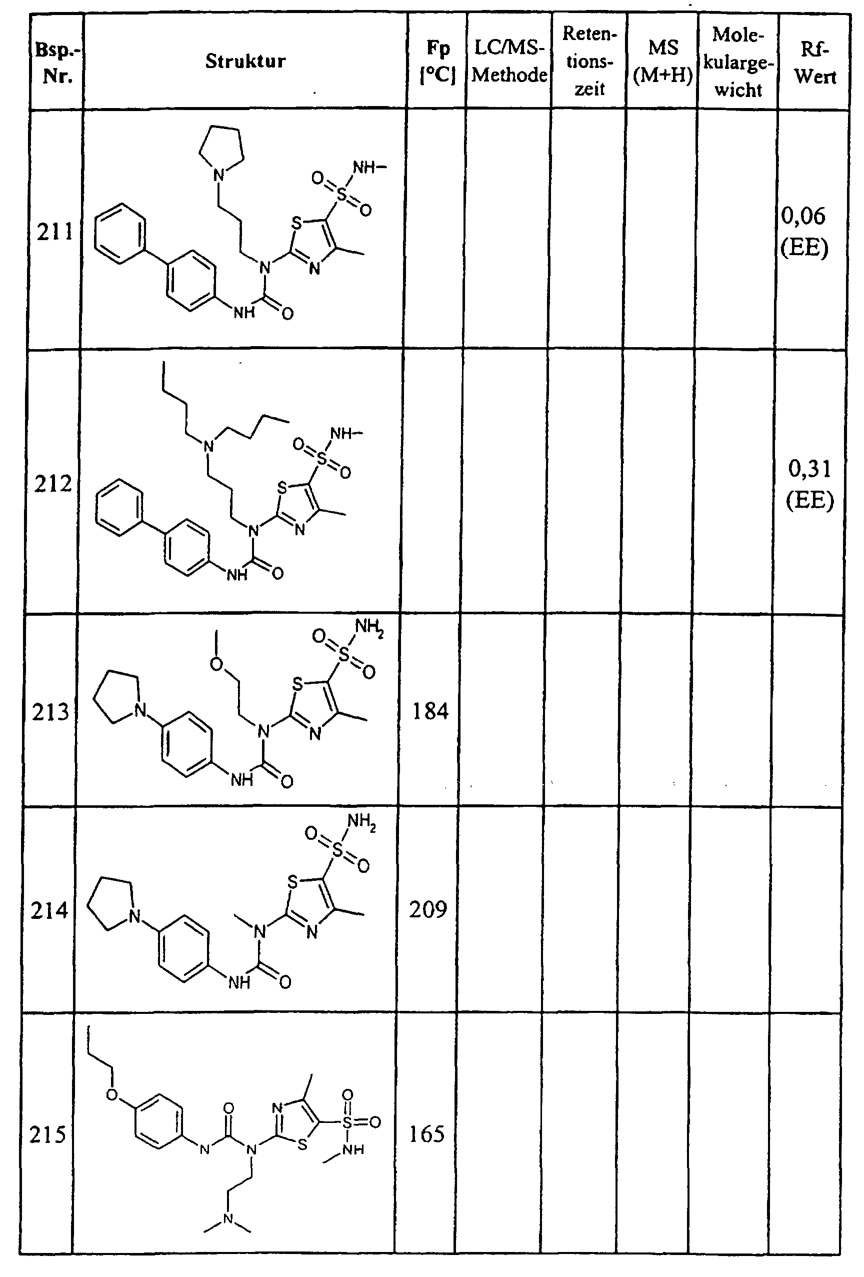

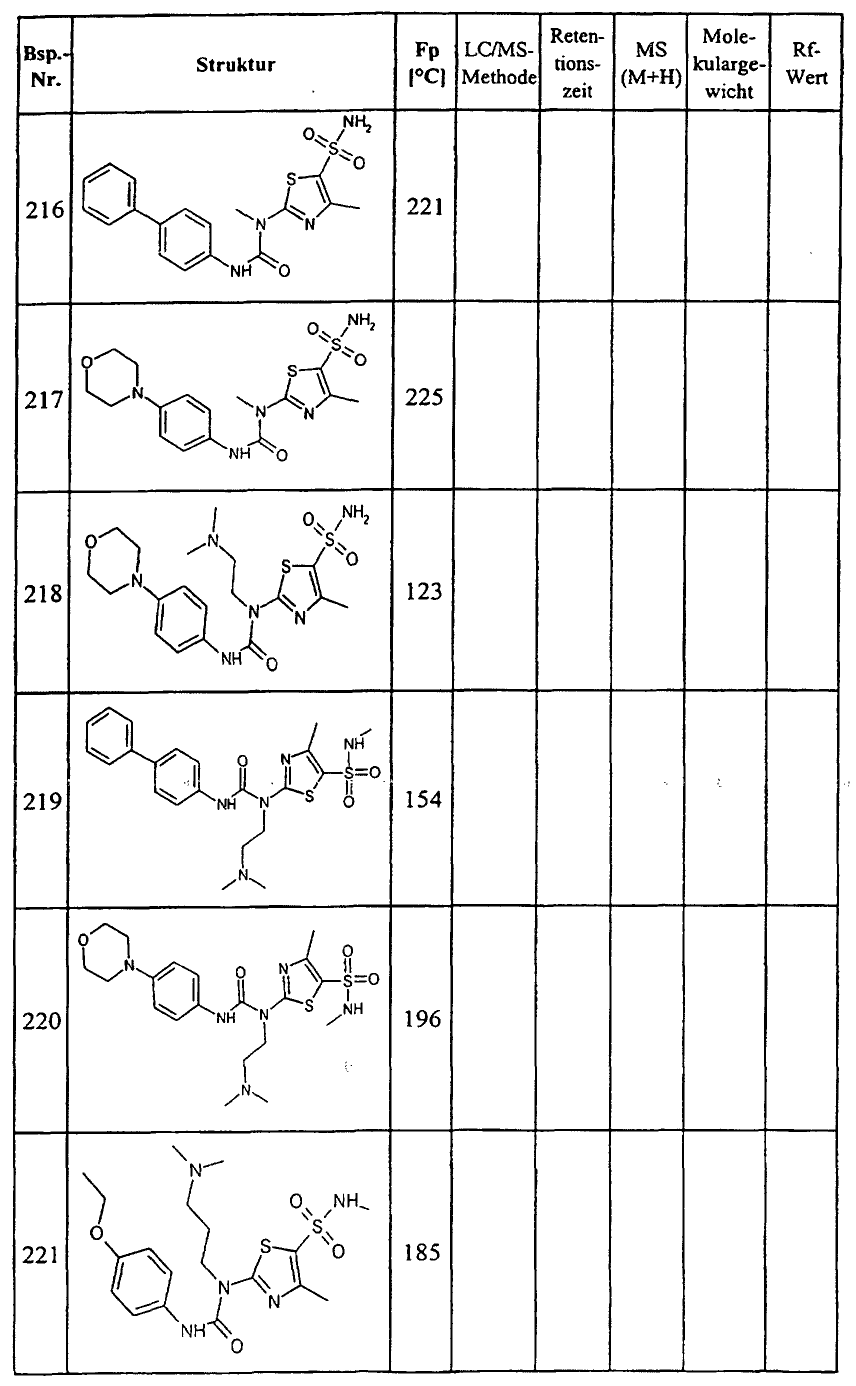

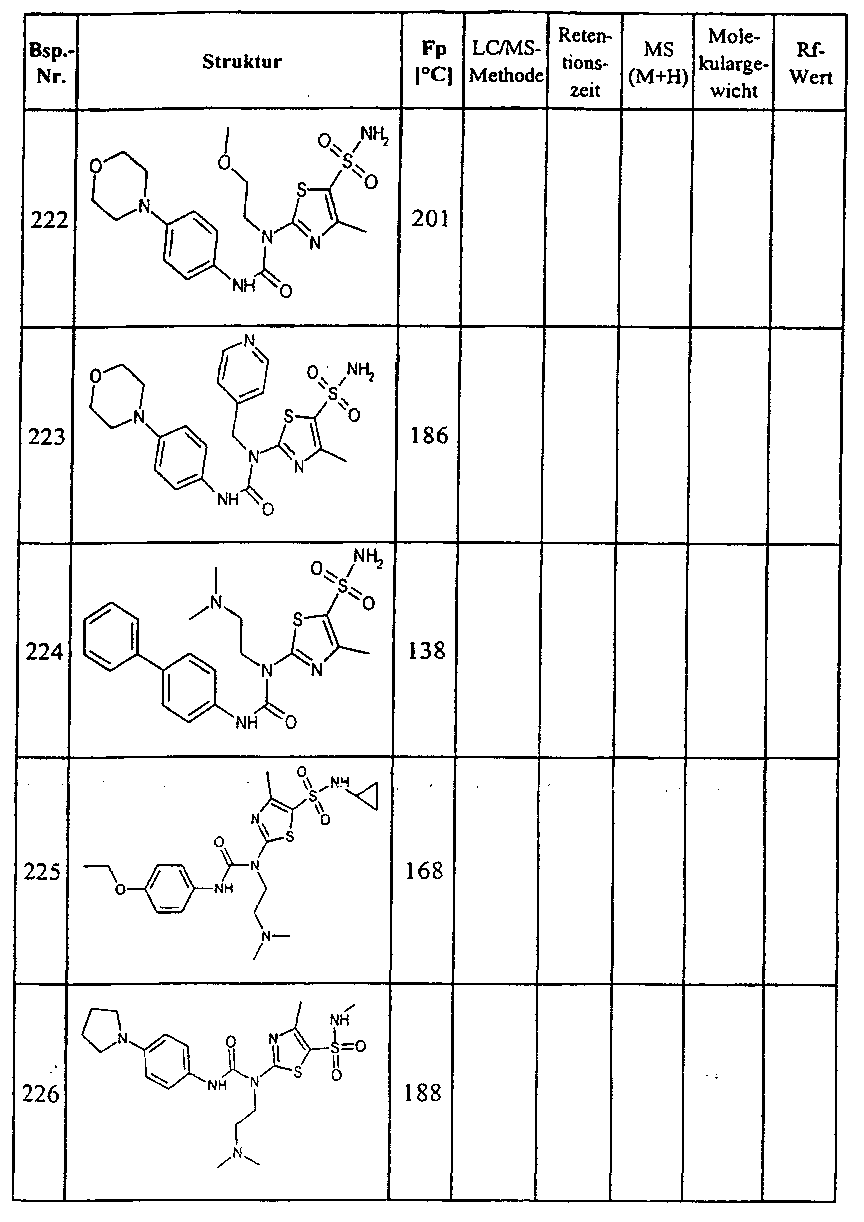

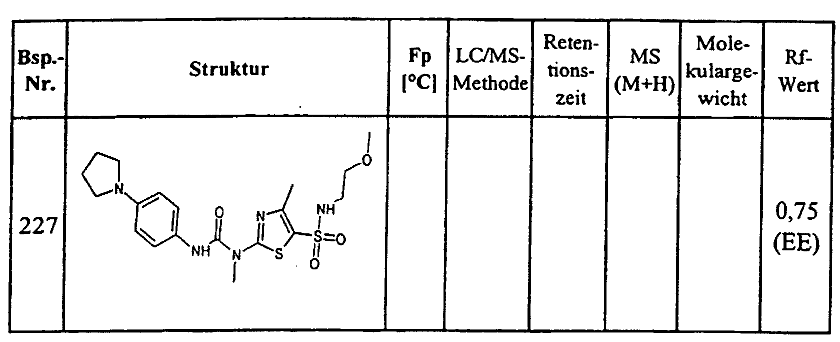

In der vorstehenden Tabelle bedeutet der Rf-Wert den Retentionsindex bei der Dünnschichtchromatographie an Kieselgel mit den folgenden Laufmitteln:
EE: Essigsäureethylester,
EP 1: Essigsäureethylester/Petrolether (Volumenverhältnis 1:1),
EP2: Essigsäureethylester/Petrolether (Volumenverhältnis 2:1)

SMKL-N1-1 bezeichnet die folgende LC-MS-Methode.

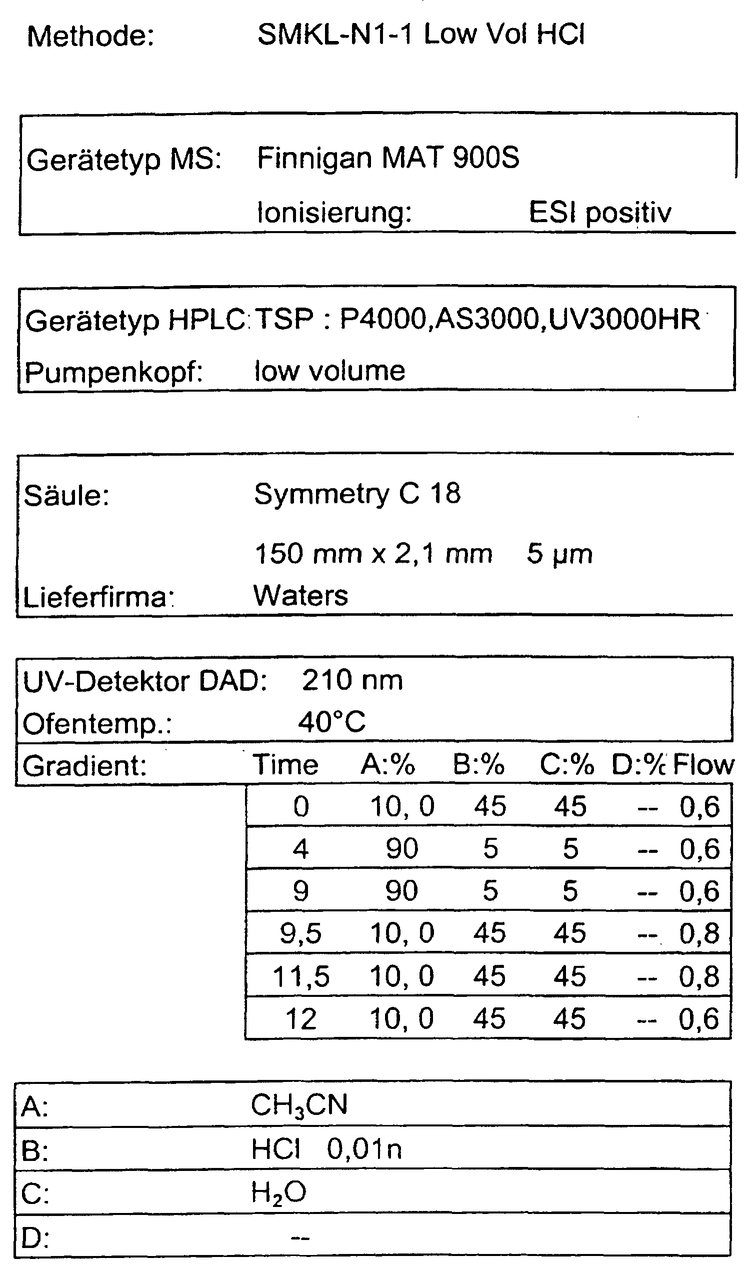

## Patentansprüche

1. Verbindungen der allgemeinen Formel (I), 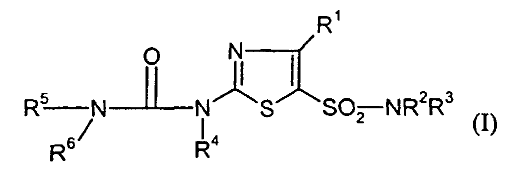 in welcher
R¹ für Wasserstoff, Halogen, (C₁-C₆)-Alkyl, (C₁-C₆)-Alkoxy, Amino(C₁-C₆)alkyl oder Halogen(C₁-C₆)alkyl steht,
R² und R³ gleich oder verschieden sind und für Wasserstoff, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Resten der Formel 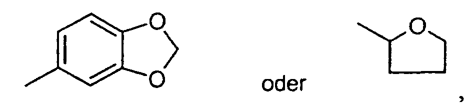 einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8- gliedrigen gesättigten oder ungesättigten, nicht aromatischen, Heterocyclus mit bis zu 3 Heteroatomen aus der Reihe S, N und/oder O, und (C₆-C₁₀)-Aryl, das seinerseits durch Hydroxy oder (C₁-C₆)-Alkoxy substituiert sein kann, besteht, oder
R² und R³ gemeinsam mit dem Stickstoffatom einen 5- bis 6-gliedrigen gesättigten Heterocyclus bildet, der gegebenenfalls noch ein Sauerstoffatom besitzen kann,
R⁴ für Wasserstoff, (C₁-C₆)-Acyl, (C₂-C₆)-Alkenyl steht oder
R⁴ für (C₁-C₆)-Alkyl steht, das gegebenfalls substituiert sein kann durch 1 bis 3 Substituenten, die aus der Gruppe ausgewählt werden, die aus Halogen, Hydroxy, (C₁-C₆)-Acyl, (C₁-C₆)-Alkoxy, Phenoxy, (C₆-C₁₀)-Aryl und -NR⁷R⁸ besteht,
worin R⁷ und R⁸ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxycarbonyl bedeuten, oder
R⁷ und R⁸ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR⁹ enthalten kann, und durch Oxo substituiert sein kann,
worin
R⁹ Wasserstoff oder (C₁-C₄)-Alkyl bedeutet, oder
R⁴ für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 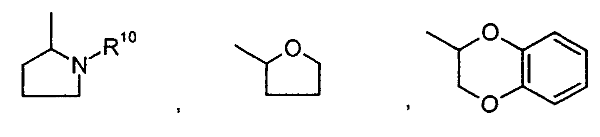 oder 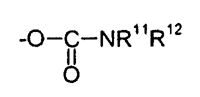 substituiert ist,
worin
R¹⁰ Wasserstoff oder (C₁-C₆)-Alkyl bedeutet,
R¹¹ und R¹² gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
R⁵ für Wasserstoff oder (C₁-C₆)-Alkyl steht,
R⁶ für einen Rest der Formel 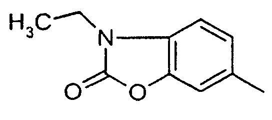 steht,
oder
R⁶ für Phenyl steht, das gegebenenfalls mit ein bis drei Substituenten substituiert sein kann, die aus der Gruppe ausgewählt werden, die aus Halogen, (C₆-C₁₀)-Aryl, das gegebenenfalls durch 1 bis 3 Substituenten, ausgewählt aus (C₁-C₆)Alkanoyl, (C₁-C₆)Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Resten der Formeln 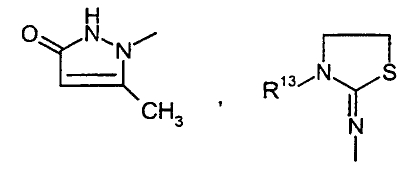 oder, 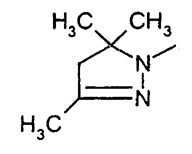 worin
R¹³ Wasserstoff oder (C₁-C₆)-Alkyl bedeutet,
(C₁-C₆)-Alkyl, das gegebenenfalls durch einen Rest der Formel 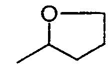 substituiert ist,
einem gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₁)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann,
einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)Alkoxycarbonylamino, (C₁-C₆)-Alkyl, Halogen(C₁-C₆)-alkyl und Hydroxy(C₁-C₆)-alkyl substituiert sein kann,
und Gruppen der Formeln -OR¹⁴, -NR¹⁵R¹⁶ oder -CO-NR¹⁷R¹⁸ besteht,
worin
R¹⁴ einen Rest der Formel 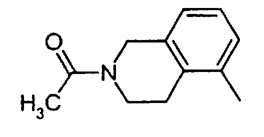 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten,
oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten,
und deren Salze.

2. Verbindungen der allgemeinen Formel (I) nach Anspruch 1, worin
R⁶ für Phenyl steht, das gegebenenfalls mit ein bis drei Substituenten substituiert sein kann, die aus der Gruppe ausgewählt werden, die aus Halogen, (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen, (C₁-C₆)-Alkyl, einem gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder 9, der gegebenenfalls durch 1 bis 3 Substituenten ausgewählt aus Oxo, Halogen, Hydroxy, (C₁-C₆)-Alkoxycarbonyl, (C₁-C₆)Alkoxycarbonylamino, (C₁-C₆)-Alkyl, Halogen(C₁-C₆)-alkyl und Hydroxy(C₁-C₆)-alkyl substituiert sein kann,
und Gruppen der Formeln -OR¹⁴, -NR¹⁵R¹⁶ oder -CO-NR¹⁷R¹⁸ besteht,
worin R¹⁴ Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten,
oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten,
und deren Salze.

3. Verbindungen der allgemeinen Formel (I) nach Anspruch 1, worin
R⁶ für Phenyl steht, das gegebenenfalls mit ein bis drei Substituenten substituiert sein kann, die aus der Gruppe ausgewählt werden, die aus Halogen, (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen und (C₁-C₆)-Alkyl besteht,
und deren Salze.

4. Verbindungen der allgemeinen Formel (I) nach Anspruch 1
in welcher
R¹ für Wasserstoff, Halogen oder für (C₁-C₆)-Alkyl steht,
R² und R³ gleich oder verschieden sind und für Wasserstoff oder (C₃-C₈)-Cycloalkyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Resten der Formel 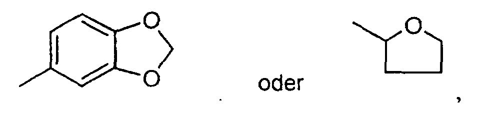 einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann, und
(C₆-C₁₀)-Aryl, das seinerseits durch Hydroxy oder (C₁-C₆)-Alkoxy substituiert sein kann, besteht, oder
R² und R³ gemeinsam mit dem Stickstoffatom einen 5- bis 6-gliedrigen gesättigten Heterocyclus bildet, der gegebenenfalls noch ein Sauerstoffatom besitzen kann,
R⁴ für Wasserstoff, (C₁-C₆)-Acyl, (C₂-C₆)-Alkenyl steht oder
R⁴ für (C₁-C₆)-Alkyl steht, das gegeben falls substituiert sein kann durch 1 bis 3 Substituenten, die aus der Gruppe ausgewählt werden, die aus Halogen, Hydroxy, (C₁-C₆)-Acyl, (C₁-C₆)-Alkoxy, Phenoxy, (C₆-C₁₀)-Aryl und -NR⁷R⁸ besteht,
worin R⁷ und R⁸ gleich oder verschieden sind und Wasserstoff, (C₁-C₆)-Acyl, (C₁-C₆)-Alkyl, Carbamoyl, Mono- oder Di(C₁-C₆)-alkylaminocarbonyl oder (C₁-C₆)-Alkoxycarbonyl bedeuten, oder
R⁷ und R⁸ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR⁹ enthalten kann,
worin
R⁹ Wasserstoff oder (C₁-C₄)-Alkyl bedeutet, oder
R⁴ für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch
Reste der Formeln 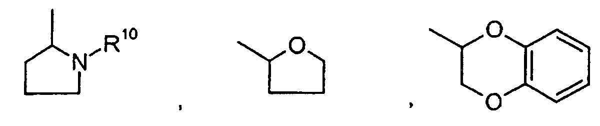 oder 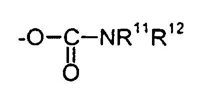 substituiert ist,
worin
R¹⁰ Wasserstoff oder (C₁-C₆)-Alkyl bedeutet,
R¹¹ und R¹² gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
R⁵ für Wasserstoff oder (C₁-C₆)-Alkyl steht,
R⁶ für einen Rest der Formel 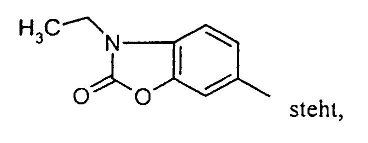
oder
R⁶ für Phenyl steht, das gegebenenfalls mit ein bis zwei Substituenten substituiert sein kann, die aus der Gruppe ausgewählt werden, die aus Halogen, (C₆-C₁₀)-Aryl, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Resten der Formeln 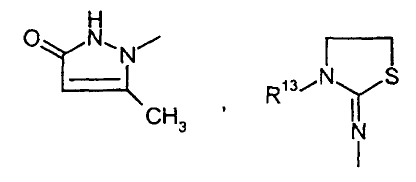 oder, 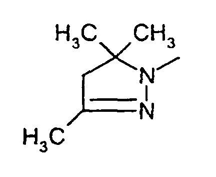 worin
R¹³ Wasserstoff oder (C₁-C₆)-Alkyl bedeutet,
(C₁-C₆)-Alkyl, das gegebenenfalls durch einen Rest der Formel 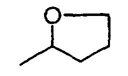 substituiert ist,
einem gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch ein bis drei Halogenatome substituiert sein kann,
einem gegebenenfalls über ein Stickstoffatom gebundenen 3- bis 8- gliedrigen gesättigten oder ungesättigten, nicht aromatischen Heterocyclus mit bis zu 3 Heteroatomen aus der Reihe S, N und/oder O der gegebenenfalls durch 1 bis 3 Substituenten ausgewählt aus Oxo, Halogen, Hydroxy, (C₁-C₆)-Alkoxycarbonyl, (C₁-C₆)-Alkyl, Halogen(C₁-C₆)-alkyl und Hydroxy(C₁-C₆)-alkyl substituiert sein kann, und Gruppen der Formeln -OR¹⁴, -NR¹⁵R¹⁶ oder -CO-NR¹⁷R¹⁸ besteht,
worin
R¹⁴ einen Rest der Formel 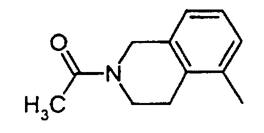 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten,
oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten,
und deren Salze.

5. Verbindungen der allgemeinen Formel (I) nach Anspruch 4,
in welcher
R¹ für Wasserstoff, Chlor oder für (C₁-C₃)-Alkyl steht,
R² und R³ gleich oder verschieden sind und für Wasserstoff oder Cyclopropyl oder Cyclopentyl stehen, oder
für (C₁-C₃)-Alkyl stehen, das gegebenenfalls durch 1 bis 3 Substituenten substituiert ist, die aus der Gruppe ausgewählt werden, die aus Cyclopropyl, Cyclopentyl, (C₁-C₃)-Alkoxy, Chlor, Fluor, Hydroxy, Resten der Formel 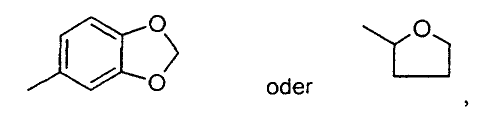 und Pyridyl, Furyl, Thienyl, Imidazolyl, N-Triazolyl oder Pyrrolyl, Phenyl, das seinerseits durch Hydroxy oder (G₁-C₃)-Alkoxy substituiert sein kann, besteht, oder
R² und R³ gemeinsam mit dem Stickstoffatom einen Morpholin-, Piperidin oder Pyrrolidinring bilden,
R⁴ für Wasserstoff, (C₁-C₃)-Acyl, (C₂-C₃)-Alkenyl steht oder
R⁴ für (C₁-C₆)-Alkyl steht, das gegebenfalls substituiert sein kann durch 1 bis 3 Substituenten, die aus der Gruppe ausgewählt werden, die aus Chlor, Fluor, Hydroxy, (C₁-C₃)-Acyl, (C₁-C₃)-Alkoxy, Phenoxy, Phenyl und -NR⁷R⁸ besteht,
worin R⁷ und R⁸ gleich oder verschieden sind und Wasserstoff, (C₁-C₄)-Acyl, (C₁-C₄)-Alkyl, Carbamoyl, Mono- oder Di(C₁-C₃)-alkylaminocarbonyl oder (C₁-C₄)-Alkoxycarbonyl bedeuten, oder
R⁷ und R⁸ gemeinsam mit dem Stickstoffatom einen Morpholino-, Piperidinyl- oder Pyrrolidinylring bilden, oder
R⁴ für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch
Reste der Formeln 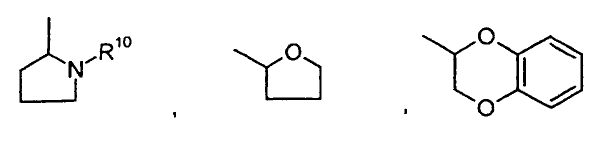 oder 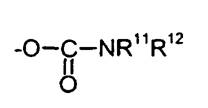 substituiert ist,
worin
R¹⁰ Wasserstoff oder (C₁-C₄)-Alkyl bedeutet,
R¹¹ und R¹² gleich oder verschieden sind und Wasserstoff, (C₁-C₃) Alkyl oder Phenyl bedeuten, wobei zuvor genanntes (C₁-C₃)-Alkyl und Phenyl gegebenenfalls durch 1 bis 3 Substituenten substituiert sein können, die aus der Gruppe ausgewählt werden, die aus Hydroxy, (C₁-C₃)-Alkoxy, Chlor und Fluor besteht,
R⁵ für Wasserstoff oder (C₁-C₃)-Alkyl steht,
R⁶ für Phenyl steht, das gegebenenfalls mit ein bis zwei Substituenten substituiert sein kann, die aus der Gruppe ausgewählt werden, die aus
Chlor, Fluor, Phenyl, (C₁-C₄)-Alkoxy, (C₁-C₄)-Alkoxycarbonyl, (C₁-C₃)-Alkylthio, Hydroxy, Carboxyl, partiell fluoriertem (C₁-C₄)-Alkoxy mit bis zu 5 Fluoratomen,
Resten der Formeln 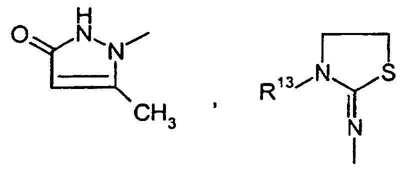 oder 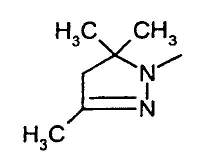 worin
R¹³ Wasserstoff oder (C₁-C₃)-Alkyl bedeutet,
(C₁-C₆)-Alkyl, das gegebenenfalls durch einen Rest der Formel 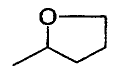 substituiert ist, Triazolyl,
Morpholino, Thiomorpholino, Piperidinyl, Pyrrolidinyl, Azacycloheptanyl, Azacyclobutanyl, die gegebenenfalls durch 1 bis 2 Substituenten ausgewählt aus Oxo, Chlor, Fluor, Hydroxy, (C₁-C₄)-Alkoxycarbonyl, (C₁-C₃)-Alkyl, Chlor- oder Fluor(C₁-C₃)-alkyl und Hydroxy(C₁-C₄)-alkyl substituiert sein können, 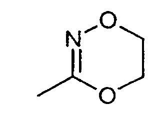 und Gruppen der Formeln -OR¹⁴, -NR¹⁵R¹⁶ oder -CO-NR¹⁷R¹⁸ besteht,
worin
R¹⁴ einen Rest der Formel 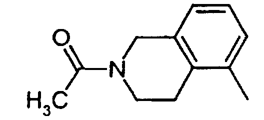 bedeutet, oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₃)-Alkyl oder (C₁-C₃)-Acyl bedeuten,
oder
R¹⁴ (C₁-C₄)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₃)alkylaminocarbonyl, Phenyl, (C₁-C₃)-Acyl oder (C₁-C₃)-Alkyl bedeuten, wobei (C₁-C₃)-Alkyl gegebenenfalls durch (C₁-C₃)-Alkoxy, (C₁-C₃)-Acyl, durch Phenyl oder Pyridyl substituiert ist,
wobei zuvor genanntes Phenyl und zuvor genanntes Pyridyl gegebenenfalls ein- bis zweifach gleich oder verschieden durch Chlor, Fluor und/oder Hydroxy substituiert sind, und
R¹⁷ und R¹⁸ gleich oder verschieden sind und Wasserstoff oder (C₁-C₄)-Alkyl bedeuten,
und deren Salze.

6. Verbindungen der allgemeinen Formel (I) nach irgendeinem der Ansprüche 1 bis 5, worin R⁵ Wasserstoff ist.

7. Verbindungen der allgemeinen Formel (I) nach irgend einem der Ansprüche 1 bis 5, worin R² und R³ Wasserstoff oder (C₁-C₃)Alkyl sind.

8. Verbindungen der allgemeinen Formel (I) nach irgend einem der Ansprüche 1 bis 7, worin R⁶ eine para-substituierte Phenylengruppe ist.

9. Verbindungen nach Anspruch 1, die die folgende Formel aufweisen: 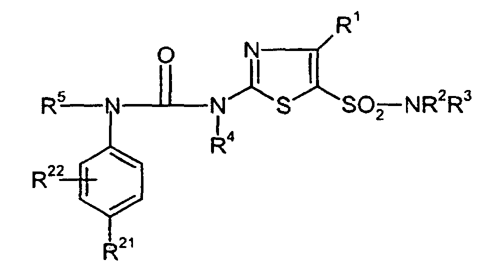 worin
R¹ bis R⁵ wie oben definiert sind und R²¹ für
Halogen, (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Reste der Formeln 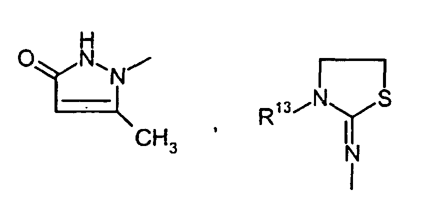 oder, 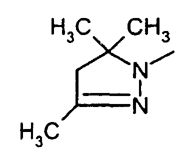 worin
R¹³ Wasserstoff oder (C₁-C₆)-Alkyl bedeutet,
(C₁-C₆)-Alkyl, das gegebenenfalls durch einen Rest der Formel 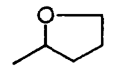 substituiert ist,
einen gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di (C₁-C₆)alkylaminocarbonyl, Mono- oder Di(C₁-C₆)acylamino, (C₁-C₆)Alkylsulfoxy, (C₁-C₆)Alkylsulfonyl, und/oder Cyano substituiert sein kann,
einen gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen(C₁-C₆)-alkyl und Hydroxy(C₁-C₆)-alkyl substituiert sein kann, oder für Gruppen der Formeln -OR¹⁴, -NR¹⁵R¹⁶ oder -CO-NR¹⁷R¹⁸ steht,
worin
R¹⁴ einen Rest der Formel 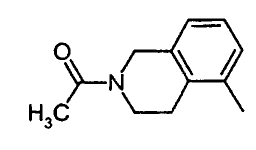 bedeutet, oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten,
oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten, und
R²² die obige Bedeutung von R²¹ aufweisen kann und mit dieser gleich oder verschieden sein kann oder R²² Wasserstoff ist,
und deren Salze.

10. Verbindungen nach Anspruch 4 , die die folgende Formel aufweisen: 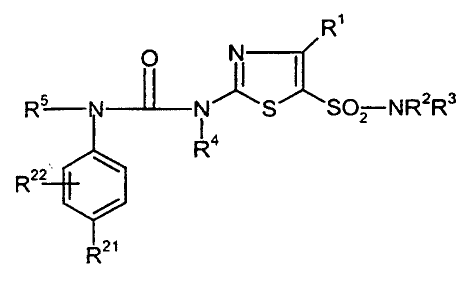 worin
R¹ bis R⁵ wie oben definiert sind und R²¹ für Halogen, (C₆-C₁₀)-Aryl, (C₁-C₆)-Alkoxy, (C₁-C₆)-Alkoxycarbonyl, (C₁-C₆)-Alkylthio, Hydroxy, Carboxyl, partiell fluoriertem (C₁-C₆)-Alkoxy mit bis zu 6 Fluoratomen,
Reste der Formeln 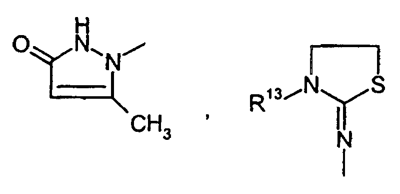 oder, 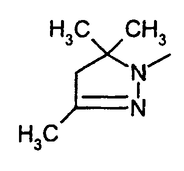 worin
R¹³ Wasserstoff oder (C₁-C₆)-Alkyl bedeutet,
(C₁-C₆)-Alkyl, das gegebenenfalls durch einen Rest der Formel 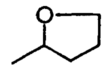 substituiert ist,
einen gegebenenfalls über ein Stickstoffatom gebundenen 5- bis 6- gliedrigen aromatischen Heterocyclus mit bis zu 3 Heteroatomen aus der Reihe S, N und/oder O, der gegebenenfalls durch ein bis drei Halogenatome substituiert sein kann,
einen gegebenenfalls über ein Stickstoffatom gebundenen 3- bis 8- gliedrigen gesättigten oder ungesättigten, nicht aromatischen Heterocyclus mit bis zu 3 Heteroatomen aus der Reihe S, N und/oder O der gegebenenfalls durch 1 bis 3 Substituenten ausgewählt aus Oxo, Halogen, Hydroxy, (C₁-C₆)-Alkoxycarbonyl, (C₁-C₆)-Alkyl, Halogen-(C₁-C₆)-alkyl und Hydroxy(C₁-C₆)-alkyl substituiert sein kann,
oder für Gruppen der Formeln -OR¹⁴, -NR¹⁵R¹⁶ oder -CO-NR¹⁷R¹⁸ steht,
worin
R¹⁴ einen Rest der Formel 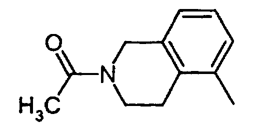 bedeutet,
oder Phenyl bedeutet, das seinerseits gegebenenfalls durch eine Gruppe der Formel -NR¹⁹R²⁰ substituiert ist,
worin
R¹⁹ und R²⁰ gleich oder verschieden sind und Wasserstoff, (C₁-C₆)-Alkyl oder (C₁-C₆)-Acyl bedeuten,
oder
R¹⁴ (C₁-C₆)-Alkyl bedeutet, das gegebenenfalls ein- bis dreifach durch Hydroxy substituiert ist,
R¹⁵ und R¹⁶ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei (C₁-C₆)-Alkyl gegebenenfalls durch (C₁-C₆)-Alkoxy, (C₁-C₆)-Acyl, durch Phenyl oder durch einen 5- bis 6- gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R¹⁷ und R¹⁸ gleich oder verschieden sind und Wasserstoff oder (C₁-C₆)-Alkyl bedeuten, und
R²² die obige Bedeutung von R²¹ aufweisen kann und mit dieser gleich oder verschieden sein kann oder R²² Wasserstoff ist.

11. Verbindungen nach Anspruch 9 oder 10, worin R²² Wasserstoff ist.

12. Verbindungen nach Anspruch 10 oder 11, worin R²¹ für Phenyl, (C₁-C₄)-Alkoxy oder einen gegebenenfalls über ein Stickstoffatom gebundenen 3- bis 8- gliedrigen gesättigten oder ungesättigten, nicht aromatischen Heterocyclus mit bis zu 3 Heteroatomen aus der Reihe S, N und/oder O steht, der gegebenenfalls durch 1 bis 3 Substituenten ausgewählt aus Oxo, Halogen, Hydroxy, (C₁-C₆)-Alkoxycarbonyl, (C₁-C₆)-Alkyl, Halogen(C₁-C₆)-alkyl und Hydroxy(C₁-C₆)-alkyl substituiert sein kann.

13. Verbindungen der allgemeinen Formel (II) 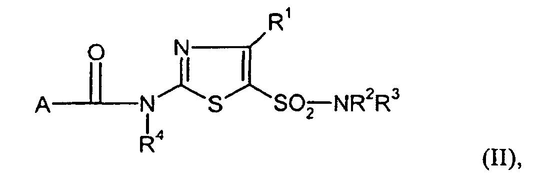 worin R¹, R², R³ und R⁴ die im Anspruch 1 angegebene Bedeutung haben, und A für ein Halogenatom steht.

14. Verfahren zur Herstellung der Verbindungen der allgemeinen Formel (I) nach Anspruch 1, **dadurch gekennzeichnet, dass** man
[A] Verbindungen der allgemeinen Formel (II) 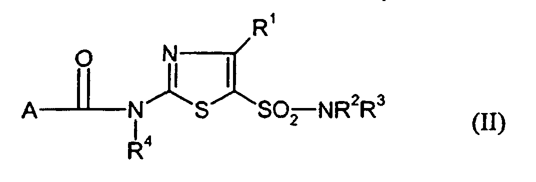 in welcher
R¹, R², R³ und R⁴ die im Anspruch 1 angegebene Bedeutung haben,
und
A für Halogen, vorzugsweise für Chlor steht,
mit Aminen der allgemeinen Formel (III)
HNR⁵R⁶ (III)
in welcher
R⁵ und R⁶ die im Anspruch 1 angegebene Bedeutung haben,
in inerten Lösemitteln, gegebenenfalls in Anwesenheit einer Base und/oder Hilfsmittels umsetzt,
oder
[B] Isocyanate der allgemeinen Formel (IV)
R⁶-NCO (IV)
in welcher
R⁶ die im Anspruch 1 angegebene Bedeutung hat,
mit Thiazolylaminen der allgemeinen Formel (V) 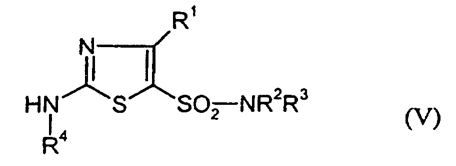 in welcher
R¹, R², R³ und R⁴ die im Anspruch 1 angegebene Bedeutung haben,
in inerten Lösemitteln umsetzt, und im Falle, dass R⁵ von Wasserstoff verschieden ist, eine Alkylierung nach üblichen Verfahren durchführt.

15. Verwendung von 2-[(Aminocarbonyl)amino]-1,3-thiazol-5-sulfonamid-Derivaten der allgemeinen Formel (I) nach Anspruch 1 zur Herstellung von Arzneimitteln.

16. Verwendung nach Anspruch 15 zur Herstellung von Mitteln zur Behandlung und/oder Prävention von viralen Infektionen bei Menschen oder Tieren.

17. Verwendung nach Anspruch 16 zur Herstellung von Mitteln zur Behandlung und/oder Prävention von viralen Infektionen bei Menschen oder Tieren durch Herpes-Viren.

18. Verwendung nach Anspruch 16 zur Herstellung von Mitteln zur Behandlung und/oder Prävention von viralen Infektionen bei Menschen oder Tieren durch Herpes Simplex-Viren.

19. Verbindungen nach Anspruch 1 zur Verwendung als Arzneimittel.

20. Pharmazeutische Zusammensetzung, die eine Verbindung der allgemeinen Formel (I) nach Anspruch 1 in Mischung mit einem pharmazeutisch verträglichen Träger oder Exzipienten umfasst.

## Claims

1. Compounds of the general formula (I), 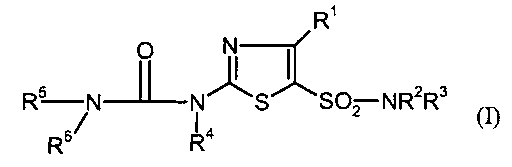 in which
R¹ represents hydrogen, halogen, (C₁-C₆)-alkyl, (C₁-C₆)-alkoxy, amino(C₁-C₆)alkyl or halogeno(C₁-C₆)alkyl,
R² and R³ are identical or different and represent hydrogen, (C₃-C₈)-cycloalkyl or biphenylylaminocarbonyl, or
represent (C₁-C₆)-alkyl which is optionally substituted by 1 to 3 substituents selected from the group consisting of (C₃-C₆)-cycloalkyl, (C₁-C₆)-alkoxy, halogen, hydroxyl, radicals of the formula 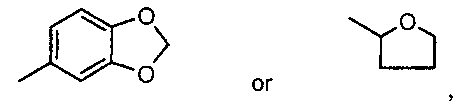 a 5- to 6-membered aromatic heterocycle with up to 3 heteroatoms from the series S, N and/or O, it also being possible for a nitrogen-containing heterocycle to be bonded via the nitrogen atom,
a 3- to 8-membered saturated or unsaturated, nonaromatic heterocycle which has up to 3 heteroatoms from the series S, N and/or O and is optionally bonded via a nitrogen atom, and (C₆-C₁₀)-aryl which in turn may be substituted by hydroxyl or (C₁-C₆)-alkoxy, or
R² and R³ form, together with the nitrogen atom, a 5- to 6-membered saturated heterocycle which may optionally also have an oxygen atom,
R⁴ represents hydrogen, (C₁-C₆)-acyl, (C₂-C₆)-alkenyl, or
R⁴ represents (C₁-C₆)-alkyl which can optionally be substituted by 1 to 3 substituents selected from the group consisting of halogen, hydroxyl, (C₁-C₆)-acyl, (C₁-C₆)-alkoxy, phenoxy, (C₆-C₁₀)-aryl and -NR⁷R⁸,
in which R⁷ and R⁸ are identical or different and denote hydrogen, (C₁-C₆)-acyl, (C₁-C₆)-alkyl, carbamoyl, mono- or di(C₁-C₆)-alkylamino(Ci-C6)alkyl, mono- or di(C₁-C₆)-alkylaminocarbonyl, (C₆-C₁₀)-aryl or (C₁-C₆)-alkoxycarbonyl, or R⁷ and R⁸ form, together with the nitrogen atom, a 5- to 6-membered saturated heterocycle which may optionally contain another heteroatom from the series S or O or a radical of the formula -NR⁹ and may be substituted by oxo,
in which R⁹ denotes hydrogen or (C₁-C₄)-alkyl, or
R⁴ represents (C₁-C₆)-alkyl which is substituted by a 5- to 6-membered aromatic, optionally benzo-fused heterocycle with up to 3 heteroatoms from the series S, N and/or O, it also being possible for a nitrogen-containing heterocycle to be bonded via the nitrogen atom, or is substituted by radicals of the formulae 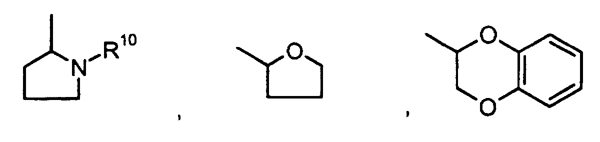 or 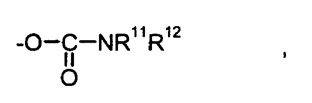 in which
R¹⁰ denotes hydrogen or (C₁-C₆)-alkyl,
R¹¹ and R¹² are identical or different and denote hydrogen, (C₁-C₆)-alkyl or (C₆-C₁₀)-aryl, it being possible for the aforementioned (C₁-C₆)-alkyl and (C₆-C₁₀)-aryl optionally to be substituted by 1 to 3 substituents selected from the group consisting of hydroxyl, (C₁-C₆)-alkoxy and halogen,
R⁵ represents hydrogen or (C₁-C₆)-alkyl,
R⁶ represents a radical of the formula 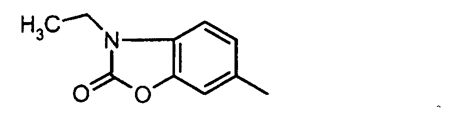
or
R⁶ represents phenyl which can optionally be substituted by one to three substituents selected from the group consisting of halogen, (C₆-C₁₀)-aryl which can optionally be substituted by 1 to 3 substituents selected from (C₁-C₆)alkanoyl, (C₁-C₆)-alkoxy, (C₁-C₆)-alkyl, halogen, (C₁-C₆)alkoxycarbonyl, nitro, halogeno(C₁-C₆)alkyl, halogeno(C₁-C₆)alkoxy, amino, (C₁-C₆)alkylthio, hydroxyl, carboxyl, carbamoyl, mono- or di(C₁-C₆)alkylaminocarbonyl, mono- or di(C₁-C₆)acylamino, (C₁-C₆)alkylsulphinyl, (C₁-C₆)alkylsulphonyl, and/or cyano, or (C₁-C₆)-alkoxy, (C₁-C₆)-alkoxycarbonyl, (C₁-C₆)-alkylthio, hydroxyl, carboxyl, partially fluorinated (C₁-C₆)-alkoxy with up to 6 fluorine atoms,
radicals of the formulae 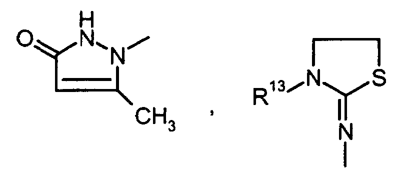 or, 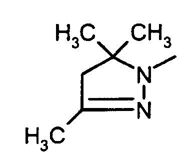 in which R¹³ denotes hydrogen or (C₁-C₆)-alkyl,
(C₁-C₆)-alkyl which is optionally substituted by a radical of the formula 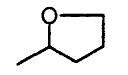 a 5- to 6-membered aromatic heterocycle which has up to 3 heteroatoms from the series S, N and/or O, is optionally bonded via a nitrogen atom and can optionally be substituted by 1 to 3 substituents selected from (C₁-C₆)alkanoyl, (C₁-C₆)-alkoxy, (C₁-C₆)-alkyl, halogen, (C₁-C₆)alkoxycarbonyl, nitro, halogeno(C₁-C₆)alkyl, halogeno(C₁-C₆)alkoxy, amino, (C₁-C₆)alkylthio, hydroxyl, carboxyl, carbamoyl, mono- or di(C₁-C₆)alkylaminocarbonyl, mono- or di(C₁-C₆)acylamino, (C₁-C₆)alkylsulphinyl, (C₁-C₆)alkylsulphonyl, and/or cyano,
a 3- to 8-membered saturated or unsaturated, nonaromatic mono- or bicyclic heterocycle which has up to 3 heteroatoms from the series S, N and/or O, is optionally bonded via a nitrogen atom and may optionally be substituted by 1 to 3 substituents selected from oxo, halogen, hydroxyl, (C₁-C₆)-alkoxycarbonyl, (C₁-C₆)alkoxycarbonylamino, (C₁-C₆)-alkyl, halogeno(C₁-C₆)-alkyl and hydroxy(C₁-C₆)-alkyl,
and groups of the formulae -OR¹⁴, -NR¹⁵R¹⁶ or -CO-NR¹⁷R¹⁸,
in which
R¹⁴ denotes a radical of the formula 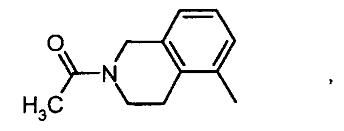 or denotes phenyl which in turn is optionally substituted by a group of the formula -NR¹⁹R²⁰,
in which
R¹⁹ and R²⁰ are identical or different and denote hydrogen, (C₁-C₆)-alkyl or (C₁-C₆)-acyl,
or
R¹⁴ denotes (C₁-C₆)-alkyl which is optionally substituted once to three times by hydroxyl,
R¹⁵ and R¹⁶ are identical or different and denote hydrogen, carbamoyl, mono- or di(C₁-C₆)alkylaminocarbonyl, phenyl, (C₁-C₆)-acyl or (C₁-C₆)-alkyl,
where (C₁-C₆)-alkyl is optionally substituted by (C₁-C₆)-alkoxy, (C₁-C₆)-acyl, by phenyl or by a 5- to 6-membered aromatic heterocycle with up to 3 heteroatoms from the series S, N and/or O,
where aforementioned phenyl and aforementioned aromatic heterocycle are optionally substituted once to three times, identically or differently, by halogen and/or hydroxyl, and
R¹⁷ and R¹⁸ are identical or different and denote hydrogen or (C₁-C₆)-alkyl,
and the salts thereof.

2. Compounds of the general formula (I) according to Claim 1, in which
R⁶ represents phenyl which may optionally be substituted by one to three substituents selected from the group consisting of halogen, (C₆-C₁₀)-aryl which can optionally be substituted by 1 to 3 substituents selected from (C₁-C₆)alkanoyl, (C₁-C₆)-alkoxy, (C₁-C₆)-alkyl, halogen, (C₁-C₆)alkoxycarbonyl, nitro, halogeno(C₁-C₆)alkyl, halogeno(C₁-C₆)alkoxy, amino, (C₁-C₆)alkylthio, hydroxyl, carboxyl, carbamoyl, mono- or di(C₁-C₆)alkylaminocarbonyl, mono- or di(C₁-C₆)acylamino, (C₁-C₆)alkylsulphinyl, (C₁-C₆)alkylsulphonyl, and/or cyano, or of (C₁-C₆)-alkoxy, (C₁-C₆)-alkoxycarbonyl, (C₁-C₆)-alkylthio, hydroxyl, carboxyl, partially fluorinated (C₁-C₆)-alkoxy with up to 6 fluorine atoms, (C₁-C₆)-alkyl, a 5- to 6-membered aromatic heterocycle which has up to 3 heteroatoms from the series S, N and/or O, is optionally bonded via a nitrogen atom and may optionally be substituted by 1 to 3 substituents selected from (C₁-C₆)alkanoyl, (C₁-C₆)-alkoxy, (C₁-C₆)-alkyl, halogen, (C₁-C₆)alkoxycarbonyl, nitro, halogeno(C₁-C₆)alkyl, halogeno(C₁-C₆)alkoxy, amino, (C₁-C₆)alkylthio, hydroxyl, carboxyl, carbamoyl, mono- or di(C₁-C₆)alkylaminocarbonyl, mono- or di(C₁-C₆)acylamino, (C₁-C₆)alkylsulphinyl, (C₁-C₆)alkylsulphonyl, and/or cyano, or of a 3- to 8-membered saturated or unsaturated, nonaromatic, mono- or bicyclic heterocycle which has up to 3 heteroatoms from the series S, N and/or O, is optionally bonded via a nitrogen atom and can optionally be substituted by 1 to 3 substituents selected from oxo, halogen, hydroxyl, (C₁-C₆)-alkoxycarbonyl, (C₁-C₆)alkoxycarbonylamino, (C₁-C₆)-alkyl, halogeno(C₁-C₆)-alkyl and hydroxy(C₁-C₆)-alkyl,
and groups of the formulae -OR¹⁴, -NR¹⁵R¹⁶ or -CO-NR¹⁷R¹⁸,
in which R¹⁴ is phenyl, which in turn is optionally substituted by a group of the formula -NR¹⁹R²⁰,
in which
R¹⁹ and R²⁰ are identical or different and denote hydrogen, (C₁-C₆)-alkyl or (C₁-C₆)-acyl,
or
R¹⁴ denotes (C₁-C₆)-alkyl which is optionally substituted once to three times by hydroxyl,
R¹⁵ and R¹⁶ are identical or different and denote hydrogen, carbamoyl, mono- or di(C₁-C₆)alkylaminocarbonyl, phenyl, (C₁-C₆)-acyl or (C₁-C₆)-alkyl,
where (C₁-C₆)-alkyl is optionally substituted by (C₁-C₆)-alkoxy, (C₁-C₆)-acyl, by phenyl or by a 5- to 6-membered aromatic heterocycle with up to 3 heteroatoms from the series S, N and/or O,
where aforementioned phenyl and aforementioned aromatic heterocycle are optionally substituted once to three times, identically or differently, by halogen and/or hydroxyl, and
R¹⁷ and R¹⁸ are identical or different and denote hydrogen or (C₁-C₆)-alkyl,
and the salts thereof.

3. Compounds of the general formula (I) according to Claim 1, in which
R⁶ represents phenyl which may optionally be substituted by one to three substituents selected from the group consisting of halogen, (C₆-C₁₀)-aryl which may optionally be substituted by 1 to 3 substituents selected from (C₁-C₆)alkanoyl, (C₁-C₆)-alkoxy, (C₁-C₆)-alkyl, halogen, (C₁-C₆)alkoxycarbonyl, nitro, halogeno(C₁-C₆)alkyl, halogeno(C₁-C₆)alkoxy, amino, (C₁-C₆)alkylthio, hydroxyl, carboxyl, carbamoyl, mono- or di(C₁-C₆)alkylaminocarbonyl, mono- or di(C₁-C₆)acylamino, (C₁-C₆)alkylsulphinyl, (C₁-C₆)alkylsulphonyl, and/or cyano, or (C₁-C₆)-alkoxy, (C₁-C₆)-alkoxycarbonyl, (C₁-C₆)-alkylthio, hydroxyl, carboxyl, partially fluorinated (C₁-C₆)-alkoxy with up to 6 fluorine atoms and (C₁-C₆)-alkyl,
and the salts thereof.

4. Compounds of the general formula (I) according to Claim 1, in which
R¹ represents hydrogen, halogen or represents (C₁-C₆)-alkyl,
R² and R³ are identical or different and represent hydrogen or (C₃-C₈)-cycloalkyl, or
represent (C₁-C₆)-alkyl which is optionally substituted by 1 to 3 substituents selected from the group consisting of (C₃-C₆)-cycloalkyl, (C₁-C₆)-alkoxy, halogen, hydroxyl, radicals of the formula 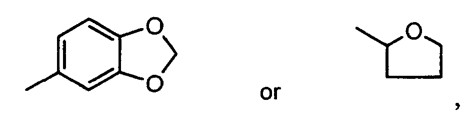 a 5- to 6-membered aromatic heterocycle with up to 3 heteroatoms from the series S, N and/or O, it also being possible for a nitrogen-containing heterocycle to be bonded via the nitrogen atom, and
(C₆-C₁₀)-aryl which in turn can be substituted by hydroxyl or (C₁-C₆)-alkoxy, or
R² and R³ form, together with the nitrogen atom, a 5- to 6-membered saturated heterocycle which may optionally also have an oxygen atom,
R⁴ represents hydrogen, (C₁-C₆)-acyl, (C₂-C₆)-alkenyl or
R⁴ represents (C₁-C₆)-alkyl which can optionally be substituted by 1 to 3 substituents selected from the group consisting of halogen, hydroxyl, (C₁-C₆)-acyl, (C₁-C₆)-alkoxy, phenoxy, (C₆-C₁₀)-aryl and -NR⁷R⁸,
in which R⁷ and R⁸ are identical or different and denote hydrogen, (C₁-C₆)-acyl, (C₁-C₆)-alkyl, carbamoyl, mono- or di(C₁-C₆)-alkylaminocarbonyl or (C₁-C₆)-alkoxycarbonyl, or R⁷ and R⁸ form, together with the nitrogen atom, a 5- to 6-membered saturated heterocycle which may optionally contain another heteroatom from the series S or O or a radical of the formula -NR⁹,
in which R⁹ denotes hydrogen or (C₁-C₄)-alkyl, or
R⁴ represents (C₁-C₆)-alkyl which is substituted by a 5- to 6-membered aromatic, optionally benzo-fused heterocycle with up to 3 heteroatoms from the series S, N and/or O, it being possible for a nitrogen-containing heterocycle also to be bonded via the nitrogen atom, or is substituted by radicals of the formulae 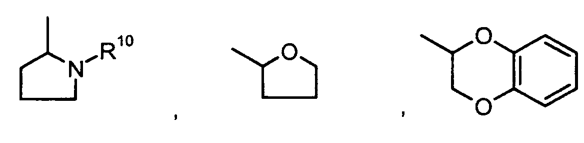 or 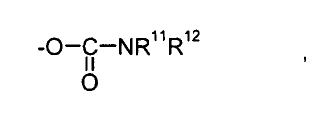 in which
R¹⁰ denotes hydrogen or (C₁-C₆)-alkyl,
R¹¹ and R¹² are identical or different and denote hydrogen, (C₁-C₆)-alkyl or (C₆-C₁₀)-aryl, where aforementioned (C₁-C₆)-alkyl and (C₆-C₁₀)-aryl can optionally be substituted by 1 to 3 substituents selected from the group consisting of hydroxyl, (C₁-C₆)-alkoxy and halogen,
R⁵ represents hydrogen or (C₁-C₆)-alkyl,
R⁶ represents a radical of the formula 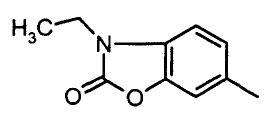
or
R⁶ represents phenyl which can optionally be substituted by one to two substituents selected from the group consisting of
halogen, (C₆-C₁₀)-aryl, (C₁-C₆)-alkoxy, (C₁-C₆)-alkoxycarbonyl, (C₁-C₆)-alkylthio, hydroxyl, carboxyl, partially fluorinated (C₁-C₆)-alkoxy with up to 6 fluorine atoms,
radicals of the formulae 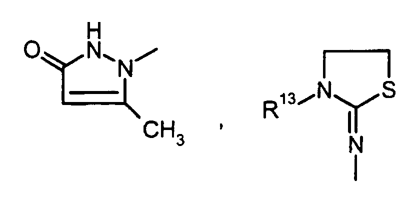 or 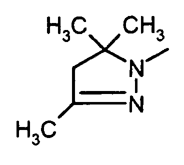 in which R¹³ denotes hydrogen or (C₁-C₆)-alkyl,
(C₁-C₆)-alkyl, which is optionally substituted by a radical of the formula 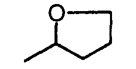 a 5- to 6-membered aromatic heterocycle which has up to 3 heteroatoms from the series S, N and/or O, is optionally bonded via a nitrogen atom and can optionally be substituted by one to three halogen atoms,
a 3- to 8-membered, saturated or unsaturated, nonaromatic heterocycle which has up to 3 heteroatoms from the series S, N and/or O, is optionally bonded via a nitrogen atom and can optionally be substituted by 1 to 3 substituents selected from oxo, halogen, hydroxyl, (C₁-C₆)-alkoxycarbonyl, (C₁-C₆)-alkyl, halogeno(C₁-C₆)-alkyl and hydroxy(C₁-C₆)-alkyl,
and groups of the formulae -OR¹⁴, -NR¹⁵R¹⁶ or -CO-NR¹⁷R¹⁸,
in which
R¹⁴ denotes a radical of the formula 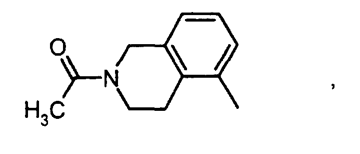 or phenyl which in turn is optionally substituted by a group of the formula -NR¹⁹R²⁰,
in which
R¹⁹ and R²⁰ are identical or different and denote hydrogen, (C₁-C₆)-alkyl or (C₁-C₆)-acyl,
or
R¹⁴ denotes (C₁-C₆)-alkyl which is optionally substituted once to three times by hydroxyl,
R¹⁵ and R¹⁶ are identical or different and denote hydrogen, carbamoyl, mono- or di(C₁-C₆)alkylaminocarbonyl, phenyl, (C₁-C₆)-acyl or (C₁-C₆)-alkyl,
where (C₁-C₆)-alkyl is optionally substituted by (C₁-C₆)-alkoxy, (C₁-C₆)-acyl, by phenyl or by a 5- to 6-membered aromatic heterocycle with up to 3 heteroatoms from the series S, N and/or O,
where aforementioned phenyl and aforementioned aromatic heterocycle are optionally substituted once to three times, identically or differently, by halogen and/or hydroxyl, and
R¹⁷ and R¹⁸ are identical or different and denote hydrogen or (C₁-C₆)-alkyl,
and the salts thereof.

5. Compounds of the general formula (I) according to Claim 4,
in which
R¹ represents hydrogen, chlorine or represents (C₁-C₃)-alkyl,
R² and R³ are identical or different and represent hydrogen or cyclopropyl or cyclopentyl, or
represent (C₁-C₃)-alkyl which is optionally substituted by 1 to 3 substituents selected from the group consisting of cyclopropyl, cyclopentyl, (C₁-C₃)-alkoxy, chlorine, fluorine, hydroxyl, radicals of the formula 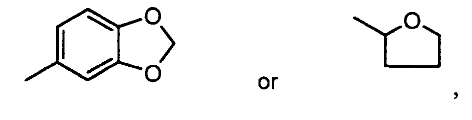 and pyridyl, furyl, thienyl, imidazolyl, N-triazolyl or pyrrolyl, phenyl which in turn may be substituted by hydroxyl or (C₁-C₃)-alkoxy, or
R² and R³ form, together with the nitrogen atom, a morpholine, piperidine or pyrrolidine ring,
R⁴ represents hydrogen, (C₁-C₃)-acyl, (C₂-C₃)-alkenyl, or
R⁴ represents (C₁-C₆)-alkyl which can optionally be substituted by 1 to 3 substituents selected from the group consisting of chlorine, fluorine, hydroxyl, (C₁-C₃)-acyl, (C₁-C₃)-alkoxy, phenoxy, phenyl and -NR⁷R⁸,
in which R⁷ and R⁸ are identical or different and denote hydrogen, (C₁-C₄)-acyl, (C₁-C₄)-alkyl, carbamoyl, mono- or di(C₁-C₃)-alkylaminocarbonyl or (C₁-C₄)-alkoxycarbonyl, or
R⁷ and R⁸ form, together with the nitrogen atom, a morpholino, piperidinyl or pyrrolidinyl ring, or
R⁴ represents (C₁-C₆)-alkyl which is substituted by a 5- to 6-membered aromatic, optionally benzo-fused heterocycle with up to 3 heteroatoms from the series S, N and/or O, it also being possible for a nitrogen-containing heterocycle to be bonded via the nitrogen atom, or is substituted by radicals of the formulae 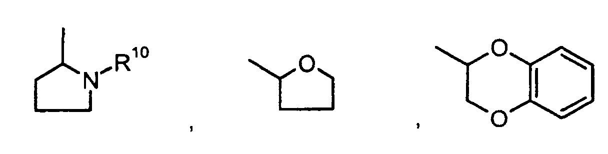 or 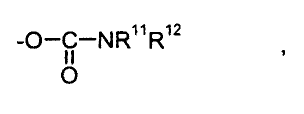 in which
R¹⁰ denotes hydrogen or (C₁-C₄)-alkyl,
R¹¹ and R¹² are identical or different and denote hydrogen, (C₁-C₃)-alkyl or phenyl, where aforementioned (C₁-C₃)-alkyl and phenyl can optionally be substituted by 1 to 3 substituents selected from the group consisting of hydroxyl, (C₁-C₃)-alkoxy, chlorine and fluorine,
R⁵ represents hydrogen or (C₁-C₃)-alkyl,
R⁶ represents phenyl which may be optionally be substituted by one to two substituents selected from the group consisting of
chlorine, fluorine, phenyl, (C₁-C₄)-alkoxy, (C₁-C₄)-alkoxycarbonyl, (C₁-C₃)-alkylthio, hydroxyl, carboxyl, partially fluorinated (C₁-C₄)-alkoxy with up to 5 fluorine atoms,
radicals of the formulae 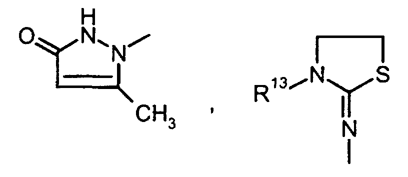 or , 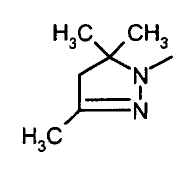 in which R¹³ denotes hydrogen or (C₁-C₃)-alkyl,
(C₁-C₆)-alkyl which is optionally substituted by a radical of the formula 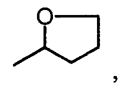 triazolyl,
morpholino, thiomorpholino, piperidinyl, pyrrolidinyl, azacycloheptanyl, azacyclobutanyl, each of which may optionally be substituted by 1 to 2 substituents selected from oxo, chlorine, fluorine, hydroxyl, (C₁-C₄)-alkoxycarbonyl, (C₁-C₃)-alkyl, chloro- or fluoro(C₁-C₃)-alkyl and hydroxy(C₁-C₄)-alkyl, 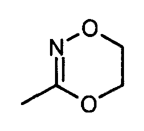 and groups of the formulae -OR¹⁴, -NR¹⁵R¹⁶ or -CO-NR¹⁷R¹⁸,
in which
R¹⁴ denotes a radical of the formula 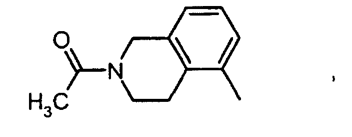 or
or phenyl which is in turn optionally substituted by a group of the formula -NR¹⁹R²⁰,
in which
R¹⁹ and R²⁰ are identical or different and denote hydrogen, (C₁-C₃)-alkyl or (C₁-C₃)-acyl,
R¹⁴ denotes (C₁-C₄)-alkyl which is optionally substituted once to three times by hydroxyl,
R¹⁵ and R¹⁶ are identical or different and denote hydrogen, carbamoyl, mono- or di(C₁-C₃)alkylaminocarbonyl, phenyl, (C₁-C₃)-acyl or (C₁-C₃)-alkyl, where (C₁-C₃)-alkyl is optionally substituted by (C₁-C₃)-alkoxy, (C₁-C₃)-acyl, by phenyl or pyridyl,
where aforementioned phenyl and aforementioned pyridyl are optionally substituted once to twice, identically or differently, by chlorine, fluorine and/or hydroxyl, and
R¹⁷ and R¹⁸ are identical or different and denote hydrogen or (C₁-C₄)-alkyl,
and the salts thereof.

6. Compounds of the general formula (I) according to any of Claims 1 to 5, in which R⁵ is hydrogen.

7. Compounds of the general formula (I) according to any of Claims 1 to 5, in which R² and R³ are hydrogen or (C₁-C₃)alkyl.

8. Compounds of the general formula (I) according to any of Claims 1 to 7, in which R⁶ is a para-substituted phenyl group.

9. Compounds according to Claim 1, which have the following formula: 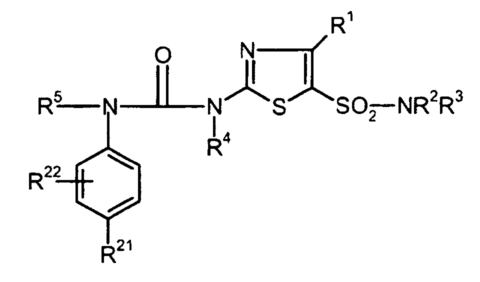 in which
R¹ to R⁵ are as defined above, and R²¹ represents
halogen, (C₆-C₁₀)-aryl which may optionally be substituted by 1 to 3 substituents selected from (C₁-C₆)alkanoyl, (C₁-C₆)-alkoxy, (C₁-C₆)-alkyl, halogen, (C₁-C₆)alkoxycarbonyl, nitro, halogeno(C₁-C₆)alkyl, halogeno(C₁-C₆)alkoxy, amino, (C₁-C₆)alkylthio, hydroxyl, carboxyl, carbamoyl, mono- or di(C₁-C₆)alkylaminocarbonyl, mono- or di(C₁-C₆)acylamino, (C₁-C₆)alkylsulphinyl, (C₁-C₆)alkylsulphonyl, and/or cyano, or (C₁-C₆)-alkoxy, (C₁-C₆)-alkoxycarbonyl, (C₁-C₆)-alkylthio, hydroxyl, carboxyl, partially fluorinated (C₁-C₆)-alkoxy with up to 6 fluorine atoms,
radicals of the formulae 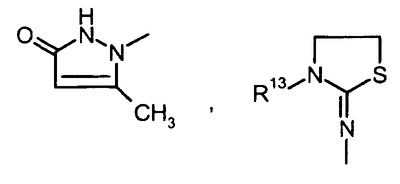 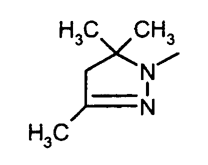 in which R¹³ denotes hydrogen or (C₁-C₆)-alkyl,
(C₁-C₆)-alkyl which is optionally substituted by a radical of the formula 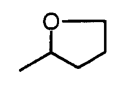 a 5- to 6-membered aromatic heterocycle which has up to 3 heteroatoms from the series S, N and/or O, is optionally bonded via a nitrogen atom and can optionally be substituted by 1 to 3 substituents selected from (C₁-C₆)alkanoyl, (C₁-C₆)-alkoxy, (C₁-C₆)-alkyl, halogen, (C₁-C₆)alkoxycarbonyl, nitro, halogeno(C₁-C₆)alkyl, halogeno(C₁-C₆)alkoxy, amino, (C₁-C₆)alkylthio, hydroxyl, carboxyl, carbamoyl, mono- or di(C₁-C₆)alkylaminocarbonyl, mono- or di(C₁-C₆)acylamino, (C₁-C₆)alkylsulphinyl, (C₁-C₆)alkylsulphonyl, and/or cyano,
a 3- to 8-membered saturated or unsaturated, nonaromatic mono- or bicyclic heterocycle which has up to 3 heteroatoms from the series S, N and/or O, is optionally bonded via a nitrogen atom and may optionally be substituted by 1 to 3 substituents selected from oxo, halogen, hydroxyl, (C₁-C₆)-alkoxycarbonyl, (C₁-C₆)-alkoxycarbonylamino, (C₁-C₆)-alkyl, halogeno(C₁-C₆)-alkyl and hydroxy(C₁-C₆)-alkyl,
and groups of the formulae -OR¹⁴, -NR¹⁵R¹⁶ or -CO-NR¹⁷R¹⁸,
in which
R¹⁴ denotes a radical of the formula 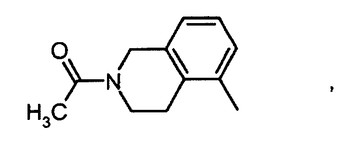 or denotes phenyl which in turn is optionally substituted by a group of the formula -NR¹⁹R²⁰,
in which
R¹⁹ and R²⁰ are identical or different and denote hydrogen, (C₁-C₆)-alkyl or (C₁-C₆)-acyl,
or
R¹⁴ denotes (C₁-C₆)-alkyl which is optionally substituted once to three times by hydroxyl,
R¹⁵ and R¹⁶ are identical or different and denote hydrogen, carbamoyl, mono- or di(C₁-C₆)alkylaminocarbonyl, phenyl, (C₁-C₆)-acyl or (C₁-C₆)-alkyl,
where (C₁-C₆)-alkyl is optionally substituted by (C₁-C₆)-alkoxy, (C₁-C₆)-acyl, by phenyl or by a 5- to 6-membered aromatic heterocycle with up to 3 heteroatoms from the series S, N and/or O,
where aforementioned phenyl and aforementioned aromatic heterocycle are optionally substituted once to three times, identically or differently, by halogen and/or hydroxyl, and
R¹⁷ and R¹⁸ are identical or different and denote hydrogen or (C₁-C₆)-alkyl, and salts thereof
R²² may have the above meaning of R²¹ and may be identical to or different from the latter, or R²² is hydrogen, and their salts.

10. Compounds according to Claim 4, which have the following formula: 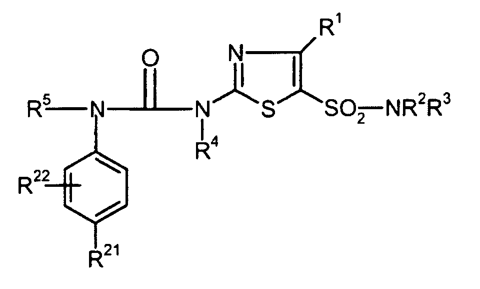 in which
R¹ to R⁵ are as defined above, and R²¹ represents halogen, (C₆-C₁₀)-aryl, (C₁-C₆)-alkoxy, (C₁-C₆)-alkoxycarbonyl, (C₁-C₆)-alkylthio, hydroxyl, carboxyl, partially fluorinated (C₁-C₆)-alkoxy with up to 6 fluorine atoms,
radicals of the formulae 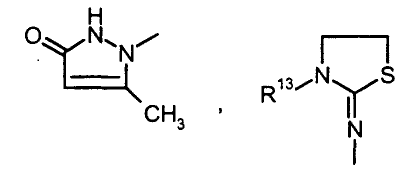 or, 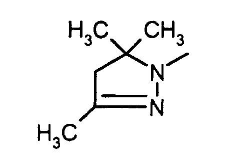 in which R¹³ denotes hydrogen or (C₁-C₆)-alkyl,
(C₁-C₆)-alkyl which is optionally substituted by a radical of the formula 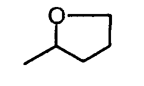 a 5- to 6-membered aromatic heterocycle which has up to 3 heteroatoms from the series S, N and/or O, is optionally bonded via a nitrogen atom and may optionally be substituted by one to three halogen atoms,
a 3- to 8-membered saturated or unsaturated, nonaromatic heterocycle which has up to 3 heteroatoms from the series S, N and/or O, is optionally bonded via a nitrogen atom and may optionally be substituted by 1 to 3 substituents selected from oxo, halogen, hydroxyl, (C₁-C₆)-alkoxycarbonyl, (C₁-C₆)-alkyl, halogeno(C₁-C₆)-alkyl and hydroxy(C₁-C₆)-alkyl,
or represents groups of the formulae -OR¹⁴, -NR¹⁵R¹⁶ or -CO- NR¹⁷R¹⁸,
in which
R¹⁴ denotes a radical of the formula 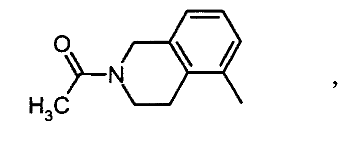 or denotes phenyl which in turn is optionally substituted by a group of the formula -NR¹⁹R²⁰,
in which
R¹⁹ and R²⁰ are identical or different and denote hydrogen, (C₁-C₆)-alkyl or (C₁-C₆)-acyl,
or
R¹⁴ denotes (C₁-C₆)-alkyl which is optionally substituted once to three times by hydroxyl,
R¹⁵ and R¹⁶ are identical or different and denote hydrogen, carbamoyl, mono- or di(C₁-C₆)alkylaminocarbonyl, phenyl, (C₁-C₆)-acyl or (C₁-C₆)-alkyl,
where (C₁-C₆)-alkyl is optionally substituted by (C₁-C₆)-alkoxy, (C₁-C₆)-acyl, by phenyl or by a 5- to 6-membered aromatic heterocycle with up to 3 heteroatoms from the series S, N and/or O,
where aforementioned phenyl and aforementioned aromatic heterocycle are optionally substituted once to three times, identically or differently, by halogen and/or hydroxyl, and
R¹⁷ and R¹⁸ are identical or different and denote hydrogen or (C₁-C₆)-alkyl, and
R²² may have the above meaning of R²¹ and may be identical to or different from the latter, or R²² is hydrogen.

11. Compounds according to Claim 9 or 10, in which R²² is hydrogen.

12. Compounds according to Claim 10 or 11, in which R²¹ represents phenyl, (C₁-C₄)-alkoxy or a 3- to 8-membered saturated or unsaturated, nonaromatic heterocycle which has up to 3 heteroatoms from the series S, N and/or O, is optionally bound via a nitrogen atom and may optionally be substituted by 1 to 3 substituents selected from oxo, halogen, hydroxyl, (C₁-C₆)-alkoxycarbonyl, (C₁-C₆)-alkyl, halogeno(C₁-C₆)alkyl and hydroxy(C₁-C₆)-alkyl.

13. Compounds of the general formula (II) 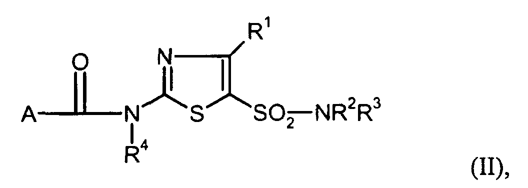 in which R¹, R², R³ and R⁴ have the meaning indicated in Claim 1, and A represents a halogen atom.

14. Process for preparing the compounds of the general formula (I) according to Claim 1, **characterized in that**
[A] compounds of the general formula (II) 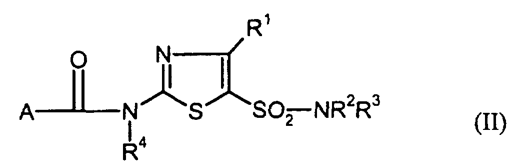 in which
R¹, R², R³ and R⁴ have the meaning indicated in Claim 1,
and
A represents halogen, preferably represents chlorine,
are reacted with amines of the general formula (III)
HNR⁵R⁶ (III)
in which
R⁵ and R⁶ have the meaning indicated in Claim 1,
in inert solvents, where appropriate in the presence of a base and/or aid,
or
[B] isocyanates of the general formula (IV)
R⁶-NCO (IV)
in which
R⁶ has the meaning indicated in Claim 1,
are reacted with thiazolylamines of the general formula (V) 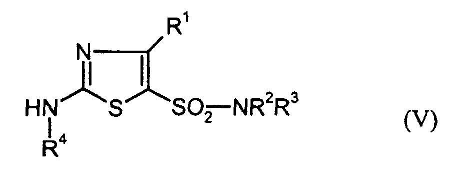 in which
R¹, R², R³ and R⁴ have the meaning indicated in Claim 1,
in inert solvents and, in the case where R⁵ is different from hydrogen, an alkylation is carried out by conventional processes.

15. Use of compounds of the general formula (I) according to Claim 1 for producing pharmaceuticals.

16. Use according to Claim 15 for producing compositions for the treatment and/or prevention of viral infections in humans or animals.

17. Use according to Claim 16 for producing compositions for the treatment and/or prevention of viral infections in humans or animals by herpes viruses.

18. Use according to Claim 16 for producing compositions for the treatment and/or prevention of viral infections in humans or animals by herpes simplex viruses.

19. Compounds according to Claim 1 for use as pharmaceuticals.

20. Pharmaceutical composition which comprises a compound of the general formula (I) according to Claim 1 mixed with a pharmaceutically acceptable carrier or excipient.

## Revendications

1. Composés de formule générale (I) 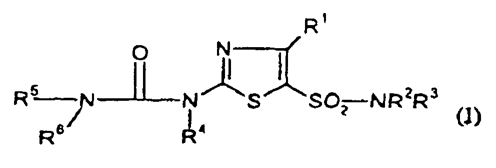 dans laquelle
R¹ représente l'hydrogène, un halogène, un reste alkyle en C₁ à C₆, alkoxy en C₁ à C₆, aminoalkyle en C₁ à C₆ ou halogénalkyle en C₁ à C₆,
R² et R³ sont identiques ou différents et représentent l'hydrogène, un reste cycloalkyle en C₃ à C₈ ou le reste biphénylaminocarbonyle, ou bien un reste alkyle en C₁ à C₆ qui est éventuellement substitué par 1 à 3 substituants qui sont choisis dans le groupe comprenant un reste cycloalkyl" en C₃ à C₆, alkoxy en C₁ à C₆, un halogène, un groupe hydroxy, des restes de formule 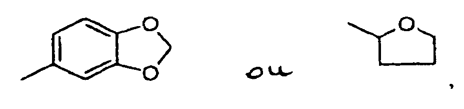 un hétérocycle aromatique pentagonal ou hexagonal ayant jusqu'à 3 hétéroatomes de la série S, N et/ou O, un hétérocycle contenant de l'azote pouvant aussi être lié par l'intermédiaire de l'atome d'azote, un hétérocycle non aromatique ayant jusqu'à 3 hétéroatomes de la série S, N et/ou O, saturé ou non saturé, de 3 à 8 chaînons, éventuellement, lié par l'intermédiaire d'un atome d'azote, et un reste aryle en C₆ à C₁₈ qui peut lui-même être substitué par un radical hydroxy ou alkoxy en C₁ à C₆, ou bien
R² et R³ forment conjointement avec l'atome d'azote un hétérocycle saturé pentagonal ou hexagonal qui peut encore porter le cas échéant un atome d'oxygène,
R⁴ représente l'hydrogène, un reste acyle en C₁ à C₆, alcényle en C₂ à C₆, ou bien
R⁴ représente un reste alkyle en C₁ à C₆ qui peut éventuellement être substitué par 1 à 3 substituants qui sont choisis dans le groupe constitué d'un halogène, d'un groupe hydroxy, d'un reste acyle en C₁ à C₆, alkoxy en C₁ à C₆, phénoxy, aryle en C₆ à C₁₀ et -NR⁷R⁸,
où R⁷ et R⁸ sont identiques ou différents et représentent l'hydrogène, un radical acyle en C₁ à C₆, alkyle en C₁ à C₆, carbamoyle, mono- ou di- (alkyle en C₁ à C₆) -amino- (alkyle en C₁ à C₆) , mono- ou di-(alkyle en C₁ à C₆)-aminocarbonyle, aryle en C₆ à C₁₀ ou (alkoxy en C₁ à C₆)-carbonyle, ou bien
R⁷ et R⁸ forment conjointement avec l'atome d'azote un hétérocycle saturé pentagonal ou hexagonal, qui peut éventuellement contenir un autre hétéroatome de la série S ou O ou un reste de formule -NR⁹ et qui peut être substitué par un groupe oxo,
R⁹ représentant l'hydrogène ou un radical alkyle en C₁ à C₄,
ou bien
R⁴ est un reste alkyle en C₁ à C₆ qui est substitué par un hétérocycle aromatique pentagonal ou hexagonal éventuellement condensé au benzène, ayant jusqu'à 3 hétéroatomes de la série S, N et/ou O, un hétérocycle contenant de l'azote pouvant aussi être lié par l'intermédiaire de l'atome d'azote, ou bien substitué par des restes de formules 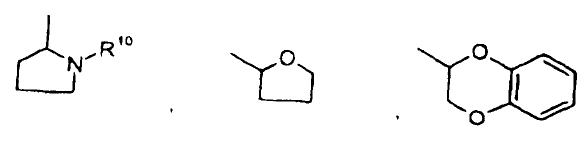 ou 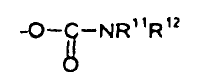 dans lesquelles
R¹⁰ représente l'hydrogène ou un reste alkyle en C₁ à C₆,
R¹¹ et R¹² sont identiques ou différents et représentent l'hydrogène, un reste alkyle en C₁ à C₆ ou aryle en C₆ à C₁₀, le reste alkyle en C₁ à C₆ et le reste aryle en C₆ à C₁₀ mentionnés ci-dessus pouvant éventuellement être substitués par 1 à 3 substituants qui sont choisis dans le groupe des radicaux hydroxy, alkoxy en C₁ à C₆ et halogéno,
R⁵ représente l'hydrogène ou un reste alkyle en C₁ à C₆,
R⁶ est un reste de formule 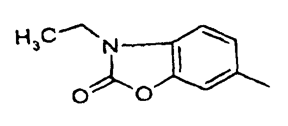
ou bien
R⁶ est un reste phényle qui peut éventuellement être substitué par 1 à 3 substituants qui sont choisis dans le groupe comprenant un halogène, un reste alkyle en C₆ à C₁₀ qui peut éventuellement être substitué par 1 à 3 substituants choisis entre alcanoyle en C₁ à C₆, alkoxy en C₁ à C₆, alkyle en C₁ à C₆, halogène, (alkoxy en C₁ à C₆)-carbonyle, nitro, halogénalkyle en C₁ à C₆, halogènalkoxy en C₁ à C₆, amino, alkylthio en C₁ à C₆, hydroxy, carboxyle, carbamoyle, mono- ou di- (alkyle en C₁ à C₆) -amino-carbonyle, mono- ou di-(acyle en C₁ à C₆)-amino, alkylsulfoxy en C₁ à C₆, alkylsulfonyle en C₁ à C₆ et/ou cyano, un reste alkoxy en C₁ à C₆, (alkoxy en C₁ à C₆)-carbonyle, alkylthio en C₁ à C₆, hydroxy, carboxyle, alkoxy en C₁ à C₆ partiellement fluoré ayant jusqu'à 6 atomes de fluor,
des restes de formules 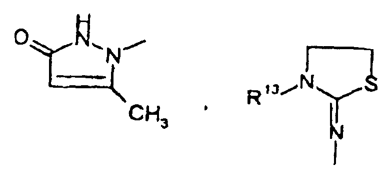 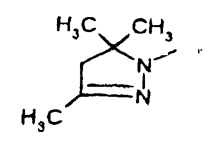 ou
dans lesquelles R¹³ représente l'hydrogène ou un radical alkyle en C₁ à C₆,
alkyle en C₁ à C₆ qui est éventuellement substitué par un reste de formule 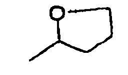 un hétérocycle aromatique pentagonal ou hexagonal ayant jusqu'à 3 hétéroatomes de la série S, N et/ou O éventuellement lié par l'intermédiaire d'un atome d'azote, et pouvant être substitué le cas échéant par 1 à 3 substituants choisis entre alcanoyle en C₁ à C₆, alkoxy en C₁ à C₆, alkyle en C₁ à C₆, halogéno, (alkoxy en C₁ à C₆)-carbonyle, nitro, halogénalkyle en C₁ à C₆, halogénalkoxy en C₁ à C₆, amino, alkylthio en C₁ à C₆, hydroxy, carboxyle, carbamoyle, mono- ou di-(alkyle en C₁ à C₆)-aminocarbonyle, mono- ou di-(acyle en C₁ à C₆) -amino, alkylsulfoxy en C₁ à C₆, alkylsulfonyle en C₁ à C₆ et/ou cyano,
un hétérocycle non aromatique monocyclique ou bicyclique ayant jusqu'à 3 hétéroatomes de la série S, N et/ou O, saturé ou non saturé, ayant 3 à 8 chaînons, éventuellement lié par l'intermédiaire d'un atome d'azote et pouvant être substitué par 1 à 3 substituants choisis entre oxo, halogéno, hydroxy, (alkoxy en C₁ à C₆)-carbonyle, (alkoxy en C₁ à C₆)-carbonylamino, alkyle en C₁ à C₆, halogénalkyle en C₁ à C₆ et hydroxyalkyle en C₁ à C₆,
et des groupes de formules -OR¹⁴, -NR¹⁵R¹⁶ ou -CO-NR¹⁷R¹⁸,
où
R¹⁴ est un reste de formule 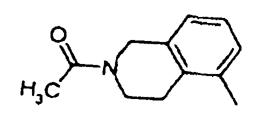 ou un reste phényle qui est lui-même éventuellement substitué par un groupe de formule -NR¹⁹R²⁰,
dans laquelle
R¹⁹ et R²⁰ sont identiques ou différents et représentent l'hydrogène, un reste alkyle en C₁ à C₆ ou acyle en C₁ à C₆,
ou bien
R¹⁴ est un reste alkyle en C₁ à C₆ qui est éventuellement substitué une à trois fois par un radical hydroxy,
R¹⁵ et R¹⁶ sont identiques ou différents et représentent l'hydrogène, un groupe carbamoyle, un reste mono- ou di-(alkyle en C₁ à C₆)-aminocarbonyle, phényle, acyle en C₁ à C₆ ou alkyle en C₁ à C₆,
le radical alkyle en C₁ à C₆ pouvant être substitué par un reste alkoxy en C₁ à C₆, acyle en C₁ à C₆, par un reste phényle ou par un hétérocycle aromatique pentagonal ou hexagonal ayant jusqu'à 3 hétéroatomes de la série S, N et/ou O,
le reste phényle mentionné ci-dessus et l'hétérocycle aromatique mentionné ci-dessus étant substitués une à trois fois identiques ou différentes par un halogène et/ou par un radical hydroxy, et
R¹⁷ et R¹⁸ sont identiques ou différents et représentent l'hydrogène ou un reste alkyle en C₁ à C₆,
et leurs sels.

2. Composés de formule générale (I) suivant la revendication 1, formule dans laquelle
R⁶ représente un reste phényle, qui peut éventuellement porter un à trois substituants qui sont choisis dans le groupe constitué d'un halogène, d'un reste aryle en C₆ à C₁₀ qui peut éventuellement porter 1 à 3 substituants choisis entre alcanoyle en C₁ à C₆, alkoxy en C₁ à C₆, alkyle en C₁ à C₆, halogène, (alkoxy en C₁ à C₆)-carbonyle, nitro, halogénalkyle en C₁ à C₆, halogénalkoxy en C₁ à C₆, amino, alkylthio en C₁ à C₆, hydroxy, carboxyle, carbamoyle, mono- ou di-(alkyle en C₁ à C₆)-aminocarbonyle, mono- ou di-(acyle en C₁ à C₆)-amino, alkylsulfoxy en C₁ à C₆, alkylsulfonyle en C₁ à C₆ et/ou cyano, d'un reste alkoxy en C₁ à C₆, (alkoxy en C₁ à C₆)-carbonyle, alkylthio en C₁ à C₆, hydroxy, carboxyle, alkoxy en C₁ à C₆ partiellement fluoré ayant jusqu'à 6 atomes de fluor, alkyle en C₁ à C₆, un hétérocycle aromatique pentagonal ou hexagonal ayant jusqu'à 3 hétéroatomes de la série S, N et/ou O, éventuellement lié par l'intermédiaire d'un atome d'azote et pouvant éventuellement porter 1 à 3 substituants choisis entre alcanoyle en C₁ à C₆, alkoxy en C₁ à C₆, alkyle en C₁ à C₆, halogène, (alkoxy en C₁ à C₆)-carbonyle, nitro, halogénalkyle en C₁ à C₆, halogénalkoxy en C₁ à C₆, amino, alkylthio en C₁ à C₆, hydroxy, carboxyle, carbamoyle, mono- ou di-(alkyle en C₁ à C₆) -aminocarbonyle, mono- ou di-(acyle en C₁ à C₆)-amino, alkylsulfoxy en C₁ à C₆, alkylsulfonyle en C₁ à C₆ et/ou cyano, un hétérocycle monocyclique ou bicyclique non aromatique ayant jusqu'à 3 hétéroatomes de la série S, N et/ou O, saturé ou non saturé, de 3 à 8 chaînons, éventuellement lié par l'intermédiaire d'un atome d'azote et pouvant porter 1 à 3 substituants choisis entre oxo, halogène, hydroxy, (alkoxy en C₁ à C₆)-carbonyle, (alkoxy en C₁ à C₆)-carbonylamino, alkyle en C₁ à C₆, halogénalkyle en C₁ à C₆ et hydroxyalkyle en C₁ à C₆,
et de groupes de formules -OR¹⁴, -NR¹⁵R¹⁶ ou -CO-NR¹⁷R¹⁸, formules dans lesquelles R¹⁴ désigne un reste phényle qui est lui-même éventuellement substitué par un groupe de formule -NR¹⁹R²⁰,
où
R¹⁹ et R²⁰ sont identiques ou différents et représentent l'hydrogène, un radical alkyle en C₁ à C₆ ou acyle en C₁ à C₆,
ou bien
R¹⁴ désigne un reste alkyle en C₁ à C₆ qui est éventuellement substitué une à trois fois par un radical hydroxy,
R¹⁵ et R¹⁶ sont identiques ou différents et représentent l'hydrogène, un radical carbamoyle, mono- ou di-(alkyle en C₁ à C₆) -aminocarbonyle, phényle, acyle en C₁ à C₆ ou alkyle en C₁ à C₆, le radical alkyle en C₁ à C₆ étant éventuellement substitué par un reste alkoxy en C₁ à C₆, acyle en C₁ à C₆, par un reste phényle ou par un hétérocycle aromatique pentagonal ou hexagonal ayant jusqu'à 3 hétéroatomes de la série S, N et/ou O,
le radical phényle mentionné ci-dessus et l'hétérocycle aromatique mentionné ci-dessus étant éventuellement substitués une à trois fois identiques ou différentes par un halogène et/ou par un radical hydroxy, et
R¹⁷ et R¹⁸ sont identiques ou différents et représentent l'hydrogène ou un reste alkyle en C₁ à C₆,
et leurs sels.

3. Composés de formule générale (I) suivant la revendication 1, formule dans laquelle
R⁶ est un reste phényle qui peut éventuellement porter 1 à 3 substituants qui sont choisis dans le groupe constitué d'un halogène, d'un reste alkyle en C₆ à C₁₀ qui peut porter éventuellement 1 à 3 substituants choisis entre alcanoyle en C₁ à C₆, alkoxy en C₁ à C₆, alkyle en C₁ à C₆, halogène, (alkoxy en C₁ à C₆)-carbonyle, nitro, halogénalkyle en C₁ à C₆, halogénalkoxy en C₁ à C₆, amino, alkylthio en C₁ à C₆, hydroxy, carboxyle, carbamoyle, mono- ou di-(alkyle en C₁ à C₆)-aminocarbonyle, mono- ou di-(acyle en C₁ à C₆) -amino, alkylsulfoxy en C₁ à C₆, alkylsulfonyle en C₁ à C₆ et/ou cyano, d'un reste alkoxy en C₁ à C₆, (alkoxy en C₁ à C₆)-carbonyle, alkylthio en C₁ à C₆, hydroxy, carboxyle, alkoxy en C₁ à C₆ partiellement fluoré ayant jusqu'à 6 atomes de fluor et alkyle en C₁ à C₆,
et leurs sels.

4. Composés de formule générale (I) suivant la revendication 1, formule dans laquelle
R¹ représente l'hydrogène, un halogène ou un reste alkyle en C₁ à C₆,
R² et R³ sont identiques ou différents et représentent l'hydrogène ou un reste cycloalkyle en C₃ à C₈, ou bien
un reste alkyle en C₁ à C₆, qui porte éventuellement 1 à 3 substituants choisis dans le groupe constitué d'un reste cycloalkyle en C₃ à C₆, alkoxy en C₁ à C₆, d'un halogène, d'un groupe hydroxy, de restes de formule 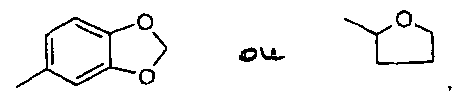 d'un hétérocycle aromatique pentagonal ou hexagonal ayant jusqu'à 3 hétéroatomes de la série S, N et/ou O, un hétérocycle contenant de l'azote pouvant aussi être lié par l'intermédiaire d'un atome d'azote, et d'un reste aryle en C₆ à C₁₀ qui peut lui-même être substitué par un radical hydroxy ou alkoxy en C₁ à C₆, ou bien
R² et R³ forment conjointement avec l'atome d'azote, un hétérocycle saturé pentagonal ou hexagonal qui peut encore porter le cas échéant un atome d'oxygène,
R⁴ représente l'hydrogène, un reste acyle en C₁ à C₆, alcényle en C₂ à C₆, ou bien
R⁴ représente un reste alkyle en C₁ à C₆ qui peut porter éventuellement 1 à 3 substituants choisis dans le groupe constitué d'un halogène, d'un reste hydroxy, acyle en C₁ à C₆, alkoxy en C₁ à C₆, phénoxy, aryle en C₆ à C₁₀ et -NR⁷R⁸,
où R⁷ et R⁸ sont identiques ou différents et représentent l'hydrogène, un radical acyle en C₁ à C₆, alkyle en C₁ à C₆, carbamoyle, mono- ou di- (alkyle en C₁ à C₆)-aminocarbonyle ou (alkoxy en C₁ à C₆)-carbonyle, ou bien
R⁷ et R⁸ forment conjointement avec l'atome d'azote un hétérocycle saturé pentagonal ou hexagonal qui peut éventuellement porter un autre hétéroatome de la série S ou O ou un reste de formule -NR⁹
dans laquelle R⁹ représente l'hydrogène ou un radical alkyle en C₁ à C₄,
ou bien
R⁴ désigne un reste alkyle en C₁ à C₆ qui est substitué par un hétérocycle pentagonal ou hexagonal aromatique éventuellement condensé au benzène ayant jusqu'à 3 hétéroatomes de la série S, N et/ou O, un hétérocycle contenant de l'azote pouvant aussi être lié par l'intermédiaire de l'atome d'azote, ou substitué par
des restes de formules 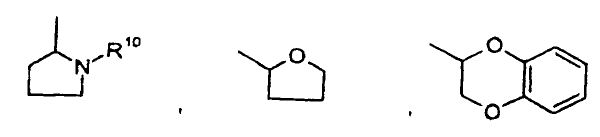 ou 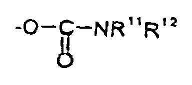 dans lesquelles
R¹⁰ représente l'hydrogène ou un reste alkyle en C₁ à C₆,
R¹¹ et R¹² sont identiques ou différents et représentent l'hydrogène, un reste alkyle en C₁ à C₆ ou aryle en C₆ à C₁₀, les restes alkyle en C₁ à C₆ et aryle en C₆ à C₁₀ mentionnés ci-dessus pouvant être substitués par 1 à 3 substituants qui sont choisis dans le groupe des radicaux hydroxy, alkoxy en C₁ à C₆ et halogèno,
R⁵ représente l'hydrogène ou un reste alkyle en C₁ à C₆,
R⁶ est un reste de formule 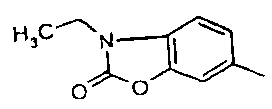
ou bien
R⁶ est un reste phényle qui peut porter éventuellement 1 ou 2 substituants qui sont choisis dans le groupe constitué d'un halogène, d'un reste aryle en C₆ à C₁₀, alkoxy en C₁ à C₆, (alkoxy en C₁ à C₆)-cabonyle, alkylthio en C₁ à C₆, hydroxy, carboxyle, alkoxy en C₁ à C₆ partiellement fluoré avec jusqu'à 6 atomes de fluor,
des restes de formules 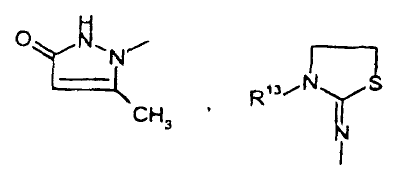 ou 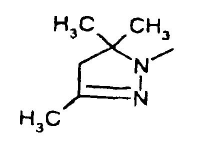 dans lesquelles R¹³ représente l'hydrogène ou un reste alkyle en C₁ à C₆,
un reste alkyle en C₁ à C₆ qui est éventuellement substitué par un reste de formule 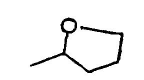 un hétérocycle aromatique pentagonal ou hexagonal éventuellement lié par l'intermédiaire d'un atome d'azote, ayant jusqu'à 3 hétéroatomes de la série S, N et/ou O, qui peut éventuellement être substitué par 1 à 3 atomes d'halogène,
un hétérocycle non aromatique, saturé ou non saturé, de 3 à 8 chaînons éventuellement liés par l'intermédiaire d'un atome d'azote, avec jusqu'à 3 hétéroatomes de la série S, N et/ou O, qui peut être substitué le cas échéant par 1 à 3 substituants choisis entre oxo, halogéno, hydroxy, (alkoxy en C₁ à C₆)-carbonyle, alkyle en C₁ à C₆, halogénalkyle en C₁ à C₆ et hydroxyalkyle en C₁ à C₆,
et des groupes de formules -OR¹⁴, -NR¹⁵R¹⁶ ou -CO-NR¹⁷R¹⁸,
où
R¹⁴ est un reste de formule 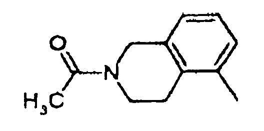 ou un reste phényle qui est lui-même éventuellement substitué par un groupe de formule -NR¹⁹R²⁰,
où
R¹⁹ et R²⁰ sont identiques ou différents et représentent l'hydrogène, un radical alkyle en C₁ à C₆ ou acyle en C₁ à C₆,
ou bien
R¹⁴ est un reste alkyle en C₁ à C₆ qui est éventuellement substitué une à trois fois par un radical hydroxy,
R¹⁵ et R¹⁶ sont identiques ou différents et représentent l'hydrogène, un reste carbamoyle, mono- ou di-(alkyle en C₁ à C₆)-aminocarbonyle, phényle, acyle en C₁ à C₆ ou alkyle en C₁ à C₆, le radical alkyle en C₁ à C₆ pouvant être substitué par un radical alkoxy en C₁ à C₆, acyle en C₁ à C₆, par un radical phényle ou par un hétérocycle aromatique pentagonal ou hexagonal ayant jusqu'à 3 hétéroatomes de la série S, N et/ou O,
le radical phényle qui vient d'être mentionné et l'hétérocycle aromatique qui vient d'être mentionné étant éventuellement substitués une à trois fois identiques ou différentes par un halogène et/ou par un groupe hydroxy, et
R¹⁷ et R¹⁸ sont identiques ou différents et représentent l'hydrogène ou un reste alkyle en C₁ à C₆,
et leurs sels.

5. Composés de formule générale (I) suivant la revendication 4, formule dans laquelle
R¹ représente l'hydrogène, le chlore ou un reste alkyle en C₁ à C₃,
R² et R³ sont identiques ou différents et représentent l'hydrogène ou un reste cyclopropyle ou cyclopentyle,
ou bien
un reste alkyle en C₁ à C₃ qui porte éventuellement 1 à 3 substituants choisis dans le groupe constitué des restes cyclopropyle, cyclopentyle, alkoxy en C₁ à C₃, chloro, fluoro, hydroxy, des restes de formule 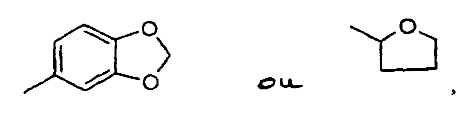 et pyridyle, furyle, thiényle, imidazolyle, N-triazolyle ou pyrrolyle, un reste phényle qui peut lui-même être substitué par un radical hydroxy ou alkoxy en C₁ à C₃, ou bien
R² et R³ forment conjointement avec l'atome d'azote un noyau morpholine, pipéridine ou pyrrolidine,
R⁴ représente l'hydrogène, un reste acyle en C₁ à C₃, alcényle en C₂ ou C₃, ou bien
R⁴ représente un reste alkyle en C₁ à C₆ qui peut éventuellement porter 1 à 3 substituants choisis dans le groupe constitué du chlore, du fluor, des groupes hydroxy, acyle en C₁ à C₃, alkoxy en C₁ à C₃, phénoxy, phényle et -NR⁷R⁸,
où R⁷ et R⁸ sont identiques ou différents et représentent l'hydrogène, un radical acyle en Ci à C₄, alkyle en C₁ à C₄, carbamoyle, mono- ou di-(alkyle en C₁ à C₃)-aminocarbonyle, ou (alkoxy en C₁ à C₄)-carbonyle, ou bien
R⁷ et R⁸ forment conjointement avec l'atome d'azote un noyau morpholino, pipéridinyle ou pyrrolidinyle, ou bien
R⁴ est un reste alkyle en C₁ à C₆ qui est substitué par un hétérocycle aromatique pentagonal ou hexagonal éventuellement condensé au benzène, ayant jusqu'à 3 hétéroatomes de la série S, N et/ou O, un hétérocycle contenant de l'azote pouvant aussi être lié par l'intermédiaire de l'atome d'azote, ou substitué par
des restes de formules 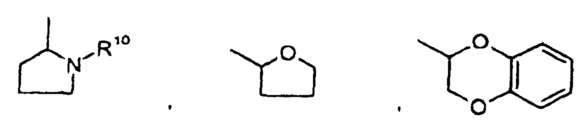 ou 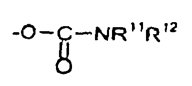 où
R¹⁰ représente l'hydrogène ou un reste alkyle en C₁ à C₄,
R¹¹ et R¹² sont identiques ou différents et représentent l'hydrogène, un reste alkyle en C₁ à C₃ ou phényle, le reste alkyle en C₁ à C₃ et le reste phényle venant d'être mentionnés pouvant éventuellement porter 1 à 3 substituants choisis dans le groupe comprenant les substituants hydroxy, alkoxy en C₁ à C₃, chlore et fluor,
R⁵ représente l'hydrogène ou un radical alkyle en C₁ à C₃,
R⁶ représente un radical phényle qui peut éventuellement porter un ou deux substituants choisis dans le groupe constitué des substituants chloro, fluoro, phényle, alkoxy en C₁ à C₄, (alkoxy en C₁ à C₄)-carbonyle, alkylthio en Ci à C₃, hydroxy, carboxyle, alkoxy en C₁ à C₄ partiellement fluoré ayant jusqu'à 5 atomes de fluor,
des restes de formules 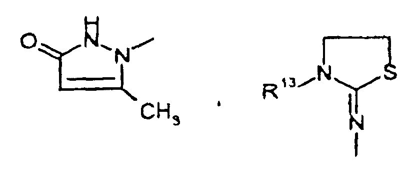 ou 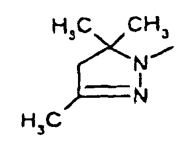 où R¹³ représente l'hydrogène ou un radical alkyle en C₁ à C₃,
un reste alkyle en C₁ à C₆ qui est éventuellement substitué par un reste de formule 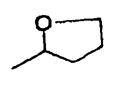 un reste triazolyle,
des restes morpholino, thiomorpholino, pipéridinyle, pyrrolidinyle, azacycloheptanyle, azacyclobutanyle, qui peuvent éventuellement porter 1 ou 2 substituants choisis entre oxo, chloro, fluoro, hydroxy, (alkoxy en C₁ à C₄)-carbonyle, alkyle en C₁ à C₃, chloralkyle ou fluoralkyle en C₁ à C₃ et hydroxyalkyle en C₁ à C₄, 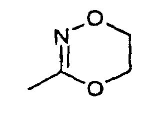 et des groupes de formules -OR¹⁴, -NR¹⁵R¹⁶ ou -CO- NR¹⁷R¹⁸,
où
R¹⁴ représente un reste de formule 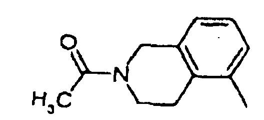 ou un reste phényle qui peut lui-même être substitué le cas échéant par un groupe de formule -NR¹⁹R²⁰,
où
R¹⁹ et R²⁰ sont identiques ou différents et représentent l'hydrogène, un reste alkyle en C₁ à C₃ ou acyle en C₁ à C₃,
ou bien
R¹⁴ désigne un reste alkyle en C₁ à C₄ qui est éventuellement substitué une à trois fois par un radical hydroxy,
R¹⁵ et R¹⁶ sont identiques ou différents et représentent l'hydrogène, un reste carbamoyle, mono- ou di-(alkyle en C₁ à C₃)-aminocarbonyle, phényle, acyle en C₁ à C₃ ou alkyle en C₁ à C₃, le reste alkyle en C₁ à C₃ étant éventuellement substitué par un radical alkoxy en C₁ à C₃, acyle en C₁ à C₃, par un radical phényle ou pyridyle,
le radical phényle et le radical pyridyle venant d'être mentionnés étant éventuellement substitués une ou deux fois identiques ou différentes par du chlore, du fluore et/ou un radical hydroxy, et
R¹⁷ et R¹⁸ sont identiques ou différents et représentent l'hydrogène ou un reste alkyle en C₁ à C₄,
et leurs sels.

6. Composés de formule générale (I) suivant l'une quelconque des revendications 1 à 5, formule dans laquelle R⁵ représente l'hydrogène.

7. Composés de formule générale (I) suivant l'une quelconque des revendications 1 à 5, formule dans laquelle R² et R³ représentent l'hydrogène ou un reste alkyle en C₁ à C₃.

8. Composés de formule générale (I) suivant l'une quelconque des revendications 1 à 7, formule dans laquelle R⁶ est un groupe phénylène substitué en para.

9. Composés suivant la revendication 1, qui présentent la formule suivante : 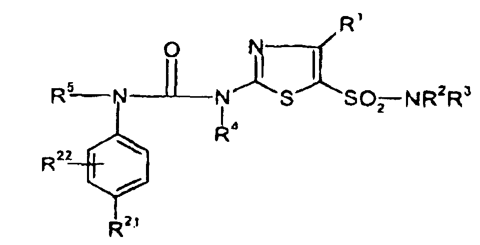 dans laquelle
R¹ à R⁵ sont tels que définis ci-dessus et R²¹ représente
un halogène, un reste aryle en C₆ à C₁₀ qui peut éventuellement porter 1 à 3 substituants choisis entre alcanoyle en C₁ à C₆, alkoxy en C₁ à C₆, alkyle en C₁ à C₆, halogéno, (alkoxy en C₁ à C₆)-carbonyle, nitro, halogénalkyle en C₁ à C₆, halogénalkoxy en C₁ à C₆, amino, alkylthio en C₁ à C₆, hydroxy, carboxyle, carbamoyle, mono- ou di- (alkyle en C₁ à C₆)-aminocarbonyle, mono- ou di-(acyle en C₁ à C₆)-amino, alkylsulfoxy en C₁ à C₆, alkylsulfonyle en C₁ à C₆ et/ou cyano, un reste alkoxy en C₁ à C₆, (alkoxy en C₁ à C₆)-carbonyle, alkylthio en C₁ à C₆, hydroxy, carboxyle, alkoxy en C₁ à C₆ partiellement fluoré ayant jusqu'à 6 atomes de fluor, des restes de formules 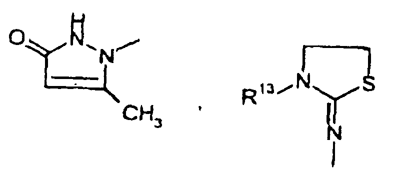 ou 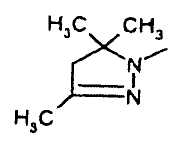 où R¹³ représente l'hydrogène ou un reste alkyle en C₁ à C₆,
un reste alkyle en C₁ à C₆ qui est éventuellement substitué par un reste de formule 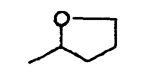 un hétérocycle aromatique pentagonal ou hexagonal éventuellement lié par l'intermédiaire d'un atome d'azote, ayant jusqu'à 3 hétéroatomes de la série S, N et/ou O, qui peut éventuellement être substitué par 1 à 3 substituants choisis entre alcanoyle en C₁ à C₆, alkoxy en C₁ à C₆, alkyle en C₁ à C₆, halogéno, (alkoxy en C₁ à C₆)-carbonyle, nitro, halogénalkyle en C₁ à C₆, halogénalkoxy en C₁ à C₆, amino, alkylthio en C₁ à C₆, hydroxy, carboxyle, carbamoyle, mono- ou di-(alkyle en C₁ à C₆)-aminocarbonyle, mono- ou di-(acyle en C₁ à C₆)-amino, alkylsulfoxy en C₁ à C₆, alkylsulfonyle en C₁ à C₆ et/ou cyano,
un hétérocycle non aromatique monocyclique ou bicyclique, saturé ou non saturé, de 3 à 8 chaînons, éventuellement lié par l'intermédiaire d'un atome d'azote, ayant jusqu'à 3 hétéroatomes de la série S, N et/ou O, qui peut éventuellement être substitué par 1 à 3 substituants choisis entre oxo, halogéno, hydroxy, (alkoxy en C₁ à C₆)-carbonyle, (alkoxy en C₁ à C₆)-carbonylamino, alkyle en C₁ à C₆, halogénalkyle en C₁ à C₆ et hydroxyalkyle en C₁ à C₆,
ou des groupes de formules -OR¹⁴, -NR¹⁵R¹⁶ ou -CO-NR¹⁷R¹⁸,
formules dans lesquelles
R¹⁴ représente un reste de formule 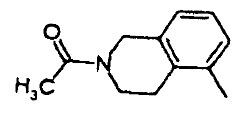 ou phényle qui est lui-même éventuellement substitué par un groupe de formule -NR¹⁹R²⁰,
où
R¹⁹ et R²⁰ sont identiques ou différents et représentent l'hydrogène, un reste alkyle en C₁ à C₆ ou acyle en C₁ à C₆,
ou bien
R¹⁴ désigne un reste alkyle en C₁ à C₆ qui est éventuellement substitué une à trois fois par un groupe hydroxy,
R¹⁵ et R¹⁶ sont identiques ou différents et représentent l'hydrogène, un reste carbamoyle, mono- ou di-(alkyle en C₁ à C₆)-aminocarbonyle, phényle, acyle en C₁ à C₆ ou alkyle en C₁ à C₆, le reste alkyle en C₁ à C₆ étant éventuellement substitué par un radical alkoxy en C₁ à C₆, acyle en C₁ à C₆, par un radical phényle ou par un hétérocycle aromatique pentagonal ou hexagonal ayant jusqu'à 3 hétéroatomes de la série S, N et/ou O,
le radical phényle et l'hétérocycle aromatique venant d'être mentionnés étant substitués une à trois fois identiques ou différentes par un halogène et/ou un groupe hydroxy, et
R¹⁷ et R¹⁸ sont identiques ou différents et représentent l'hydrogène ou un reste alkyle en C₁ à C₆, et
R²² peut présenter la définition indiquée ci-dessus pour R²¹ et peut y être identique ou en être différent, ou bien R²² est l'hydrogène,
et leurs sels.

10. Composés suivant la revendication 4, qui présente la formule suivante : 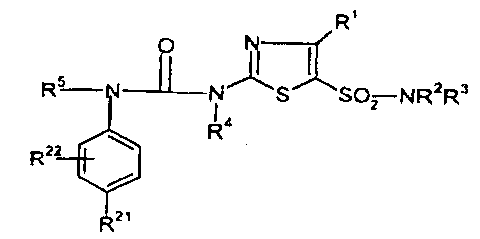 dans laquelle
R¹ à R⁵ sont tels que définis ci-dessus et R²¹ représente un halogène, un reste aryle en C₆ à C₁₀, alkoxy en C₁ à C₆, (alkoxy en C₁ à C₆)-carbonyle, alkylthio en C₁ à C₆, hydroxy, carboxyle, alkoxy en C₁ à C₆ partiellement fluoré ayant jusqu'à 6 atomes de fluor, des restes de formules 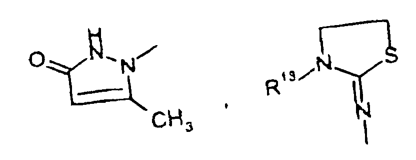 ou 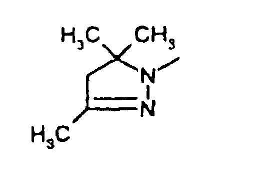 où R¹³ représente l'hydrogène ou un reste alkyle en C₁ à C₆,
un reste alkyle en C₁ à C₆ qui est éventuellement substitué par un reste de formule 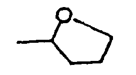 un hétérocycle aromatique pentagonal ou hexagonal éventuellement lié par l'intermédiaire d'un atome d'azote, ayant jusqu'à 3 hétéroatomes de la série S, N et/ou O, qui peut éventuellement être substitué par un à trois atomes d'halogène,
un hétérocycle non aromatique, saturé ou non saturé, de 3 à 8 chaînons, éventuellement lié par l'intermédiaire d'un atome d'azote, ayant jusqu'à 3 hétéroatomes de la série S, N et/ou O, qui peut éventuellement être substitué par 1 à 3 substituants choisis entre oxo, halogèno, hydroxy, (alkoxy en C₁ à C₆)-carbonyle, alkyle en C₁ à C₆, halogénalkyle en C₁ à C₆ et hydroxyalkyle en C₁ à C₆,
ou des groupes de formules -OR¹⁴, -NR¹⁵R¹⁶ ou -CO-NR¹⁷R¹⁸,
formules dans lesquelles
R¹⁴ est un reste de formule 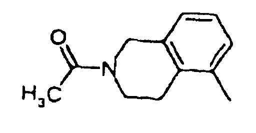 ou représente un reste phényle qui est lui-même éventuellement substitué par un groupe de formule -NR¹⁹R²⁰,
où
R¹⁹ et R²⁰ sont identiques ou différents et représentent l'hydrogène, un reste alkyle en C₁ à C₆ ou acyle en C₁ à C₆,
ou bien
R¹⁴ est un reste alkyle en C₁ à C₆ qui est éventuellement substitué une à trois fois par un radical hydroxy,
R¹⁵ et R¹⁶ sont identiques ou différents et représentent l'hydrogène, un reste carbamoyle, mono- ou di-(alkyle en C₁ à C₆)-aminocarbonyle, phényle, acyle en C₁ à C₆ ou alkyle en C₁ à C₆, le reste alkyle en C₁ à C₆ étant éventuellement substitué par un radical alkoxy en C₁ à C₆ ou acyle en C₁ à C₆, par un radical phényle ou par un hétérocycle aromatique pentagonal ou hexagonal ayant jusqu'à 3 hétéroatomes de la série S, N et/ou O,
le radical phényle et l'hétérocycle aromatique venant d'être mentionnés étant éventuellement substitués une à trois fois identiques ou différentes par un halogène et/ou un groupe hydroxy, et
R¹⁷ et R¹⁸ sont identiques ou différents et représentent l'hydrogène ou un reste alkyle en C₁ à C₆, et
R²² peut avoir la définition indiquée ci-deesus pour R²¹ et peut y être identique ou en être différent, ou bien R²² représente l'hydrogène.

11. Composés suivant la revendication 9 ou 10, dans lesquels R²² représente l'hydrogène.

12. Composés suivant la revendication 10 ou 11, dans lesquels R²¹ représente un reste phényle, alkoxy en C₁ à C₄ ou un hétérocycle non aromatique, saturé ou non saturé, de 3 à 8 chaînons éventuellement liés par l'intermédiaire d'un atome d'azote, ayant jusqu'à 3 hétéroatomes de la série S, N et/ou O, qui peut éventuellement être substitué par 1 à 3 substituants choisis entre oxo, halogéno, hydroxy, (alkoxy en C₁ à C₆)-carbonyle, alkyle en C₁ à C₆, halogénalkyle en C₁ à C₆ et hydroxyalkyle en C₁ à C₆.

13. Composés de formule générale (II) 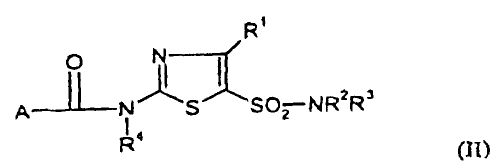 dans laquelle R¹, R², R³ et R⁴ ont la définition indiquée dans la revendication 1 et A représente un atome d'halogène.

14. Procédé de production des composés de formule générale (I) suivant la revendication 1, **caractérisé en ce que** :
[A] on fait réagir des composés de formule générale (II) 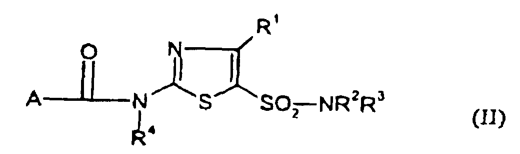 dans laquelle
R¹, R², R³ et R⁴ ont la définition indiquée dans la revendication 1
et
A représente un halogène, avantageusement le chlore,
avec des amines de formule générale (III)
HNR⁵R⁶ (III)
dans laquelle
R⁵ et R⁶ ont la définition indiquée dans la revendication 1, dans des solvants inertes, éventuellement en présence d'une base et/ou d'un agent auxiliaire
ou bien
[B] On fait réagir des isocyanates de formule générale (IV)
R⁶-NCO (IV)
dans laquelle
R⁶ a la définition indiquée dans la revendication 1,
avec des thiazolylamines de formule générale (V) 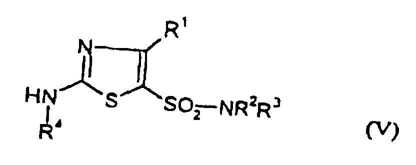 dans laquelle
R¹, R², R³ et R⁴ ont la définition indiquée dans la revendication 1,
dans des solvants inertes, et au cas où R⁵ n'est pas de l'hydrogène, on effectue une alkylation selon des modes opératoires usuels.

15. Utilisation de composés de formule générale (I) suivant la revendication 1 pour la préparation de médicaments.

16. Utilisation suivant la revendication 15 pour la préparation de médicaments destinés au traitement curatif et/ou préventif d'infections virales chez des êtres humains ou des animaux.

17. Utilisation suivant la revendication 16 pour la préparation de médicaments destinés au traitement curatif et/ou préventif d'infections virales dues à des virus de l'herpès chez des êtres humains ou des animaux.

18. Utilisation suivant la revendication 16 pour la préparation de médicaments destinés au traitement curatif et/ou préventif d'infections virales dues à des virus de l'Herpes simplex chez des êtres humains ou des animaux.

19. Composés suivant la revendication 1, destinés à être utilisés comme médicaments.

20. Composition pharmaceutique qui comprend un composé de formule générale (I) suivant la revendication 1 en mélange avec un support ou excipient acceptable du point de vue pharmaceutique.
